(19) Europäisches Patentamt
European Patent Office
Office européen des brevets

(11) **EP 4 108 675 A1**

(12) **EUROPEAN PATENT APPLICATION**
published in accordance with Art. 153(4) EPC

(43) Date of publication:
**28.12.2022 Bulletin 2022/52**

(21) Application number: **21756482.2**

(22) Date of filing: **20.02.2021**

(51) International Patent Classification (IPC):
***C07K 5/083*** *(2006.01)* ***C07K 5/093*** *(2006.01)*
***C07K 5/103*** *(2006.01)* ***A61K 31/704*** *(2006.01)*
***A61P 35/00*** *(2006.01)*

(52) Cooperative Patent Classification (CPC):
**A61K 31/555; A61K 31/704; A61K 39/395; A61K 47/64; A61K 47/65; A61P 35/00; C07F 15/00; C07K 5/0804; C07K 5/0819; C07K 5/1005**

(86) International application number:
**PCT/CN2021/077056**

(87) International publication number:
**WO 2021/164765 (26.08.2021 Gazette 2021/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.02.2020 CN 202010106067**

(71) Applicant: **Yafei Shanghai Biolog Medicine Science & Technology Co., Ltd.**
**Zhangjiang High-tech Park**
**Pudong**
**Shanghai 201203 (CN)**

(72) Inventors:
- **LIU, Chen**
**Shanghai 201203 (CN)**
- **LIU, Yuan**
**Shanghai 201203 (CN)**
- **WANG, Haiyang**
**Shanghai 201203 (CN)**

(74) Representative: **Bjerkén Hynell KB**
**Tulegatan 53**
**113 53 Stockholm (SE)**

(54) **PREPARATION AND USE OF IMMUNOSTIMULATORY COUPLING COMPLEX WHICH IS DELIVERED AND ACTIVATED IN TARGETED MANNER**

(57) The present disclosure provides for the preparation and use of immunostimulatory conjugate complexes for targeted delivery and activation. In particular, the present disclosure provides compounds represented by the formula MI-S-C-A for use as linker, and drug-linked pharmaceutical compounds represented by the formula MI-S-C-A-D. The pharmaceutical compounds of the present disclosure have improved water solubility, reduced cytotoxicity, and enhanced pharmaceutical activity.

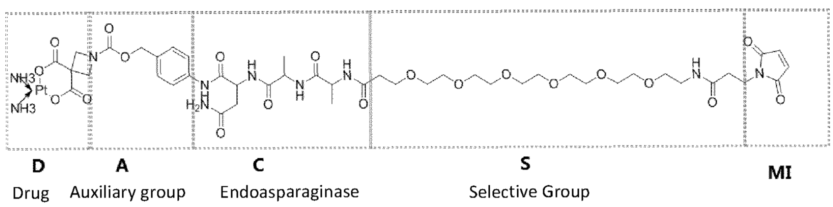


Figure 2

EP 4 108 675 A1

## Description

### TECHNICAL FIELD

The disclosure relates to an antitumor drug compound, in particular, to the preparation and use of an immunostimulatory conjugated complex for targeted delivery and activation.

### BACKGROUND

Legumain was first identified in legume seeds as an asparagine endopeptidase, a member of the C13 family of cysteine proteases. Legumain can process storage proteins during seed germination. The subsequent discovery of Legumain in parasites and mammals including humans demonstrated that this protease is highly conserved. In 1997, the pig source Legumain was first cloned and identified. Legumain is highly expressed in most solid tumors. The differential expression of legumain in normal and tumor tissues makes it an ideal target for tumor therapy. Legumain is an endopeptidase that specifically cleaves the peptide bond at the C-terminus of asparagine on the peptide chain under weakly acidic conditions. CN 201210573744.3 discloses a polypeptide doxorubicin derivative with targeted activation of aspartase, which releases Leudoxorubicin compound in tumors by cleaving a tetrapeptide group (linker) by Legumain.

### BRIEF SUMMARY

Through further compound screen and biological system research, this disclosure develops a chemical modify linker, which can further enhance the activation efficiency. In addition, the chemical modified linker of the present disclosure can enhance the selectivity of the conjugated drug to immune cells, produce immunotherapeutic enhancement properties in therapy, and enhance synergistic efficacy in combination with the PD-1 antibody.

The technical problem to be solved by the disclosure is to create a coupling linker with high efficiency and specific selection. Previous studies have found that asparagine endopeptidases preferentially recognize the substrate peptide sequence of the tetrapeptide and cleave the amide bonds between Asn and other residues. The idea of improving the activation efficiency is as follows: The mechanism of asparagine endopeptidase was further studied by synthesizing a large number of structurally different compounds at both ends of a tripeptide (e.g. AAN). According to the crystal structure of asparagine endopeptidase (Figure 1), since the active center of asparagine endopeptidase is located at the bottom of the invagination, the activation of the substrate peptide requires the enzyme active center close to the bottom; The C189 at the S1 position of the asparagine endopeptidase in the figure attack and cuts off the Asn peptide chain, and the adjacent position S2, S3, S4 and S1 are combined with that substrate to determine the digestion efficiency, and S2 and S3 correspond to Ala-Ala amino acid of the substrate peptide. Considering the possible interaction or steric hindrance caused by connecting compounds such as doxorubicin and MI group, this experiment further synthesized and screened a large number of chemical modification structures at both ends of substrate tripeptide. After the synthesized compounds with different groups are connected with MI and adriamycin, the activation efficiency is screened through tumor tissues or asparagine endopeptidase, so that we obtained a new compound coupling body with mutual structure-activity relationship through optimization. A schematic representation of this structure is shown in figure 2 and includes the MI group, the selective group S, the asparagine endopeptidase cleaved tripeptide group C, the auxiliary group A and the drug to be coupled. The added groups of the disclosure bring new functions: In addition to enhancing the activity of asparagine endopeptidase on the conjugated pharmaceutical compound (D), the physical properties and biological functions of the pharmaceutical compound are improved. The drug compound provided by the present disclosure is hydrophilic, and its cell membrane permeability is changed, so it is the most suitable compound for drug development. In addition, this disclosure also found that the pharmaceutical compound of the formula (II) has cell selectivity, can be specifically phagocytized by tumor-associated macrophage, and attack or inhibit tumor-associated macrophages and MDSC cells, so that the inhibition effect of the tumor-associated macrophages on immunity is relieved, and immunotherapy is promoted. The present disclosure also found that when coupled with doxorubicin, the length of the S group affects the activation efficiency, i.e., the longer the chain length of S, due to the relationship between the steric hindrance is not conducive to the binding of the compound to the enzyme, the activation efficiency is reduced.

Human serum albumin (HSA) is a small globular protein consisting of 585 amino acids (66- 69 kd), with many charged residues (e.g. lysine, aspartic acid, and groups without prosthetic groups or carbohydrates), and a small number of tryptophan or methionine residues. The compound of that formula (II) is couple with 34-position cysteine coupled with human serum albumin to form a macromolecular drug; it has been found experimentally that the albumin covalently coupled compounds of formula (II) or EMC-AANL-DOX of the present disclosure have reduced toxicity, improved drug stability and therapeutic efficacy.

In summary, the linker of formula (I) and the pharmaceutical compound of formula (II) of the present disclosure improve the activation efficiency, enhance the selectivity of immune cells, tissue selectivity, appropriate water solubility

and lipid solubility, and drug stability.

Accordingly, that present disclosure provides a compound of formula (I)(linker) and a pharmaceutical compound of formula (II)(conjugate) as described herein, and pharmaceutically acceptable salts thereof.

The present disclosure also provides a platinum derivative of the following structure or a pharmaceutically acceptable salt thereof:

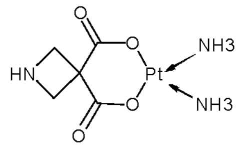

。

The disclosure also provides a pharmaceutical compound shown in the formula (II) or a pharmaceutically acceptable salt thereof which is covalently connected with albumin, and EMC-AANL-DOX which is covalently connected with albumin; preferably, the albumin is linked to the MI or EMC moiety of formula (II) via its cysteine residue at position 36.

The disclosure also provide a pharmaceutical composition which comprises that compound shown in the formula (II) or the pharmaceutically acceptable salt thereof, the platinum derivative or the pharmaceutically acceptable salt thereof, the pharmaceutical compound shown in the formula (II) covalently linked with albumin or the pharmaceutically acceptable salt thereof, or EMC-AANL-DOX covalently linked with albumin or the pharmaceutically acceptable salt thereof, and a pharmaceutically acceptable carrier.

The disclosure also provide the formula (II) or a pharmaceutically acceptable salt thereof, the platinum derivative or a pharmaceutically acceptable salt thereof, the pharmaceutical compound shown in the formula (II) covalently linked with albumin or the pharmaceutically acceptable salt thereof, or EMC-AANL-DOX covalently linked with albumin or pharmaceutically acceptable salts thereof in the preparation of drugs for treating or preventing cancer, fatty liver (including alcoholic and non-alcoholic fatty liver), steatohepatitis, fatty liver disease, liver fibrosis, liver inflammation and steatosis of liver cell injury; Preferably, the cancer is a solid cancer or a hematological tumor, preferably a cancer of the bladder, brain, breast/mammary gland, cervix, colon, rectum, esophagus, kidney, liver, lung, nasopharynx, pancreas, prostate, skin, stomach, uterus, ovary, testis and hematological sites.

The disclosure also provides an application of the compound shown in the formula (I) in enhancing the water solubility of a compound medicament, reducing the toxicity of the medicament, improving the curative effect of the medicament and/or improving the selectivity of the medicament to immune cells, or an application of the compound in preparing a medicament with improved water solubility, reduced toxicity of the medicament, improved curative effect of the medicament and/or improved selectivity of the medicament to immune cells, or an application of the compound in preparing a medicament molecule for delivering the medicament to liver.

The disclosure also provides an application of EMC-AANL-DOX compound as shown in the following formula or a medicament thereof coupled with albumin (preferably, covalently linked with the EMC part through the cysteine residue at the 36th position of albumin) in the preparation of a medicament for treating liver cancer, and an application of EMC-AANL-DOX compound and anti-PD-1 antibody and/or anti-PD-L1 antibody in the preparation of a medicament for combined treatment of tumors:

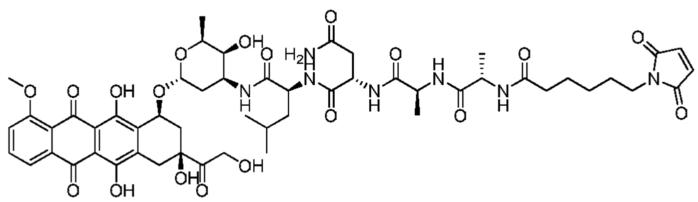

。

The disclosure also provides the formula (II) or a pharmaceutically acceptable salt thereof, a platinum derivative or a pharmaceutically acceptable salt thereof, the pharmaceutical compound shown in the formula (II) covalently linked with albumin or the pharmaceutically acceptable salt thereof, or EMC-AANL-DOX covalently linked with albumin or the pharmaceutically acceptable salt thereof in the preparation of medicaments for inhibiting immunosuppressive cells, inhibiting tumor-associated macrophages, inhibiting MDSC cells, inhibiting angiogenesis, promoting antitumor immunity and/or promoting T lymphocyte proliferation.

The disclosure also provides an application of that compound shown in the formula (II) or the pharmaceutically acceptable salt thereof, the platinum derivative or the pharmaceutically acceptable salt thereof, the pharmaceutical compound shown in the formula (II) covalently linked with albumin or the pharmaceutically acceptable salt thereof, or EMC-AANL-DOX covalently linked with albumin or the pharmaceutically acceptable salt thereof and the anti-PD-1 antibody in the preparation of a medicament for combined treatment of tumors.

## BRIEF DESCRIPTION OF THE DRAWINGS

Figure 1: Schematic representation of the crystal structure and substrate of asparagine endopeptidase.

Figure 2: Schematic representation of an immunostimulatory conjugate complex for targeted delivery and activation.

Figure 3: Comparison of cleavage kinetics of preferred compounds.

Figure 4: Isolation of mouse bone marrow mononuclear cells and induction of M2 macrophage differentiation.

Figure 5: Cytotoxicity experiments of compounds on CD8+ T cells.

Figure 6: Cytotoxicity experiments of compounds on M2 macrophages.

Figure 7: Efficacy experiments of compounds on HT1080 tumors.

Figure 8: EMC-AANL-DOX has high distribution characteristics in liver and liver cancer tissues.

Figure 9: QHL-087-DOX has high distribution characteristics in liver and liver cancer tissues.

Figure 10: Combination of QHL-087-DOX with anti-PD-1 antibody in the treatment of hepatic in situ tumors.

Figure 11: EMC-AANL-DOX combined with anti-PD-1 antibody is more effective than lenvatinib combined with anti-PD-1 antibody in the treatment of liver in situ tumors.

Figure 12: The combination therapeutic effect of HSA-EMC-AANL-DOX, QHL-087-DOX and anti-PD-1 antibody.

Figure 13: In vitro cytotoxicity experiments of N-CBP.

Figure 14: Cytotoxicity experiments of HSA-QHL-095-N-CBP.

Figure 15: Single-agent and combined treatment effects of HSA-QHL-095-N-CBP with anti-PD-1 antibody.

## DETAILED DESCRIPTION OF THE DISCLOSURE

The technical scheme of the present disclosure will be further described below in conjunction with specific embodiments. I. Linker Compound

The disclosure provides a compound with a structure shown in that following formula (I), which can be use as a linker and can enhance the water solubility of the compound medicament, reduce the toxicity of the medicament, improve the curative effect of the medicament and/or improve the selectivity of the medicament to immune cells when being linked with an interested medicament (such as an anticancer compound):

MI-S-C-A (I)

In this formula, MI is maleimide group; S is a group for improving enzyme digestion efficiency or selectivity; C is a proteolytic enzyme cleavable amino acid linker; and A is auxiliary linker.

An exemplary MI is a maleimide group of the formula:

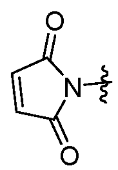

Among them, the wavy line indicates the connection position with S.

In some embodiments, S in formula (I) is represented as S1-S2-S3, wherein S1 is selected from:

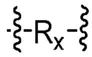

,

wherein $R_x$ is absent or selected from: $C_{1-6}$ alkylene, $C_{1-6}$ alkyleneamino, $C_{1-6}$ alkylenecarboxyl and $C_{1-6}$ alkylenecarbonylamino, the wavy line indicating the position of attachment to the adjacent moiety; S2 is absent or-$[(CH_2)_pO]_q$-; wherein p is an integer of 1-4, preferably 2; q is an integer of 0-15, preferably 1-15, more preferably 2-6; S3 is absent or selected from polar amino acid residues, such as: Glu, Asp, Gly, Ala, Val, Leu, Ile, Met, Phe, Trp, Ser, Thr, Cys, Tyr, Asn, Gln, Lys, Arg and His, preferably Glu and Asp.

It should be understood that there is at least one of S1, S2 and S3.

Preferably, MI, S1, S2, S3, C and A are connected to each other in any of the following ways:

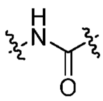 、 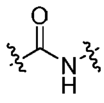 、 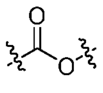 、 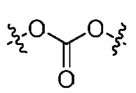 、 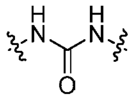 、 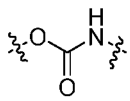 、 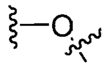

and 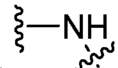 ;

wherein the wavy line represents adjacent connecting parts; Preferably, S is linked to C through the following group:

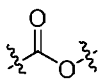 、 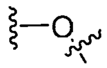 and 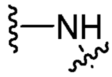 .

In some embodiments, S is -$R_1$-$[(CH_2)_pO]_q$-$R_2$-$R_3$-, wherein $R_1$ is linked to MI, is absent or is selecte from $C_{1-6}$ alkylene or $C_{1-6}$ alkylenecarbonylamino; $R_2$ is selecte from $C_{1-6}$ alkylene; $R_3$ is selected from -C(O)O-, -NH-, -O- or -C(O)-$R_4$, wherein $R_4$ is an amino acid residue selected from Glu, Asp, Gly, Ala, Val, Leu, Ile, Met, Phe, Trp, Ser, Thr, Cys, Tyr, Asn, Gln, Lys, Arg and His, and preferably Glu and Asp, and $R_4$ forms an amide bond with the -C(O)- via its amino group; p is an integer of 1-4; q is an integer of 0-15, preferably 1-15, more preferably 2-6. Preferably, $R_1$ is absent, p is 2 or 3, q is an integer of 1-15, preferably 2-6, $R_2$ is $C_{1-4}$ alkylene, and $R_3$ is selected from -C(O)O-, -NH- and -O-. In some embodiments, it is preferred that $R_1$ is absent, q is 0, $R_2$ is $C_{1-6}$ alkylene, $R_3$ is -C(O)-$R_4$, $R_4$ is preferably Glu and Asp, and $R_4$ form an amide bond with that -C(O)- through its amino group. In some embodiments, it is preferred that $R_1$ is $C_{1-6}$ alkylenecarbonylamino, p is 2 or 3, q is an integer of 1-15, preferably 2-6, $R_2$ is $C_{1-4}$ alkylene, $R_3$ is -C(O)-$R_4$, $R_4$ is preferably Glu and Asp, and $R_4$ forms an amide bond with that -C(O)- via its amino group.

Exemplary MI-S was selected from:

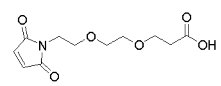 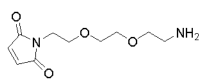 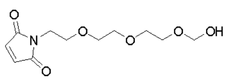

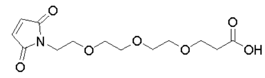 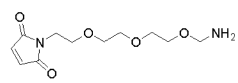 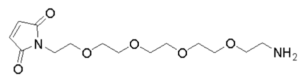

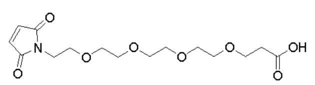 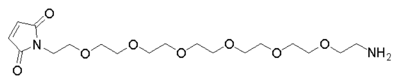

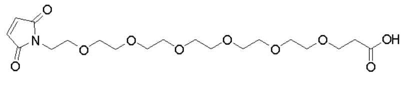 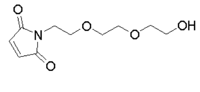

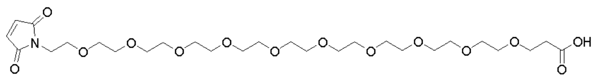
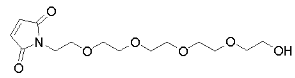
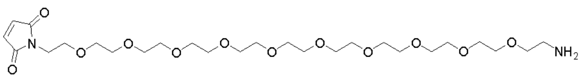
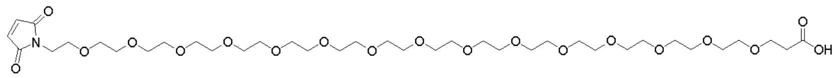
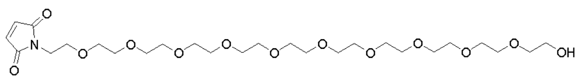
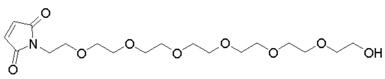
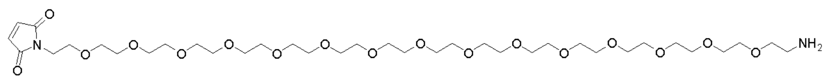
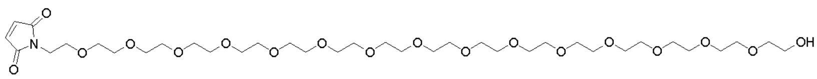
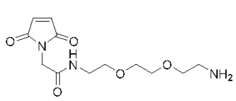
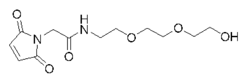
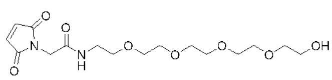
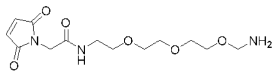
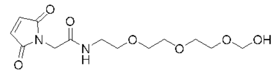
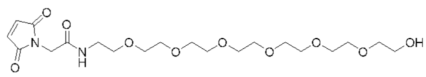
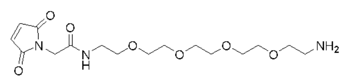
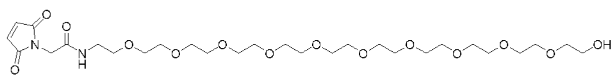
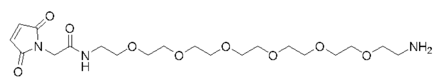
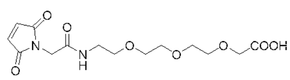
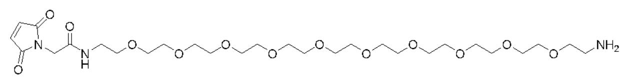
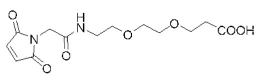
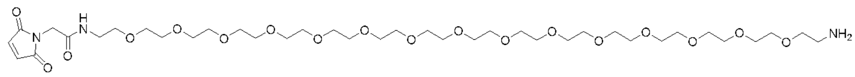
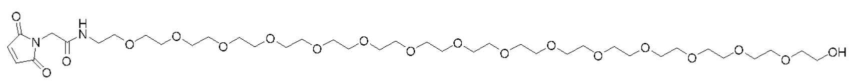
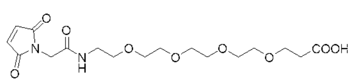
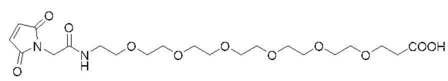

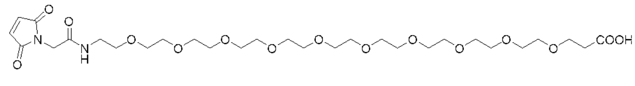

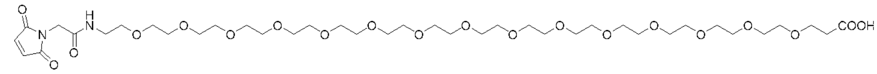

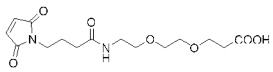

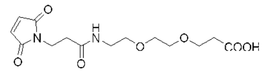

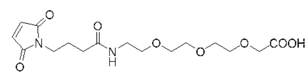

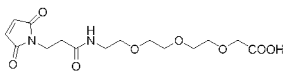

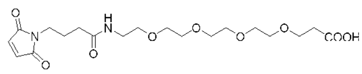

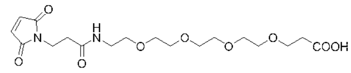

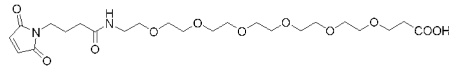

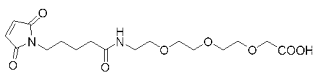

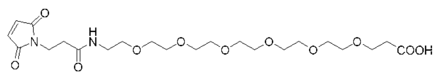

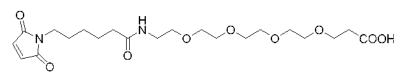

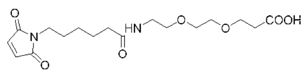

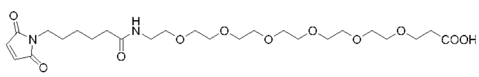

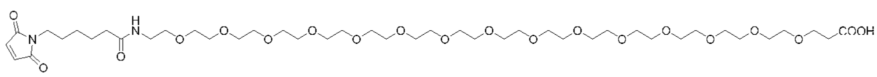

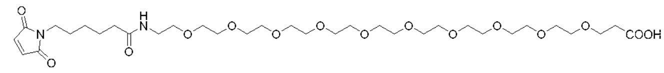

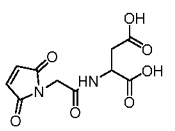

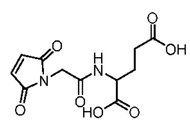

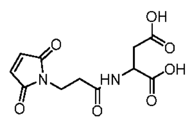

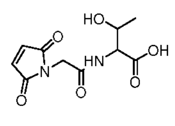

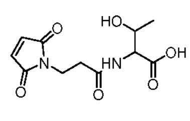

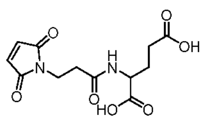

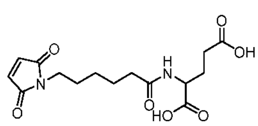

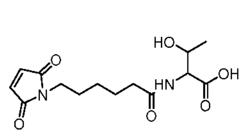

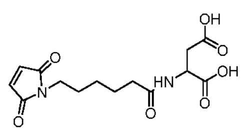

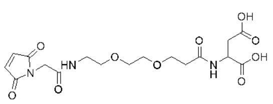

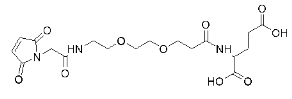

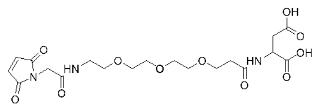

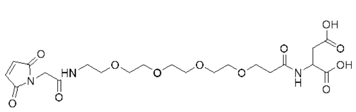

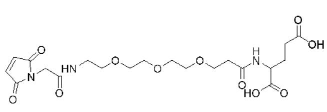

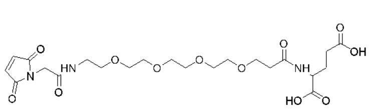

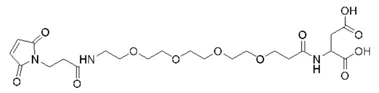 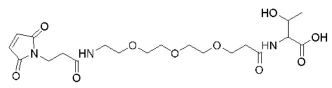 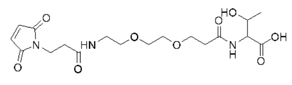

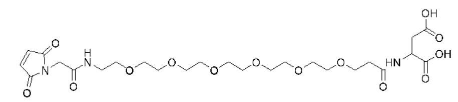 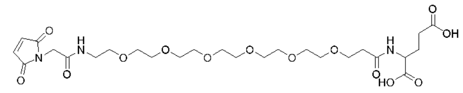

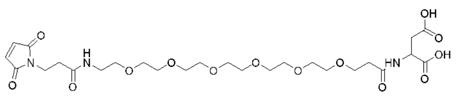 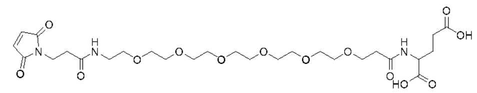

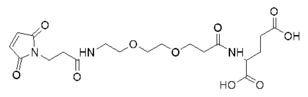 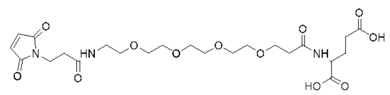 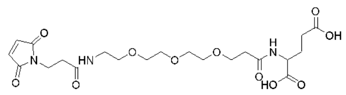 ;

Preferably, C linked to any of the above MI-S is AAN and A is any of the structures described below.

Preferably, in that compound of formula (I) according to the disclosure, C is selected from a group which is cleaved by asparagine endopeptidase expressed in the tumor microenvironment and which group comprises an Asn residue. In some embodiments, C is $X_1X_2X_3$, wherein $X_1$ is selected from that group consist of Ala, Thr, Val, and Asn of the L or D forms; $X_2$ is selected from that group consisting of Ala, Thr, Val, and Ile of the L or D form; $X_3$ is Asn, preferably not D-Asn. Exemplary C is selected from: Ala-Ala-Asn, Thr-Ala-Asn, Val-Ala-Asn, Asn-Ala-Asn, Thr-Thr-Asn, Val-Thr-Asn, Asn-Thr-Asn, Ala-Val-Asn, Thr-Val-Asn, Val-Val-Asn, Asn-Val-Asn, Ala-Ile-Asn, Thr-Ile-Asn, Val-Ile-Asn, Asn-Ile-Asn, Ala-Thr-Asn, D-Thr-L-Val-L-Asn, D-Thr-L-Ala-L-Asn, D-Ala-L-Val-L-Asn, L-Thr-D-Val-L-Asn, L-Thr-D-Ala-L-Asn, L-Ala-D-Val-L-Asn, D-Thr-D-Val-L-Asn, D-Thr-D-Ala-L-Asn, D-Ala-D-Val-L-Asn. In some particularly preferred embodiments, C is AAN.

In that compound of formula (I) of the present disclosure, A is preferably selected from the group consist of Leu, PABC-OH and PABC-NH2, the structures of which are shown in the following formulas respectively:

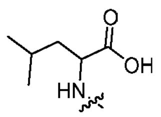 (Leu), 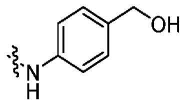 (PABC-OH), and 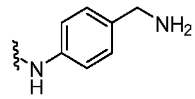 (PABC-NH2);

Where the wavy line indicates the location of the connection to C.

In some embodiments, S and A in that compounds of formula (I) of the present disclosure are selected from any one of the following groups 1-137 [wherein "2peg" represents $-(CH_2CH_2O)_2-$, 3peg represent $-(CH_2CH_2O)_3-$, 4peg represents - $(CH_2CH_2O)_4-$, 6peg represents $-(CH_2CH_2O)_6-$, and so on]:

| Compound No. | S | | | A |
|---|---|---|---|---|
| | S1 | S2 | S3 | |
| QHL-001 | / | 2peg | / | $PABC-NH_2$ |
| QHL-002 | / | 3peg | / | $PABC-NH_2$ |
| QHL-003 | / | 4peg | / | $PABC-NH_2$ |
| QHL-004 | / | 6peg | / | $PABC-NH_2$ |
| QHL-005 | / | 2peg | / | PABC-OH |
| QHL-006 | / | 3peg | / | PABC-OH |
| QHL-007 | / | 4peg | / | PABC-OH |

(continued)

| Compound No. | S | | | A |
|---|---|---|---|---|
| | S1 | S2 | S3 | |
| QHL-008 | / | 6peg | / | PABC-OH |
| QHL-009 | / | 2peg | / | Leu |
| QHL-010 | / | 3peg | / | Leu |
| QHL-011 | / | 4peg | / | Leu |
| QHL-012 | / | 6peg | / | Leu |
| QHL-013 | / | 2peg | Glu | PABC-$NH_2$ |
| QHL-014 | / | 3peg | Glu | PABC-$NH_2$ |
| QHL-015 | / | 4peg | Glu | PABC-$NH_2$ |
| QHL-016 | / | 6peg | Glu | PABC-$NH_2$ |
| QHL-017 | / | 2peg | Glu | PABC-OH |
| QHL-018 | / | 3peg | Glu | PABC-OH |
| QHL-019 | / | 4peg | Glu | PABC-OH |
| QHL-020 | / | 6peg | Glu | PABC-OH |
| QHL-021 | / | 2peg | Glu | Leu |
| QHL-022 | / | 3peg | Glu | Leu |
| QHL-023 | / | 4peg | Glu | Leu |
| QHL-024 | / | 6peg | Glu | Leu |
| QHL-025 | / | 2peg | Asp | PABC-$NH_2$ |
| QHL-026 | / | 3peg | Asp | PABC-$NH_2$ |
| QHL-027 | / | 4peg | Asp | PABC-$NH_2$ |
| QHL-028 | / | 6peg | Asp | PABC-$NH_2$ |
| QHL-029 | / | 2peg | Asp | PABC-OH |
| QHL-030 | / | 3peg | Asp | PABC-OH |
| QHL-031 | / | 4peg | Asp | PABC-OH |
| QHL-032 | / | 6peg | Asp | PABC-OH |
| QHL-033 | / | 2peg | Asp | Leu |
| QHL-034 | / | 3peg | Asp | Leu |
| QHL-035 | / | 4peg | Asp | Leu |
| QHL-036 | / | 6peg | Asp | Leu |
| QHL-037 | $-CH_2CH_2-CONH-$ | 2peg | Glu | PABC-$NH_2$ |
| QHL-038 | $-CH_2CH_2-CONH-$ | 2peg | Glu | PABC-OH |
| QHL-039 | $-CH_2CH_2-CONH-$ | 2peg | Glu | Leu |
| QHL-040 | $-CH_2CH_2-CONH-$ | 2peg | Asp | PABC-$NH_2$ |
| QHL-041 | $-CH_2CH_2-CONH-$ | 2peg | Asp | PABC-OH |
| QHL-042 | $-CH_2CH_2-CONH-$ | 2peg | Asp | Leu |
| QHL-043 | $-CH_2CH_2-CONH-$ | 3peg | Glu | PABC-$NH_2$ |
| QHL-044 | $-CH_2CH_2-CONH-$ | 3peg | Glu | PABC-OH |

(continued)

| Compound No. | S | | | A |
|---|---|---|---|---|
| | S1 | S2 | S3 | |
| QHL-045 | $-CH_2CH_2$-CONH- | 3peg | Glu | Leu |
| QHL-046 | $-CH_2CH_2$-CONH- | 3peg | Asp | PABC-$NH_2$ |
| QHL-047 | $-CH_2CH_2$-CONH- | 3peg | Asp | PABC-OH |
| QHL-048 | $-CH_2CH_2$-CONH- | 3peg | Asp | Leu |
| QHL-049 | $-CH_2CH_2$-CONH- | 4peg | Glu | PABC-$NH_2$ |
| QHL-050 | $-CH_2CH_2$-CONH- | 4peg | Glu | PABC-OH |
| QHL-051 | $-CH_2CH_2$-CONH- | 4peg | Glu | Leu |
| QHL-052 | $-CH_2CH_2$-CONH- | 4peg | Asp | PABC-$NH_2$ |
| QHL-053 | $-CH_2CH_2$-CONH- | 4peg | Asp | PABC-OH |
| QHL-054 | $-CH_2CH_2$-CONH- | 4peg | Asp | Leu |
| QHL-055 | $-CH_2CH_2$-CONH- | 6peg | Glu | PABC-$NH_2$ |
| QHL-056 | $-CH_2CH_2$-CONH- | 6peg | Glu | PABC-OH |
| QHL-057 | $-CH_2CH_2$-CONH- | 6peg | Glu | Leu |
| QHL-058 | $-CH_2CH_2$-CONH- | 6peg | Asp | PABC-$NH_2$ |
| QHL-059 | $-CH_2CH_2$-CONH- | 6peg | Asp | PABC-OH |
| QHL-060 | $-CH_2CH_2$-CONH- | 6peg | Asp | Leu |
| QHL-061 | $-CH_2CH_2CH_2$-CONH- | 2peg | Glu | PABC-$NH_2$ |
| QHL-062 | $-CH_2CH_2CH_2$-CONH- | 2peg | Glu | PABC-OH |
| QHL-063 | $-CH_2CH_2CH_2$-CONH- | 2peg | Glu | Leu |
| QHL-064 | $-CH_2CH_2CH_2$-CONH- | 2peg | Asp | PABC-$NH_2$ |
| QHL-065 | $-CH_2CH_2CH_2$-CONH- | 2peg | Asp | PABC-OH |
| QHL-066 | $-CH_2CH_2CH_2$-CONH- | 2peg | Asp | Leu |
| QHL-067 | $-CH_2CH_2CH_2$-CONH- | 3peg | Glu | PABC-$NH_2$ |
| QHL-068 | $-CH_2CH_2CH_2$-CONH- | 3peg | Glu | PABC-OH |
| QHL-069 | $-CH_2CH_2CH_2$-CONH- | 3peg | Glu | Leu |
| QHL-070 | $-CH_2CH_2CH_2$-CONH- | 3peg | Asp | PABC-$NH_2$ |
| QHL-071 | $-CH_2CH_2CH_2$-CONH- | 3peg | Asp | PABC-OH |
| QHL-072 | $-CH_2CH_2CH_2$-CONH- | 3peg | Asp | Leu |
| QHL-073 | $-CH_2CH_2CH_2$-CONH- | 4peg | Glu | PABC-$NH_2$ |
| QHL-074 | $-CH_2CH_2CH_2$-CONH- | 4peg | Glu | PABC-OH |
| QHL-075 | $-CH_2CH_2CH_2$-CONH- | 4peg | Glu | Leu |
| QHL-076 | $-CH_2CH_2CH_2$-CONH- | 4peg | Asp | PABC-$NH_2$ |
| QHL-077 | $-CH_2CH_2CH_2$-CONH- | 4peg | Asp | PABC-OH |
| QHL-078 | $-CH_2CH_2CH_2$-CONH- | 4peg | Asp | Leu |
| QHL-079 | $-CH_2CH_2CH_2$-CONH- | 6peg | Glu | PABC-$NH_2$ |
| QHL-080 | $-CH_2CH_2CH_2$-CONH- | 6peg | Glu | PABC-OH |
| QHL-081 | $-CH_2CH_2CH_2$-CONH- | 6peg | Glu | Leu |

(continued)

| Compound No. | S | | | A |
|---|---|---|---|---|
| | S1 | S2 | S3 | |
| QHL-082 | $-CH_2CH_2CH_2-CONH-$ | 6peg | Asp | $PABC-NH_2$ |
| QHL-083 | $-CH_2CH_2CH_2-CONH-$ | 6peg | Asp | PABC-OH |
| QHL-084 | $-CH_2CH_2CH_2-CONH-$ | 6peg | Asp | Leu |
| QHL-085 | $-CH_2CH_2-CONH-$ | 2peg | / | $PABC-NH_2$ |
| QHL-086 | $-CH_2CH_2-CONH-$ | 2peg | / | PABC-OH |
| QHL-087 | $-CH_2CH_2-CONH-$ | 2peg | / | Leu |
| QHL-088 | $-CH_2CH_2-CONH-$ | 3peg | / | $PABC-NH_2$ |
| QHL-089 | $-CH_2CH_2-CONH-$ | 3peg | / | PABC-OH |
| QHL-090 | $-CH_2CH_2-CONH-$ | 3peg | / | Leu |
| QHL-091 | $-CH_2CH_2-CONH-$ | 4peg | / | $PABC-NH_2$ |
| QHL-092 | $-CH_2CH_2-CONH-$ | 4peg | / | PABC-OH |
| QHL-093 | $-CH_2CH_2-CONH-$ | 4peg | / | Leu |
| QHL-094 | $-CH_2CH_2-CONH-$ | 6peg | / | $PABC-NH_2$ |
| QHL-095 | $-CH_2CH_2-CONH-$ | 6peg | / | PABC-OH |
| QHL-096 | $-CH_2CH_2-CONH-$ | 6peg | / | Leu |
| QHL-097 | $-CH_2CH_2CH_2-CONH-$ | 2peg | / | $PABC-NH_2$ |
| QHL-098 | $-CH_2CH_2CH_2-CONH-$ | 2peg | / | PABC-OH |
| QHL-099 | $-CH_2CH_2CH_2-CONH-$ | 2peg | / | Leu |
| QHL-100 | $-CH_2CH_2CH_2-CONH-$ | 3peg | / | $PABC-NH_2$ |
| QHL-101 | $-CH_2CH_2CH_2-CONH-$ | 3peg | / | PABC-OH |
| QHL-102 | $-CH_2CH_2CH_2-CONH-$ | 3peg | / | Leu |
| QHL-103 | $-CH_2CH_2CH_2-CONH-$ | 4peg | / | $PABC-NH_2$ |
| QHL-104 | $-CH_2CH_2CH_2-CONH-$ | 4peg | / | PABC-OH |
| QHL-105 | $-CH_2CH_2CH_2-CONH-$ | 4peg | / | Leu |
| QHL-106 | $-CH_2CH_2CH_2-CONH-$ | 6peg | / | $PABC-NH_2$ |
| QHL-107 | $-CH_2CH_2CH_2-CONH-$ | 6peg | / | PABC-OH |
| QHL-108 | $-CH_2CH_2CH_2-CONH-$ | 6peg | / | Leu |
| QHL-109 | $-CH_2CH_2CH_2-CONH-$ | / | Glu | $PABC-NH_2$ |
| QHL-110 | $-CH_2CH_2CH_2-CONH-$ | / | Glu | PABC-OH |
| QHL-111 | $-CH_2CH_2CH_2-CONH-$ | / | Glu | Leu |
| QHL-112 | $-CH_2CH_2CH_2-CONH-$ | / | Asp | $PABC-NH_2$ |
| QHL-113 | $-CH_2CH_2CH_2-CONH-$ | / | Asp | PABC-OH |
| QHL-114 | $-CH_2CH_2CH_2-CONH-$ | / | Asp | Leu |
| QHL-115 | $-(CH_2)_6-CONH-$ | / | Glu | $PABC-NH_2$ |
| QHL-116 | $-(CH_2)_6-CONH-$ | / | Glu | PABC-OH |
| QHL-117 | $-(CH_2)_6-CONH-$ | / | Glu | Leu |
| QHL-118 | $-(CH_2)_6-CONH-$ | / | Asp | $PABC-NH_2$ |

(continued)

| Compound No. | S | | | A |
|---|---|---|---|---|
| | S1 | S2 | S3 | |
| QHL-119 | $-(CH_2)_6$-CONH- | / | Asp | PABC-OH |
| QHL-120 | $-(CH_2)_6$-CONH- | / | Asp | Leu |
| QHL-121 | $-(CH_2)_6$-CONH- | / | Gly | Leu |
| QHL-122 | $-(CH_2)_6$-CONH- | / | Ala | Leu |
| QHL-123 | $-(CH_2)_6$-CONH- | / | Val | Leu |
| QHL-124 | $-(CH_2)_6$-CONH- | / | Leu | Leu |
| QHL-125 | $-(CH_2)_6$-CONH- | / | Ile | Leu |
| QHL-126 | $-(CH_2)_6$-CONH- | / | Met | Leu |
| QHL-127 | $-(CH_2)_6$-CONH- | / | Phe | Leu |
| QHL-128 | $-(CH_2)_6$-CONH- | / | Trp | Leu |
| QHL-129 | $-(CH_2)_6$-CONH- | / | Ser | Leu |
| QHL-130 | $-(CH_2)_6$-CONH- | / | Thr | Leu |
| QHL-131 | $-(CH_2)_6$-CONH- | / | Cys | Leu |
| QHL-132 | $-(CH_2)_6$-CONH- | / | Tyr | Leu |
| QHL-133 | $-(CH_2)_6$-CONH- | / | Asn | Leu |
| QHL-134 | $-(CH_2)_6$-CONH- | / | Gln | Leu |
| QHL-135 | $-(CH_2)_6$-CONH- | / | Lys | Leu |
| QHL-136 | $-(CH_2)_6$-CONH- | / | Arg | Leu |
| QHL-137 | $-(CH_2)_6$-CONH- | / | His | Leu |
| QHL-138 | $-(CH_2)_6$-CONH- | / | / | Leu |
| QHL-139 | $-(CH_2)_6$-CONH- | / | / | PABC-$NH_2$ |
| QHL-140 | $-(CH_2)_6$-CONH- | / | / | PABC-OH |
| QHL-141 | $-(CH_2)_4$-CONH- | / | / | Leu |
| QHL-142 | $-(CH_2)_4$-CONH- | / | / | PABC-$NH_2$ |
| QHL-143 | $-(CH_2)_4$-CONH- | / | / | PABC-OH |
| QHL-144 | $-CH_2CH_2$-CONH- | / | / | Leu |
| QHL-145 | $-CH_2CH_2$-CONH- | / | / | PABC-$NH_2$ |
| QHL-146 | $-CH_2CH_2$-CONH- | / | / | PABC-OH |
| QHL-147 | / | 12peg | / | Leu |
| QHL-148 | / | 12peg | / | PABC-$NH_2$ |
| QHL-149 | / | 12peg | / | PABC-OH |
| QHL-150 | $-(CH_2)_6$-CONH- | / | / | / |
| QHL-151 | $-(CH_2)_4$-CONH- | / | / | / |
| QHL-152 | $-CH_2CH_2$-CONH- | / | / | / |
| QHL-153 | / | 2peg | / | / |
| QHL-154 | / | 3peg | / | / |
| QHL-155 | / | 4peg | / | / |

(continued)

| Compound No. | S | | | A |
|---|---|---|---|---|
| | S1 | S2 | S3 | |
| QHL-156 | / | 6peg | / | / |
| QHL-157 | $-CH_2CH_2-CONH-$ | 2peg | / | / |
| QHL-158 | $-CH_2CH_2-CONH-$ | 3peg | / | / |
| QHL-159 | $-CH_2CH_2-CONH-$ | 4peg | / | / |
| QHL-160 | $-CH_2CH_2-CONH-$ | 6peg | / | / |
| QHL-161 | $-(CH_2)_6-CONH-$ | / | Glu | / |
| QHL-162 | $-(CH_2)_6-CONH-$ | / | Asp | / |

Preferably, in that compound of formula (I) according to the disclosure, MI is a maleimide group, S and A are any one of the group QHL-001 to QHL-162, and C is AAN.

Particularly preferred compounds of formula (I) according to the disclosure (linker) are selected from any one of QHL-005, QHL-006, QHL-008, QHL-086, QHL-087, QHL-089, QHL-090, QHL-092, QHL -093, QHL-095, QHL-096, QHL-098, QHL-099, QHL-101, QHL-102, QHL-104, QHL-105, QHL-107, QHL-108, QHL-116, QHL-119 , QHL-138, QHL-140, QHL-141, QHL-143, QHL-144, QHL-146, QHL-147, QHL-150, QHL-153, QHL-154, QHL-155, QHL-156, QHL-157, QHL-158, QHL-159, QHL-160, QHL-161 and QHL-162, more preferably any one of QHL-086, QHL-087, QHL-089 and QHL-090.

II. Pharmaceutical Compounds

The present disclosure provides a compound (conjugate) represented by the following formula (II) or a pharmaceutically acceptable salt thereof:

MI-S-C-A-D (II)

wherein MI, S, C and A form a linker compound according to any embodiment of the present disclosure; D is a drug, preferably an anticancer compound.

In formula II, when A is a linking group, it is selected from:

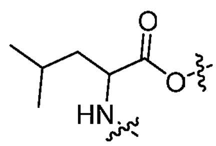

Leu,

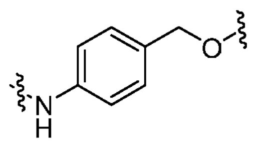

PABC-OH,

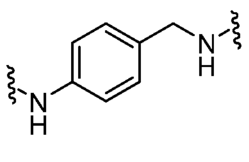

$PABC-NH_2$,

Wherein, the wavy lines indicate the connection locations to C and D. Preferably, it is linked to C via -NH-.

Preferably, D is selected from that group consisting of resiquimod, prednisone, triiodothyronine(T3), doxorubicin, daunorubicin, epirubicin, methotrexate, fludarabine, gemcitabine, cytarabine, melphalan, nimustine, mitoxantrone, mitomycin, camptothecin, 10-hydroxycamptothecin, topotecan, floxuridine, doxifluridine, etoposide, fludarabine, capecitabine, vincristine, epothilone B, paclitaxel, docetaxel, dabrafenib, dovitinib, motesanib, compound a, compound b and a platinum derivative represented by that following formula:

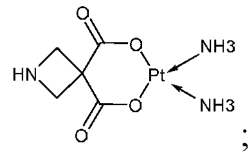

;

wherein, that structure of the compound a and the compound b are as follows:

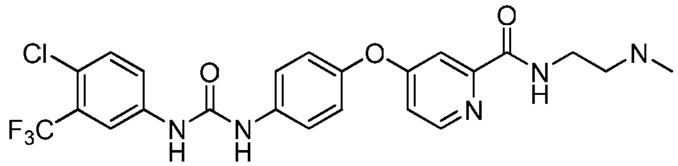

Compound a

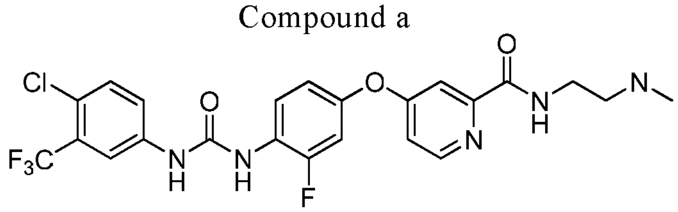

Compound b

More preferably, D is selected from that group consisting of daunorubicin, dovitinib, epirubicin, compound a, compound b, mitomycin, dabrafenib, motesanib, resiquimod, prednisone, and T3. Preferably, the compounds of formula (I) according to the disclosure (linker) for linking to these drugs (D) are selected from any one of QHL-005, QHL-006, QHL-008, QHL-086, QHL-087, QHL-089, QHL-090, QHL-092, QHL-093, QHL-095, QHL-096, QHL-098, QHL-099, QHL-101, QHL-102, QHL-104, QHL-105, QHL-107, QHL-108, QHL-116, QHL-119, QHL-138, QHL-140, QHL-141, QHL-143, QHL-144, QHL-146, QHL-147, QHL-150, QHL-153, QHL-154, QHL-155, QHL-156, QHL-157, QHL-158, QHL-159, QHL-160, QHL-161, and QHL-162, and more preferably any one of QHL-086, QHL-087, QHL-089, and QHL-090.

Preferably, A and D are linked in any of the following ways:

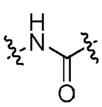 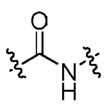 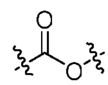 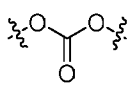 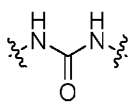 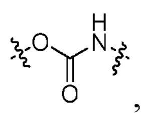 ,

The wavy line represents adjacent connecting parts.

More preferably, A is linked to D by -CO-NH-, wherein that carbonyl group is linked to or part of A (such as when A is Leu) and the amino group is linked to or part of D. Typically, the connection position of the drug compound to A does not affect the biological activity of the drug, e.g., the connection position is remote from the active center of the drug compound.

Preferably, the pharmaceutical compound of formula (II) according to the present disclosure is selected from:

| No. | Linker | D | Compound structure |
|---|---|---|---|
| QHL-140-N-CBP | QHL-140 | 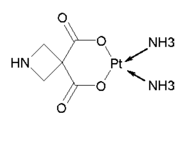 | 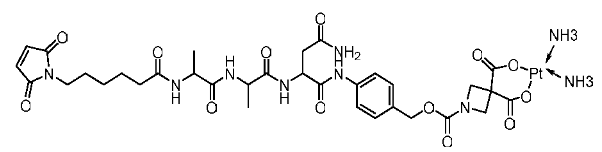 |

(continued)

| No. | Linker | D | Compound structure |
|---|---|---|---|
| QHL-143-N-CBP | QHL-143 | 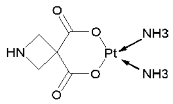 | 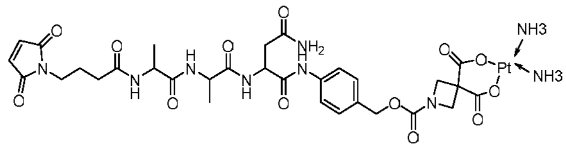 |
| QHL-095-N-CBP | QHL-095 | 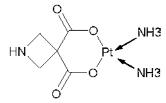 | 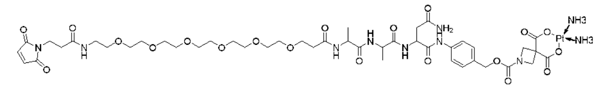 |
| QHL-092-N-CBP | QHL-092 | 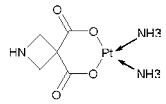 | 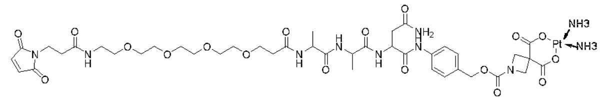 |
| QHL-089-N-CBP | QHL-089 | 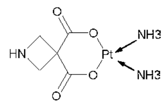 | 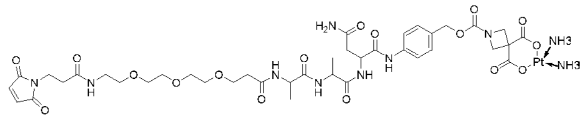 |
| QHL-107-N-CBP | QHL-107 | 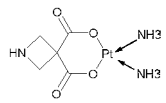 | 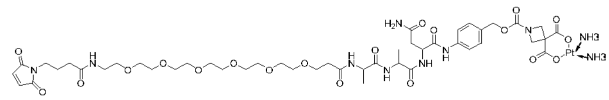 |
| QHL-104-N-CBP | QHL-104 | 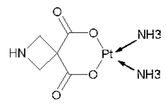 | 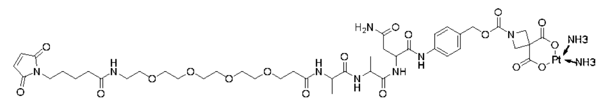 |
| QHL-101-N-CBP | QHL-101 | 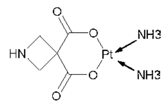 | 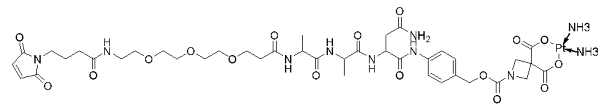 |
| QHL-098-N-CBP | QHL-098 | 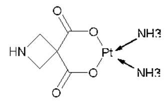 | 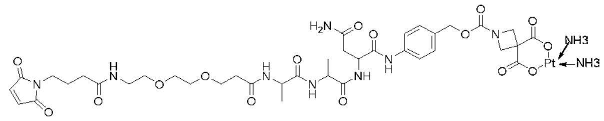 |
| QHL-146-N-CBP | QHL-146 | 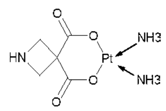 | 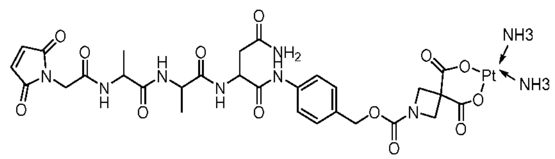 |
| QHL-086-N-CBP | QHL-086 | 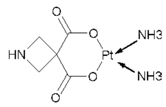 | 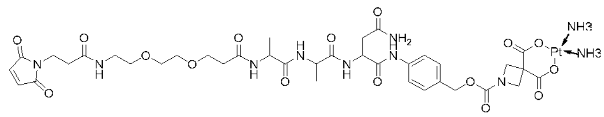 |
| QHL-086-dovitinib | QHL-086 | dovitinib | 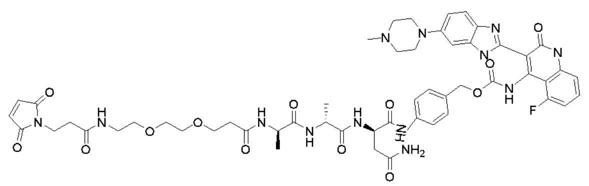 |

(continued)

| No. | Linker | D | Compound structure |
|---|---|---|---|
| QHL-089-dovitinib | QHL-089 | dovitinib | 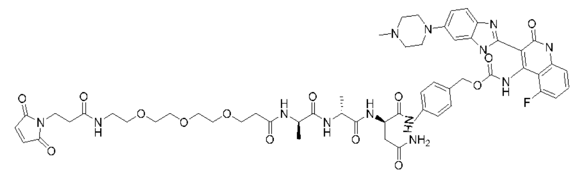 |
| QHL-086-epirubicin | QHL-086 | epirubicin | 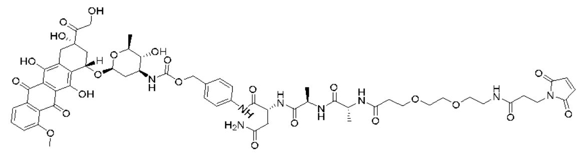 |
| QHL-089-epirubicin | QHL-089 | epirubicin | 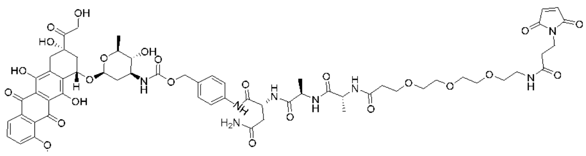 |
| QHL-086-compound a | QHL-086 | compound a | 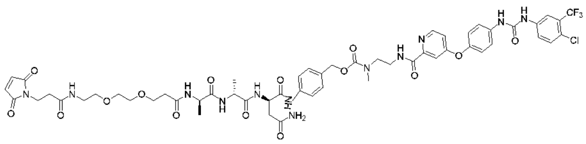 |
| QHL-089-compound a | QHL-089 | compound a | 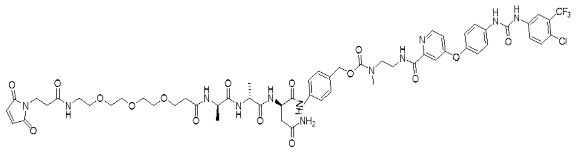 |
| QHL-086-compound b | QHL-086 | compound b | 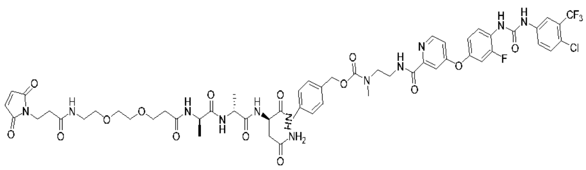 |
| QHL-089-compound b | QHL-089 | compound b | 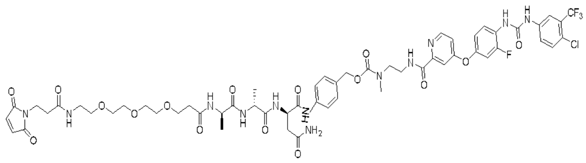 |
| QHL-086-mitomycin | QHL-086 | mitomycin | 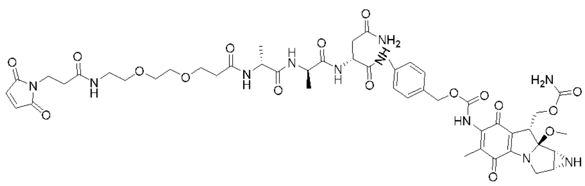 |
| QHL-089-mitomycin | QHL-089 | mitomycin | 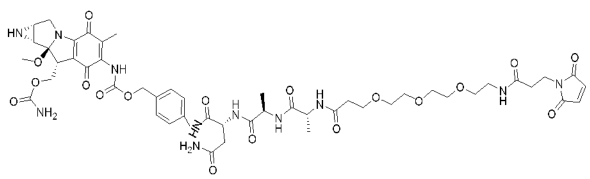 |

(continued)

| No. | Linker | D | Compound structure |
|---|---|---|---|
| QHL-086-dabrafenib | QHL-086 | dabrafenib | 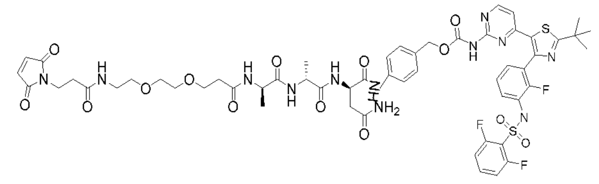 |
| QHL-089-dabrafenib | QHL-089 | dabrafenib | 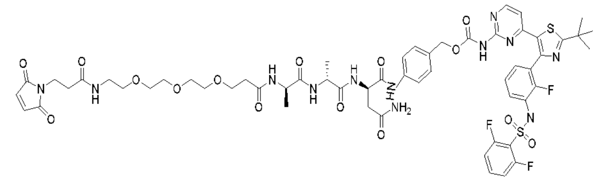 |
| QHL-086-motesanib | QHL-086 | motesanib | 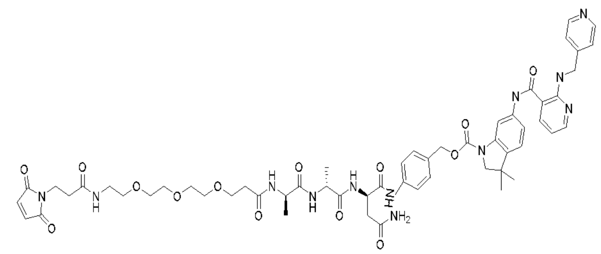 |
| QHL-089-motesanib | QHL-089 | motesanib | 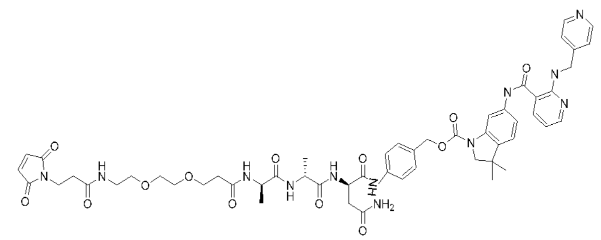 |
| QHL-138-N-CBP | QHL-138 | 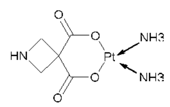 | 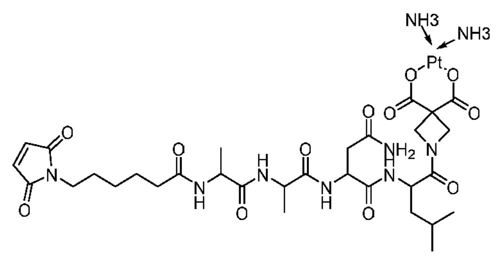 |
| QHL-141-N-CBP | QHL-141 | 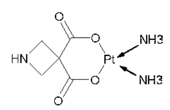 | 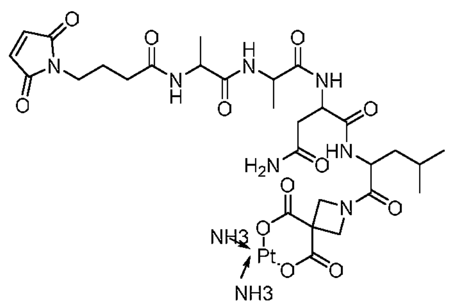 |

(continued)

| No. | Linker | D | Compound structure |
|---|---|---|---|
| QHL-096-N-CBP | QHL-096 | 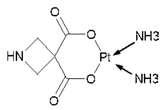 | 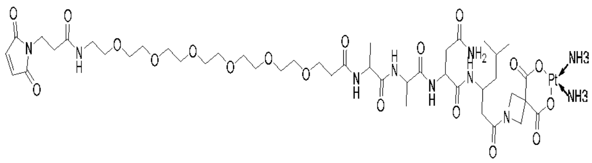 |
| QHL-093-N-CBP | QHL-093 | 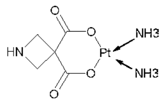 | 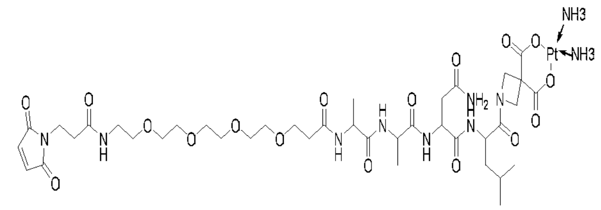 |
| QHL-090-N-CBP | QHL-090 | 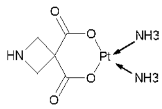 | 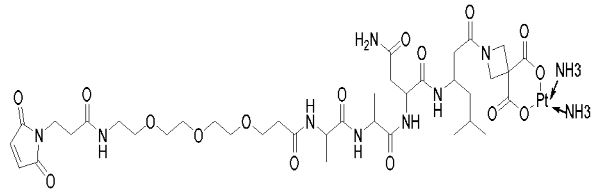 |
| QHL-108-N-CBP | QHL-108 | 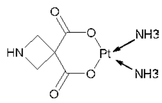 | 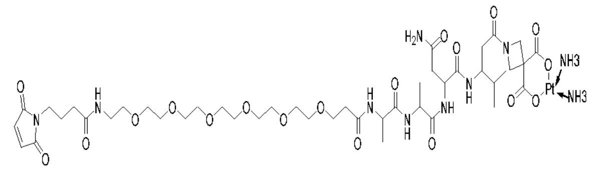 |
| QHL-105-N-CBP | QHL-105 | 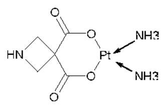 | 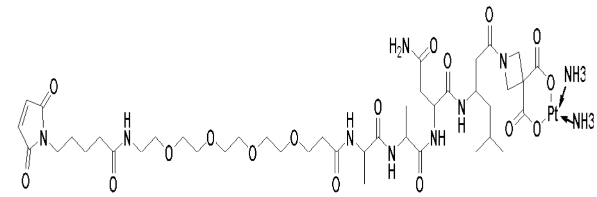 |
| QHL-102-N-CBP | QHL-102 | 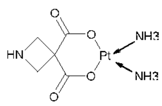 | 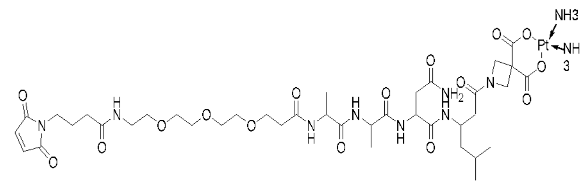 |
| QHL-099-N-CBP | QHL-099 | 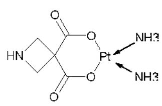 | 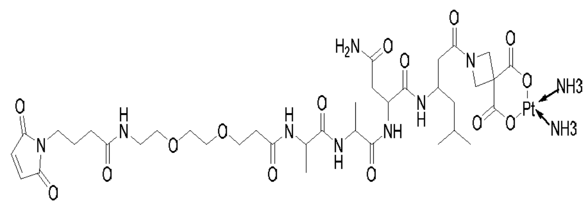 |

(continued)

| No. | Linker | D | Compound structure |
|---|---|---|---|
| QHL-144-N-CBP | QHL-144 | 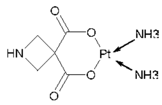 | 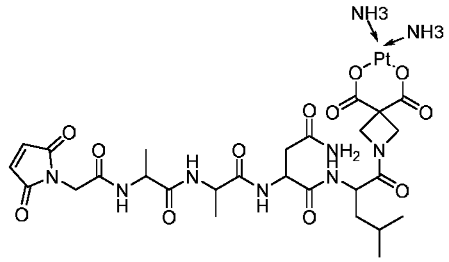 |
| QHL-087-N-CBP | QHL-087 | 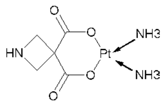 | 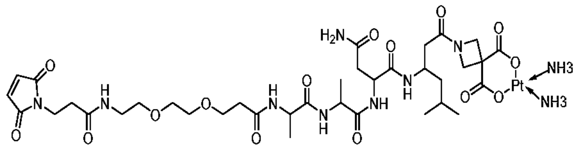 |
| QHL-087-dovitinib | QHL-087 | dovitinib | 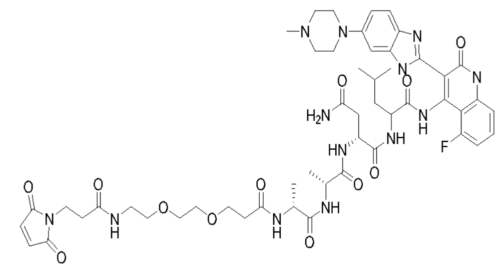 |
| QHL-090-dovitinib | QHL-090 | dovitinib | 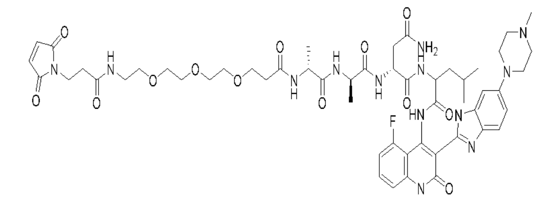 |
| QHL-087-epirubicin | QHL-087 | epirubicin | 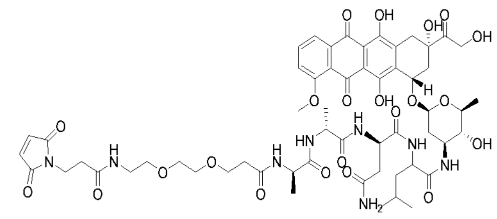 |
| QHL-090-epirubicin | QHL-090 | epirubicin | 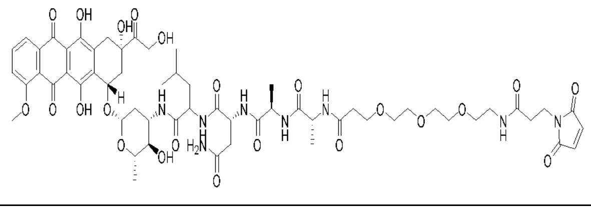 |
| QHL-087-compound a | QHL-087 | compound a | 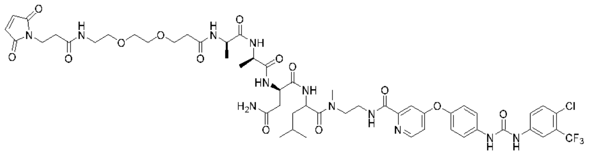 |

(continued)

| No. | Linker | D | Compound structure |
|---|---|---|---|
| QHL-090-compound a | QHL-090 | compound a | 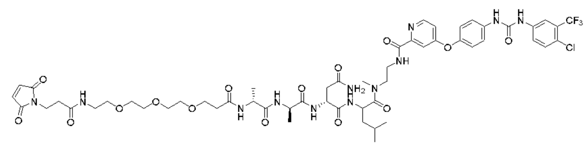 |
| QHL-087-compound b | QHL-087 | compound b | 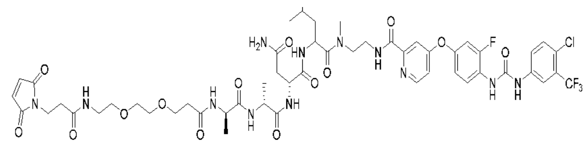 |
| QHL-090-compound b | QHL-090 | compound b | 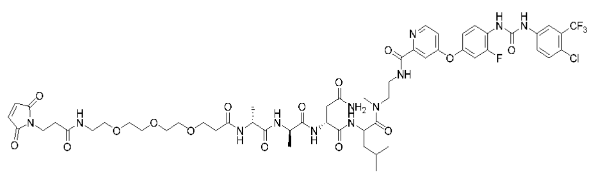 |
| QHL-087-mitomycin | QHL-087 | mitomycin | 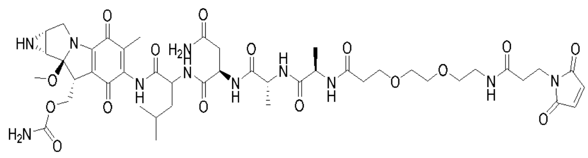 |
| QHL-090-mitomycin | QHL-090 | mitomycin | 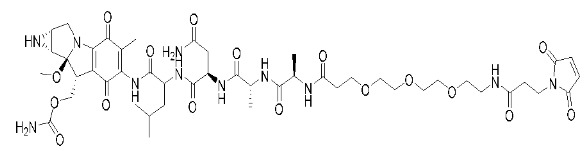 |
| QHL-087-dabrafenib | QHL-087 | dabrafenib | 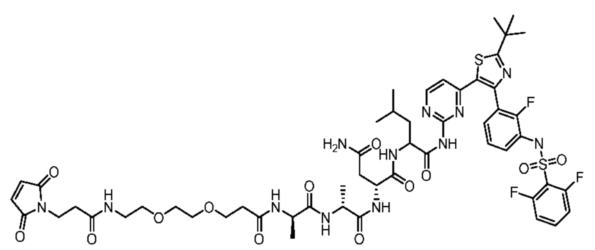 |
| QHL-090-dabrafenib | QHL-090 | dabrafenib | 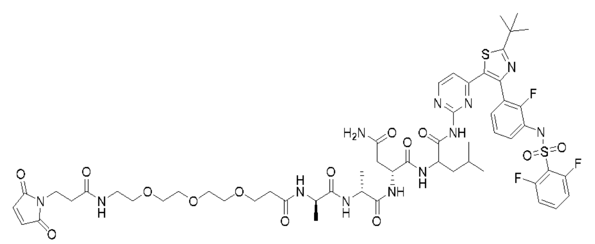 |
| QHL-087-motesanib | QHL-087 | motesanib | 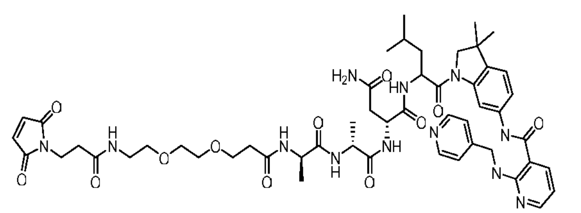 |

(continued)

| No. | Linker | D | Compound structure |
|---|---|---|---|
| QHL-090-motesanib | QHL-090 | motesanib | 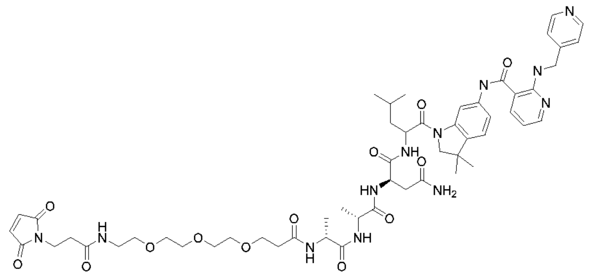 |
| QHL-140-resiquimod | QHL-140 | resiquimod | 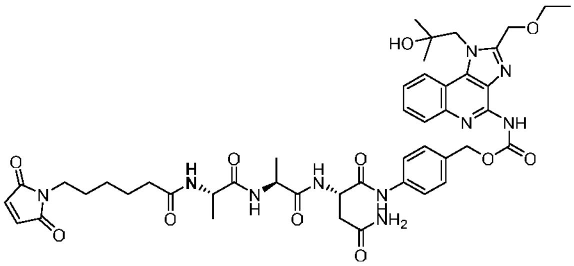 |
| QHL-086-resiquimod | QHL-086 | resiquimod | 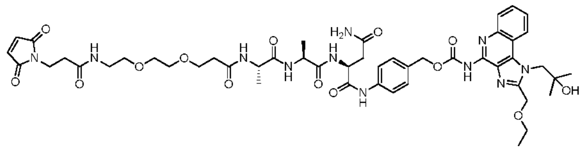 |
| QHL-089-resiquimod | QHL-089 | resiquimod | 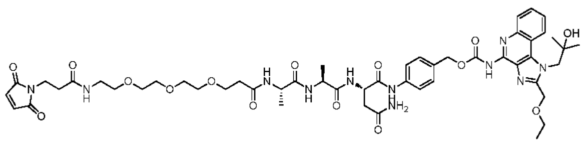 |
| QHL-092-resiquimod | QHL-092 | resiquimod | 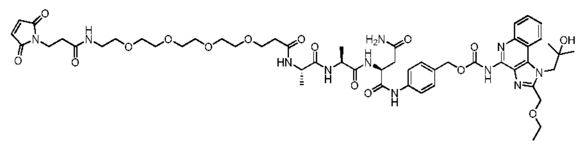 |
| QHL-095-resiquimod | QHL-095 | resiquimod | 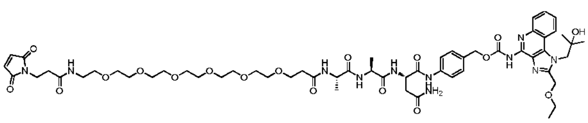 |
| QHL-005-resiquimod | QHL-005 | resiquimod | 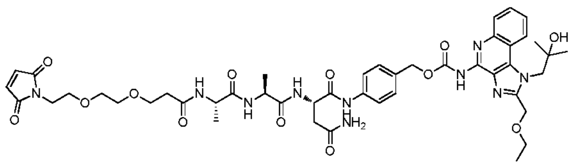 |
| QHL-006-resiquimod | QHL-006 | resiquimod | 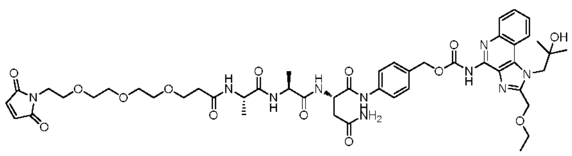 |

(continued)

| No. | Linker | D | Compound structure |
|---|---|---|---|
| QHL-008-resiquimod | QHL-008 | resiquimod | 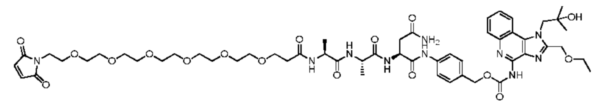 |
| QHL-147-resiquimod | QHL-147 | resiquimod | 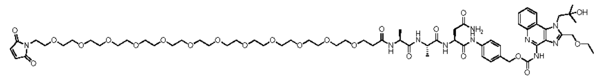 |
| QHL-116-resiquimod | QHL-116 | resiquimod | 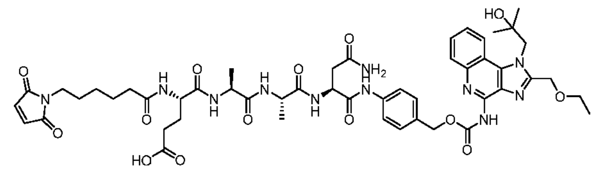 |
| QHL-119-resiquimod | QHL-119 | resiquimod | 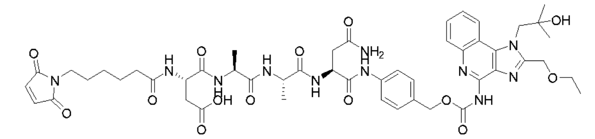 |
| QHL-140-prednisone | QHL-140 | prednisone | 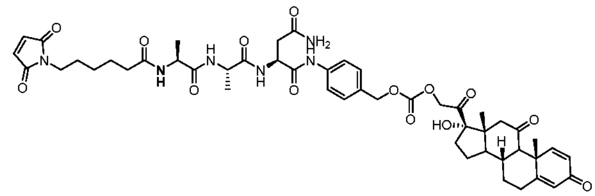 |
| QHL-086-prednisone | QHL-086 | prednisone | 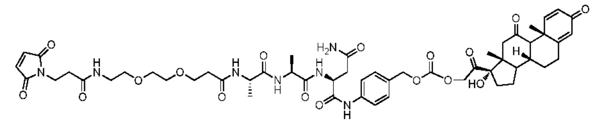 |
| QHL-089-prednisone | QHL-089 | prednisone | 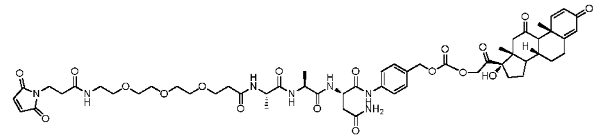 |
| QHL-092-prednisone | QHL-092 | prednisone | 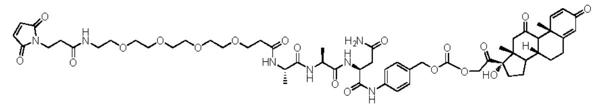 |
| QHL-095-prednisone | QHL-095 | prednisone | 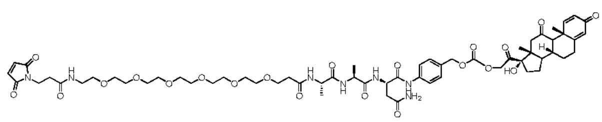 |
| QHL-005-prednisone | QHL-005 | prednisone | 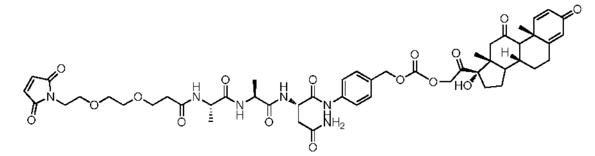 |

(continued)

| No. | Linker | D | Compound structure |
|---|---|---|---|
| QHL-006-prednisone | QHL-006 | prednisone | 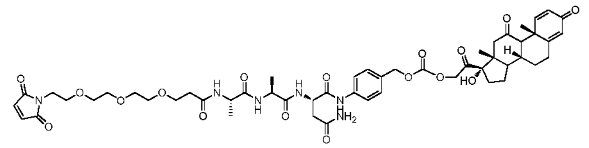 |
| QHL-008-prednisone | QHL-008 | prednisone | 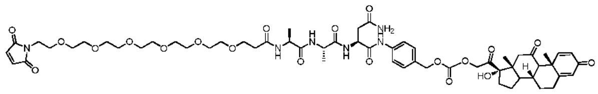 |
| QHL-147-prednisone | QHL-147 | prednisone | 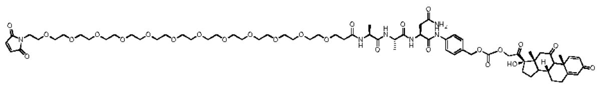 |
| QHL-116-prednisone | QHL-116 | prednisone | 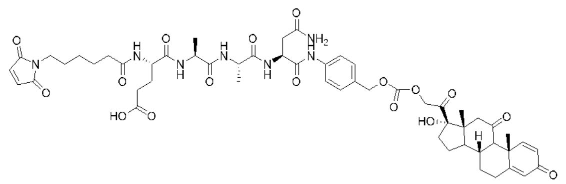 |
| QHL-119-prednisone | QHL-119 | prednisone | 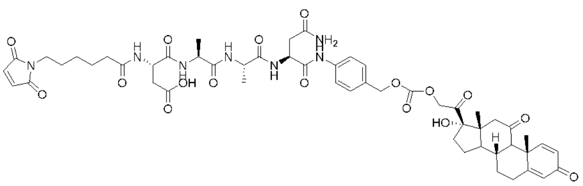 |
| QHL-150-T3 | QHL-150 | T3 | 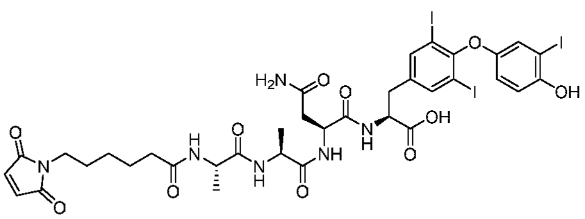 |
| QHL-157-T3 | QHL-157 | T3 | 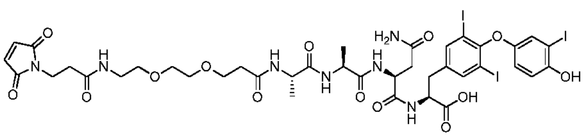 |
| QHL-158-T3 | QHL-158 | T3 | 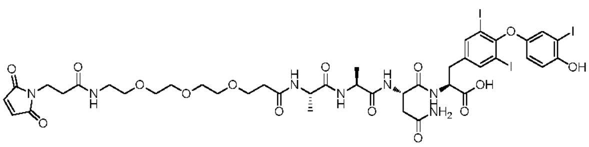 |
| QHL-159-T3 | QHL-159 | T3 | 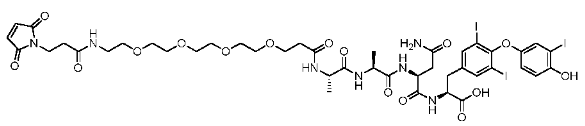 |
| QHL-160-T3 | QHL-160 | T3 | 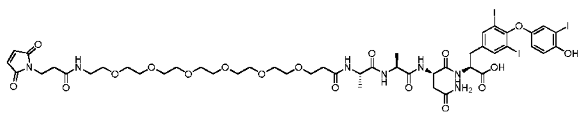 |

(continued)

| No. | Linker | D | Compound structure |
|---|---|---|---|
| QHL-153-T3 | QHL-153 | T3 | 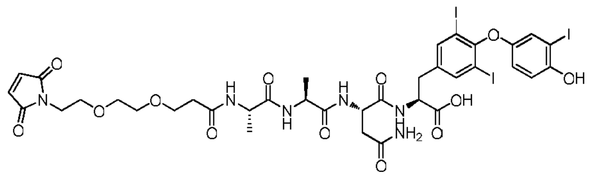 |
| QHL-154-T3 | QHL-154 | T3 | 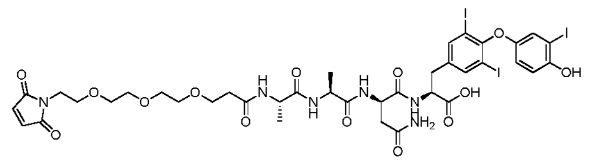 |
| QHL-155-T3 | QHL-155 | T3 | 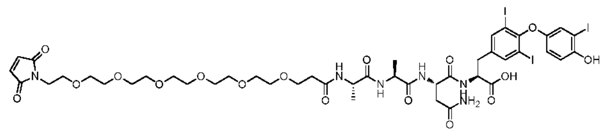 |
| QHL-156-T3 | QHL-156 | T3 | 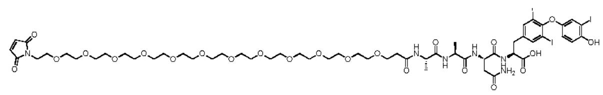 |
| QHL-161-T3 | QHL-161 | T3 | 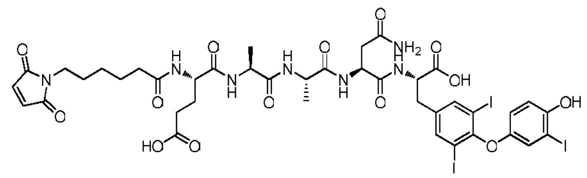 |
| QHL-162-T3 | QHL-162 | T3 | 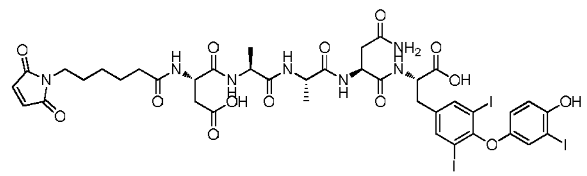 |

In some embodiments, the present disclosure also provides a platinum derivative, a prodrug thereof, or a pharmaceutically acceptable salt thereof, represented by the following formula:

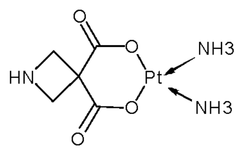

The pharmaceutical composition of the disclosure can be covalently coupled with albumin to form a new pharmaceutical compound. Accordingly, the present disclosure also includes a pharmaceutical compound of formula (II) of the present disclosure covalently linked to albumin. Typically, albumin is linked to the MI of the linker. In some embodiments, the present disclosure also includes EMC-AANL-DOX linked to albumin, pharmaceutical compositions thereof, and uses thereof. The present disclosure also includes a pharmaceutical compound of formula (II), or a pharmaceutically acceptable salt thereof, covalently linked to albumin.

In the present disclosure, the pharmaceutically acceptable salt may be various pharmaceutically acceptable salts well known in the art, including inorganic and organic acid salts such as hydrochloride, hydrobromide, phosphate, sulfate, citrate, lactate, tartrate, maleate, fumarate, mandelate and oxalate; as well as inorganic and organic base salt with bases such as sodium hydroxide, tri (hydroxymethyl) aminomethane (TRIS, tromethamine) and N-methylglucamine.

III. Preparation Method

An exemplary process for that preparation of the compounds of formula (I) and (II) of the present disclosure comprise:

Step 1, preparation of tripeptide-PABC or tetrapeptide: coupling amino acid residues and separating to obtain the formed tripeptide-PABC or tetrapeptide, namely C-A;

Step 2, preparation of MI-S: selecting a compound suitable for the MI-S group, and performing condensation or cyclization to obtain the MI-S with carboxyl at one end;

Step 3, preparation of MI-S-C-A: the C-A obtain in that step 1 and the MI-S obtained in the step 2 are couple through amino and carboxyl to obtain an intermediate (MI-S-C-A);

Step 4, covalently combining the carboxyl or hydroxyl activated product at the A end of the compound MI-S-C-A obtained in the step 3 with the amino of an optional medicament to form the immunostimulatory doxorubicin coupling complex for targeted delivery and activation.
when A is PABC-OH, that synthetic route comprise: coupling the amino acid residue suitable for the disclosure with PABC by using the known chemical and biological recombination coupling technology, and then purifying and separating to obtain C-PABC containing proper amino acid protecting group; The reaction may be carried out in the presence of a condensing agent, a base, a polar aprotic solvent. Remove that protecting group to obtain C-PABC. And then reacting the C-PABC with an acid or ester or acyl chloride containing an MI-S group in the presence of a condensing agent, a base, a polar aprotic solvent to form the MI-S-C-A of formula (I) of the present disclosure, and then reacting the MI-S-C-A with a drug compound of interest or a salt thereof in the presence of a condensing agent, a base, a polar aprotic solvent to form the drug compound of formula (II) of the present disclosure.
Examples of the base used in the preparation method include organic bases such as triethylamine, pyridine, N,N-diisopropylethylamine, 4-dimethylaminopyridine, 1,2,2,6,6-pentamethylpiperidine and the like, or inorganic bases such as sodium carbonate, potassium carbonate, sodium hydrogencarbonate and potassium hydrogencarbonate and the like. Examples of the condensing agent used in the preparation method include HBTU, DMC, HATU, HOBT, DIC, DCC, EDCI, DEPBT, etc., and the solvent used in the preparation method may be any solvent as long as the solvent itself is inert in the reaction and does not inhibit the reaction. Such solvents include halogenated hydrocarbon solvents such as methylene chloride, chloroform, etc., aromatic hydrocarbon solvents such as benzene, toluene, etc., aprotic solvents such as acetonitrile, N,N-dimethylformamide, dimethyl sulfoxide, etc., ester solvents such as methyl acetate, ethyl acetate, etc., ether solvents such as tetrahydrofuran, or a mixture of these solvents. The reaction in this preparation method can be carried out at a temperature ranging from ice-cooling to 150°C.

IV. Pharmaceutical Compositions

The disclosure includes pharmaceutical compositions, which comprise a compound of formula (II) of that disclosure or a pharmaceutically acceptable salt thereof, or a platinum derivative of the disclosure or a pharmaceutically acceptable salt thereof, or a compound of formula (II) covalently linked to albumin or a pharmaceutically acceptable salt thereof, or EMC-AANL-DOX covalently coupled to albumin or a pharmaceutically acceptable salt thereof.
The pharmaceutical composition may also contain a pharmaceutically acceptable carrier or excipient. The carrier or excipient may be any of a variety of pharmaceutically acceptable carriers or excipients well known in the art, and may vary depending on the pharmaceutical dosage form or mode of administration.
In one embodiment, the pharmaceutical composition comprises one or more of a solvent, a solubilizer/cosolvent, a pH modifier, a lyophilizing excipient, and an osmotic pressure modifier.
Lyophilization excipients suitable for use in the present disclosure include one or more of sugars (e.g., lactose, maltose, dextran, glucose, fructose), amino acids (e.g., arginine, lysine, histidine), mannitol, tartaric acid, maleic acid, citric acid, sodium chloride, and cyclodextrins (e.g., hydroxypropyl beta-cyclodextrin, sulfobutyl beta-cyclodextrin).
Suitable pH adjusting agents for use in the present disclosure include one or more of hydrochloric acid, phosphoric acid, sulfuric acid, carbonic acid, nitric acid, acetic acid, citric acid, DL-tartaric acid, D-tartaric acid, L-tartaric acid, sodium hydroxide, potassium hydroxide, meglumine, maleic acid, ethylenediamine, triethylamine, arginine, lysine, histidine, sodium dihydrogen phosphate, and disodium hydrogen phosphate.
The solvent suitable for use in the present disclosure is preferably an organic solvent, including one or more of ethanol, propylene glycol, polyethylene glycol 300, polyethylene glycol 400, tert-butanol, glycerol, Tween, soybean oil, hydroxypropyl beta cyclodextrin solution, and sulfobutyl beta cyclodextrin solution.
Osmolarity adjusting agents suitable for use in the present disclosure include one or more of glucose, sodium

chloride, mannitol, and sodium lactate.

Solubilizers/co-solvents suitable for use in the present disclosure include one or more of Tween 80, Tween 60, poloxamers, hydroxypropyl beta-cyclodextrin, polyethylene glycol (PEG), lithium 12-hydroxystearate, sulfobutyl beta-cyclodextrin, PVP, glycerol, and polyoxyethylene castor oil.

In general, the compound of the present disclosure or a pharmaceutically acceptable salt thereof is orally administered to a mammal daily in an amount of usually about 0.0025 to 50 mg/kg body weight, preferably about 0.01 to 10 mg/kg body weight. If a known anti-cancer drug or other therapy is administered concurrently, the dose should be effective to achieve its intended purpose. The optimal dosage of these known anticancer drugs is well known to those skilled in the art.

A unit oral dose may comprise from about 0.01 to 50 mg, preferably from about 0.1 to 10 mg, of a compound of this disclosure or a pharmaceutically acceptable salt thereof. The unit dose may be administered one or more times per day in one or more doses, each dose containing from about 0.1 to 50 mg, conveniently from about 0.25 to 10 mg, of a compound of this disclosure or a pharmaceutically acceptable salt thereof.

The pharmaceutical composition of that present disclosure can be prepared into any suitable dosage form, including but not limit to tablets, capsules, injections, etc. The pharmaceutical compositions of the present disclosure may be administered by routes well known in the art, such as orally, intravenously, intramuscularly, and the like.

V. Use of Compounds and Pharmaceutical Compositions

The cytokines secreted by tumor induce monocytes to transform into tumor-associated macrophages (TAM), which can stimulate strong immunosuppression and directly help tumor cells to infiltrate and metastasize. The confirmatory marker that differentiates tumor-associated macrophages (M2 type) from monocytes and inflammatory macrophages (M1 type) is the expression of asparagine endopeptidase. The compounds of the present disclosure can be activated and released in the presence of aspartate endopeptidase. Because different parts of the coupling body activated by the specificity of the asparagine endopeptidase have great influence on the functions of targeting, activation, stability, toxicity, drug effect and the like of the final drug, the coupling body activated by the specificity of the asparagine endopeptidase can effectively reduce the toxicity of the connected drug, so that the final drug has new targeting, activation and metabolism characteristics, increases the effect of treating tumors, generates new tumor indications and functions of resisting tumor metastasis, and generates brand-new structures and functions.

The disclosure also find that the compound shown in the formula (II) has the effects of kill tumor-associated macrophages, weakening immunosuppressive cytokines in a microenvironment and promote immune enhancement of toxic CD8 cells. More importantly, these tumor microenvironment-releasing compounds are activated only locally in the tumor, unlike traditional chemotherapeutic drugs that damage the overall immune system. In that experiment, the tumor microenvironment release compound and a PD-1 (programmed death-1) inhibit antibody (an anti-PD-Ll antibody, which is commercially available and is a candidate medicament which is considered to have immunotherapy effect at present) have strong synergistic treatment effect, and can solve the defect that immunotherapy is difficult to combine with chemotherapy medicaments.

Thus, the compounds of the present disclosure, pharmaceutically acceptable salts thereof, or pharmaceutical compositions may be used to treat or prevent the treatment or prophylaxis of a disease known in the art to be caused by the use of resiquimod, prednisone, T3, doxorubicin, daunorubicin, epirubicin, methotrexate, fludarabine, gemcitabine, cytarabine, melphalan, nimustine, mitoxantrone, mitomycin, camptothecin, 10-hydroxycamptothecin, topotecan, floxuridine, doxifluridine, etoposide, fludarabine, capecitabine, vincristine, epothilone B, paclitaxel, docetaxel, dabrafenib, dovitinib, motesanib, compound a, compound b, and platinum compounds (e.g., carboplatin, cisplatin, oxaliplatin) can treat a variety of diseases, including cancer, ophthalmic diseases, and liver diseases, among others.

For example, it is known in the art that camptothecin can be used for treating or preventing hepatosplenomegaly caused by malignant tumor, psoriasis, wart, acute/chronic leukemia and schistosomiasis, etc.; the 10-hydroxycamptothecin can be use for treating gastric cancer, liver cancer, head and neck cancer, leukemia, etc. Paclitaxel is mainly used for treating ovarian cancer and breast cancer, and also has therapeutic effects on lung cancer, carcinoma of large intestine, melanoma, head and neck cancer, lymphoma, cerebroma, etc. Mitomycin C can be use for treating chronic lymphoma, chronic myelogenous leukemia, esophageal cancer, gastric cancer, colon cancer, rectal cancer, lung cancer, pancreatic cancer, hepatocarcinoma, cervical cancer, carcinoma of uterine body, ovarian cancer, breast cancer, head and neck tumor, bladder tumor, malignant cavity effusion, etc.

Thus, for example, diseases that may be treated or prevented with a compound of the present disclosure, a pharmaceutically acceptable salt thereof, or a pharmaceutical composition thereof include, but are not limited to, cancers of the bladder, brain, breast/mammary gland, cervix, colon-rectum, esophagus, kidney, liver, lung, nasopharynx, pancreas, prostate, skin, stomach, uterus, ovary, testis, and blood. In particular, these cancer are selected from: liver cancer, kidney cancer, thyroid cancer, colorectal cancer, bladder cancer, brain cancer, breast cancer, cervical cancer, rectal cancer, esophageal cancer, lung cancer, (e. g., bronchogenic carcinoma of that lung, include undifferentiated small cell

and non-small cell), nasopharyngeal carcinoma, pancreatic carcinoma, prostate canc, skin cancer, gastric cancer, uterine cancer, ovarian canc, testicular cancer, leukemia (e. g., chronic or acute leukemia, including lymphocytic and granulocytic leukemia), malignant lymphoma, fibrosarcoma, soft tissue sarcoma, osteogenic sarcoma, rhabdomyosarcoma, Ewing's sarcoma, Wilms 'tumor, neuroblastoma, thyroid cancer, and squamous cell carcinoma of the head and neck.

In one embodiment, that pharmaceutical compound of formula (II) wherein D is mitomycin or a pharmaceutically acceptable salt thereof of the present disclosure can also be used for treating or preventing ophthalmic diseases, including treating or prevent healing scars or choroidal neovascularization, or inhibiting macrophages. In other embodiment, that pharmaceutical compound of formula (II) wherein D is mitomycin or a pharmaceutically acceptable salt thereof can also be used for treating or preventing corneal transplantation, glaucoma, sequela of pterygium surgery, and the like.

The compounds or pharmaceutical compositions of the present disclosure can also be used to prevent tumor metastasis, especially to prevent tumor metastasis to the lung. In one embodiment, a compound or pharmaceutical composition of the disclosure can be used to prevent lung metastasis of breast cancer.

The liver diseases of the present disclosure include, but are not limited to, fatty liver (including alcoholic and non-alcoholic fatty liver), steatohepatitis, fatty liver disease, liver fibrosis, liver inflammation, and steatosis phenomena of liver cell damage.

Accordingly, the present disclosure includes a method of treatment or prophylaxis of a disease, preferably cancer, an ophthalmic disease and a liver disease according to any of the embodiments of the present disclosure, comprising administering to a subject in need thereof a therapeutically or prophylactically effective amount of a compound of formula (II) of the present disclosure or a pharmaceutically acceptable salt thereof, or a pharmaceutical composition comprising a compound of formula (II) of the present disclosure or a pharmaceutically acceptable salt thereof. In some embodiments, either a platinum derivative or a pharmaceutically acceptable salt thereof as described herein, or a compound of formula (II) covalently linked to albumin or a pharmaceutically acceptable salt thereof, or EMC-AANL-DOX covalently coupled to albumin or a pharmaceutically acceptable salt thereof, or a pharmaceutical composition of each is administered.

The present disclosure also includes a method for preventing tumor metastasis, comprising administering an effective amount of the compound of the present disclosure or a pharmaceutically acceptable salt thereof, or a pharmaceutical composition containing the compound of the present disclosure or a pharmaceutically acceptable salt thereof, to a subject in need thereof, wherein preventing tumor metastasis includes but is not limited to preventing tumor lung metastasis and/or bone metastasis.

Tumor-associated macrophages (TAM), as a key inflammatory cell, play an important role in tumor-associated inflammation. In the tumor microenvironment, TAM promotes tumor development by affecting various aspects of tumor biological characteristics. It secretes some molecules (such as EGF) to directly promote the growth of tumor cells, promote angiogenesis, so as to create conditions for cancer cell infiltration and metastasis, but also can inhibit the adaptive immune function. Thus, the present disclosure also includes a method of inhibiting tumor-associated macrophages comprising administering to a subject in need thereof an effective amount of a compound of the present disclosure or a pharmaceutically acceptable salt thereof, or a pharmaceutical composition comprising a compound of the present disclosure or a pharmaceutically acceptable salt thereof. By inhibiting tumor-associated macrophages, tumor growth can be inhibited, angiogenesis can be inhibited, infiltration and metastasis of cancer cells can be inhibited, anti-tumor immunity can be promoted, thereby preventing and/or treating cancer. In one embodiment, the tumor-associated macrophages express aspartate endopeptidase, which is of the M2 type.

The above method of that present disclosure may be used in combination with radiation therapy or immunotherapy as known in the art.

Accordingly, the present disclosure also includes a compound of the present disclosure, a pharmaceutically acceptable salt thereof, or a pharmaceutical composition of the present disclosure for use in any of the methods or uses described above.

The disclosure also includes that use of a compound of the disclosure or a pharmaceutically acceptable salt thereof or a pharmaceutical composition of the disclosure in the manufacture of a medicament for the treatment or prevention of the above-mentioned diseases (e.g., cancer and cancer metastasis). The disclosure also comprises the application of the compound or the pharmaceutically acceptable salt thereof or the pharmaceutical composition in the preparation of medicaments for inhibiting tumor-associated macrophages, inhibiting tumor growth, inhibiting angiogenesis, inhibiting infiltration and metastasis of cancer cells and/or promoting anti-tumor immunity.

The present disclosure also provides a method of reducing the toxic side effects of an anticancer compound, particularly an anticancer compound as described herein, comprising linking the anticancer compound to a linker compound of formula (I) of the present disclosure.

The therapeutic or prophylactic methods of the present disclosure comprise administering a compound or pharmaceutical composition of the present disclosure to a subject in need thereof. Methods of administration include, but are not limited to, oral, intravenous, intramuscular, and that like. Subjects include mammals, especially human.

In some embodiments, the present disclosure also provides an application of EMC-AANL-DOX compound

having a structure shown in the following formula or a medicament thereof coupled with albumin in preparing a medicament for treating liver cancer:

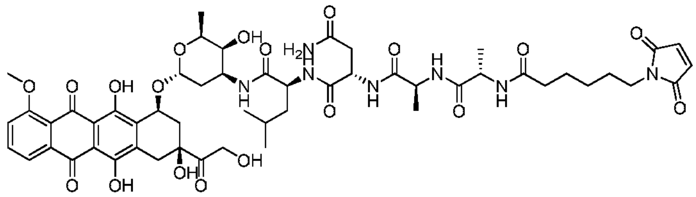

。

It is to be understood that the terms "comprising" and "including" are intended to include "consisting of" and "consisting of." The sum of all weight percentage or volume percentages should be equal to 100%. The various reagents and products used in the examples are commercially available unless otherwise indicated; the methods involved are carried out according to conventional techniques, unless otherwise indicated. The following embodiments are not intended to limit the scope of the present disclosure.

**Embodiment 1: Synthesis of QHL-095-DOX**

The synthesis of QHL-095-DOX is shown below:

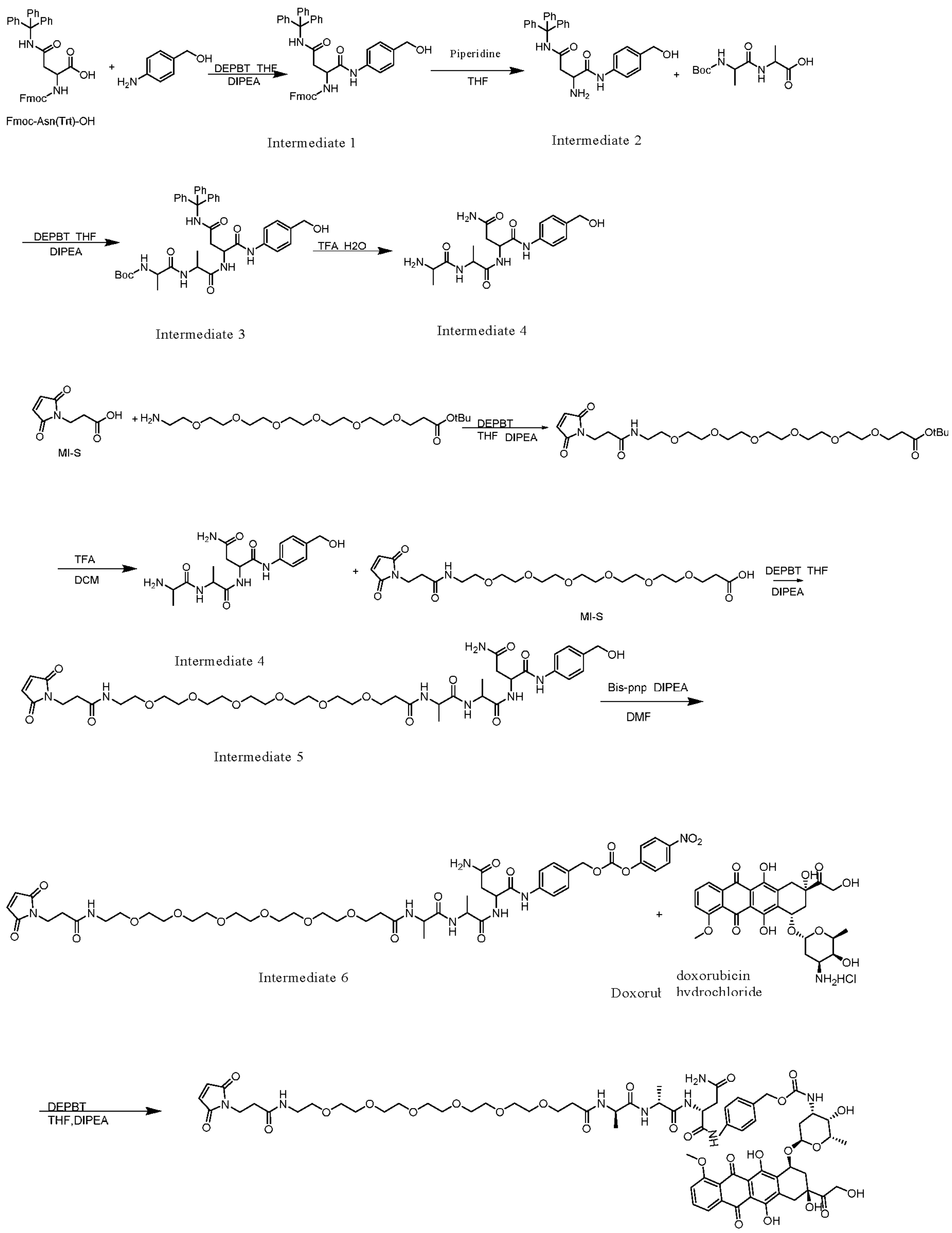


1. Synthesis of Intermediate 1

Take a dry and clean 2L reaction flask, adding 500ml of THF, weigh 80g of Fmoc-Asn(Trt)-OH, adding that Fmoc-Asn(Trt)-OH into the reaction flask, stirring for dissolving, adding 46. 6g of DEPBT, stirring at room temperature for 15 minutes, adding 16g of PABC, reacting at room temperature for 30 minutes, adding 45ml of DIPEA, performing ventilation protection with nitrogen, reacting at room temperature for 3 hours, and monitoring the completion of the reaction by TLC (the Fmoc-Asn(Trt)-OH reaction is completed).

The reaction solution was evaporated under reduced pressure, dissolved in a small amount of DMF (180 ml)

and added dropwise to 3L of stirring water to precipitate a pale yellow solid, which was washed with water for 2-3 times, filtered under suction, collected and dried under vacuum to obtain an off-white solid (yield> 90%).

2. Synthesis of Intermediate 2

Add 500 ml of THF and the off-white solid obtained in the previous step into a 2L single-neck reaction flask in turn, stir and dissolve, cool to 0-5 °C in an ice-salt bath, dropwise add 100 ml of piperidine, gradually recover to room temperature after dropwise addition, react for 1 h, and monitor the completion of reaction by TLC. Evaporate that solvent under reduce pressure, adding a small amount of DMF for dissolution, dropwise adding into 2L of water obtain during stirring, mechanically stirring for 30min, performing suction filtration, repeating water washing for 2-3 times, performing suction filtration, adding 800ml of methyl tert-butyl ether into a filter cake, stirring for 30min, performing suction filtration, adding PE: EA=10:1, suction filtration, collection of filter cake, vacuum drying, to obtain off-white solid 80g, purity of 70%.

3. Synthesis of Intermediate 3

50 ml of THF, 5.04 g of Boc-Ala-Ala-OH and 3.89 g of DEPBT were added into a dry and clean 250 ml single-neck reaction flask in sequence, reacted for 10 min at room temperature, and 2.6 g of NH2H2H2-Asn(Trt)-PABC was added, react for 15 min at room temperature under that protection of nitrogen gas exchange, dropwise adde 3.5 ml of DIPEA, reacting for 3 hours at room temperature under the protection of nitrogen gas exchange, evaporate the solvent under reduced pressure, adding water and pulping for 2-3 times, filtering to obtain 3.7 g of light yellow solid, and purifying by column chromatography to obtain 2.0 g of product, wherein the purity is 94.8%, and the yield is 26.6%.

4. Synthesis of Intermediate 4

Add 1.8 g of intermediate 3 into a 250 ml single-mouth reaction flask, add 28.5 ml of TFA, add 1.5 ml of water dropwise, react at room temperature for 30 min, monitor the reaction by TLC, evaporate the solvent under reduced pressure, add methyl tert-butyl ether for pulping, and perform suction filtration to obtain a solid. Dissolve the solution of dioxane and water in the ratio of 1:1, add IN sodium hydroxide to adjust pH to 13, stir at room temperature for 40min, evaporate the solvent under reduced pressure, mix the sample with silica gel and purified by column chromatography to obtain 450mg of product, the yield is 47.5%.

5. Synthesis of Intermediate MI-S

MI-S1 (338 mg, 2 mmol) and DEPBT (717.6 mg, 2.4 mmol) were added into a 100 ml single-neck flask, and DMF (15 ml) was added to dissolve them. The reaction was carried out at room temperature for 15 min under the protection of nitrogen.Then R3-b (819 mg, 2 mmol) was added and dissolved by stirring. The reaction was carried out for 15 min at room temperature, then DIPEA (137 μl) was added dropwise, and the reaction was carried out for 3h at room temperature under the protection of nitrogen gas exchange, and monitor the completion of R3-a reaction by TLC.The solvent was removed by distillation under reduced pressure, the crude product was dissolved in methanol, and purified by a reversed-phase high-pressure column chromatography to obtain the intermediate of R3 - 1 (720 mg, yield: 64.3%).

6. Synthesis of MI-S

Add the intermediate (720 mg, 1.28 mmol) obtained in the previous step into a 100 ml single-neck reaction flask, add 15 ml of dichloromethane to dissolve, dropwise add 5 ml of TFA and 0.25 ml of water, react at room temperature for 30 min, and monitor the completion of reaction by TLC. Evaporate under reduce pressure to remove solvent, adding methyl tert-butyl ether, pulping, filtering to obtain solid, mix with silica gel, purified by a reversed-phase column chromatography to obtain 242 mg of product. The yield thereof was found to be 37.5%.

7. Synthesis of Intermediate 5

Intermediate 4 (150 mg, 0.395 mmol) and EMC-6Peg-COOH (239 mg, 0.474 mmol) were added into a 100 ml single-necked flask, DMF (15 ml) was added to dissolve them, and the reaction was carried out at room temperature for 15 min under the protection of nitrogen gas exchange. Then 137 μl of DIPEA was added dropwise, and the reaction was carried out at room temperature for 3 h under the protection of nitrogen gas exchange. The completion of reaction of intermediate 4 was monitored by TLC. The solvent was removed by distillation under reduce pressure, that crude product was dissolved in methanol, and purified by a reversed-phase high-pressure column chromatography to give

intermediate 5 (95 mg, yield: 21%).

8. Synthesis of Intermediate 6

Sequentially added 25 ml of DMF, intermediate 5 (300 mg, 0.346 mmol) and Bis-PNP (316 mg, 1.04 mmol) into a 100 ml single-neck reaction flask, reacting for 15 min at room temperature under that protection of nitrogen gas exchange, dropwise adding 258μl of DIPEA, reacting for 3 h at room temperature under the protection of nitrogen gas exchange, monitor that 7% of raw materials remain by HPLC, terminating the reaction, evaporating the solvent under reduced pressure, and purifying by column chromatography to obtain 150 mg of the product with a yield of 42%.

9. Synthesis of the Final Product QHL-095-DOX

Add 84 mg of doxorubicin hydrochloride (1.0 eq, 0.145 mmol) and 150 mg of intermediate 6 (1.0 eq, 0.145 mmol) into a 100 mL reaction flask, and react for 15 min at room temperature under nitrogen protection. DIPEA 75 μl was added dropwise and reacted at room temperature for 4 hours. The solvent was evaporated under reduced pressure. The crude product was dissolved in methanol and purified by a reversed-phase high-pressure column chromatography to give QHL-095-DOX (49 mg red solid, yield: 23.8%).

**Embodiment 2: Synthesis of QHL-116-DOX**

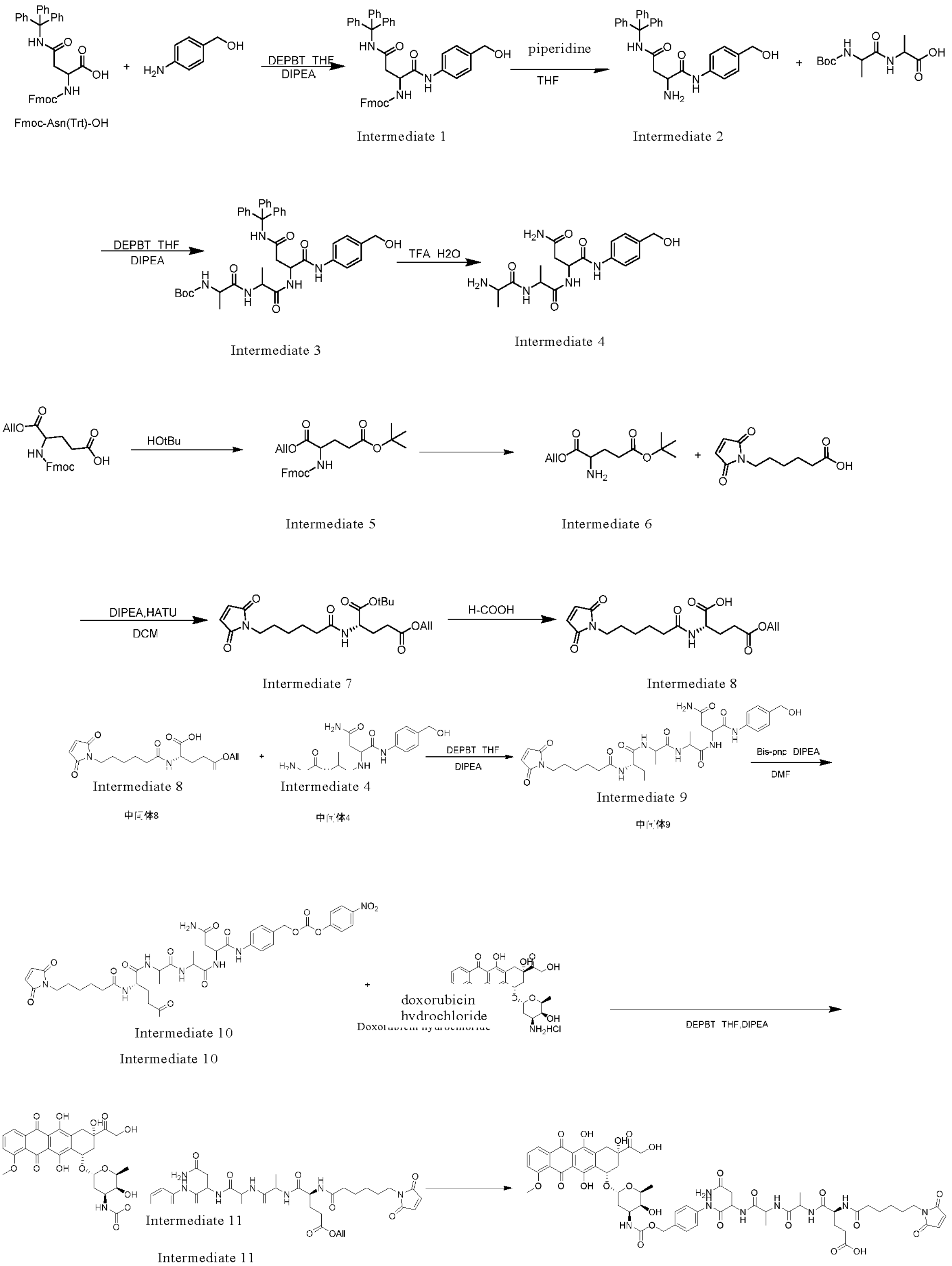


Fmoc-Asn(Trt)-OH

Intermediate 1

Intermediate 2

Intermediate 3

Intermediate 4

Intermediate 5

Intermediate 6

Intermediate 7

Intermediate 8

Intermediate 8

中间体8

Intermediate 4

中间体4

Intermediate 9

中间体9

Intermediate 10

Intermediate 10

doxorubicin hydrochloride

Doxorubicin hydrochloride

Intermediate 11

Intermediate 11

1. Synthesis of Intermediate 1

Take a dry and clean 2L reaction flask, adding 500ml of THF, weigh 80g of Fmoc-Asn(Trt)-OH, adding that Fmoc-Asn(Trt)-OH into the reaction flask, stirring for dissolving, adding 46. 6g of DEPBT, stirring at room temperature for 15 minutes, adding 16g of PABC, reacting at room temperature for 30 minutes, adding 45ml of DIPEA, performing ventilation protection with nitrogen gas exchange, reacting at room temperature for 3 hours, and monitoring the completion of the reaction by TLC (the Fmoc-Asn(Trt)-OH reaction is completed).

The reaction solution was evaporated under reduced pressure, dissolved in a small amount of DMF (180 ml) and added dropwise to 3L of stirring water to precipitate a pale yellow solid, which was washed with water for 2-3 times, filtered under suction, collected and dried under vacuum to obtain an off-white solid (yield> 90%).

2. Synthesis of Intermediate 2

Add 500 ml of THF and the off-white solid obtained in the previous step into a 2L single-neck reaction flask in turn, stir and dissolve, cool to 0-5 °C in an ice-salt bath, dropwise add 100 ml of piperidine, gradually recover to room temperature after dropwise addition, react for 1 h, and monitor the completion of reaction by TLC. Dissolve the solvent under reduced pressure, add a small amount of DMF for dissolution, dropwise add into 2L of water under stirring, mechanically stir for 30 min, and perform suction filtration. Repeat water wash for 2-3 times, suction filtering, adding 800 ml of methyl tert-butyl ether into a filt cake, stirring for 30 min, and suction filtering. Add PE and EA in a ratio of 10:1 to the filter cake, wash twice and filter with suction. Finally, the filter cake was collected and dried in vacuum to obtain 80 g of off-white solid with a purity of 70%.

3. Synthesis of Intermediate 3

50 ml of THF, 5.04 g of Boc-Ala-Ala-OH and 3.89 g of DEPBT were added into a dry and clean 250 ml single-neck reaction flask in turn, and the mixture was reacted at room temperature for 10 min.Then 2.6 g NH2H2H2-Asn(Trt)-PABC was added and the reaction was carried out at room temperature for 15 min under the protection of nitrogen gas exchange. DIPEA 3.5ml was added dropwise, the reaction was carried out at room temperature for 3 hours under the protection of nitrogen gas exchange, the solvent was evaporated under reduced pressure, water was added and the slurry was pulped for 2-3 times, and then 3.7g of light yellow solid was obtained by suction filtration. After purification by column chromatography, 2.0g of product was obtained with purity of 94.8% and yield of 26.6%.

4. Synthesis of Intermediate 4

Add 1.8 g of intermediate 3 into a 250 ml single-mouth reaction flask, add 28.5 ml of TFA, add 1.5 ml of water dropwise, react at room temperature for 30 min, monitor the completion of reaction by TLC, evaporate the solvent under reduced pressure, add methyl tert-butyl ether for pulping, and perform suction filtration to obtain a solid. Dissolve the solution of dioxane and water in the ratio of 1:1, add 1N sodium hydroxide to adjust pH to 13, stir at room temperature for 40min, evaporate the solvent under reduced pressure, mix the sample with silica gel and purified by column chromatography to obtain 450mg of product, the yield is 47.5%.

5. Synthesis of Intermediate 5

Fmoc-Glu (OAll)-COOH (1.554 g, 3.79 mmol) was weighed, dissolved in 10 ml of a mixed solution of DCM and THF, and stirred. 2.72 ml of HOtBu was added dropwise, and after the addition was completed, the reaction was carried out for 16 hours at room temperature under the protection of $N_2$ gas exchange, and the completion of the reaction was monitored by TLC. The solvent was evaporated under reduced pressure, and the silica gel was mixed with the sample and purified by column chromatography to obtain 1.4 g of the product with a yield of 79.5%.

6. Synthesis of Intermediate 6

To a dry clean 250 ml single-neck reaction flask was added 10 ml THF, followed by Intermediate 5 (1.4 g, 3 mmol) from the previous step. Stirring and dissolving, cool to 0-5 °C in an ice-salt bath, dropwise adding 3ml of piperidine, gradually heating to room temperature after dropwise added, reacting for 2 hours, and monitoring that completion of the reaction by TLC. The solvent was evaporated under reduced pressure, purified by silica gel mixing, and the eluate containing the product was collected and dried under reduced pressure to constant weight to obtain 583 mg of the product with a yield of 80%.

7. Synthesis of Intermediate 7

Add 15 ml THF, 583 mg intermediate 6 and 932.8 mg DEPBT into a dry and clean 250 ml single-neck reaction flask in turn, and react at room temperature for 10 min. Then 506.4 mg of maleimidocaproic acid was added, nitrogen was exchanged for protection, and that reaction was carry out for 15 minutes at room temperature.The reaction was carried out at room temperature for 3 hours under the protection of $N_2$ gas exchange. The solvent was evaporated under reduced pressure, and the mixture was pulped with water for 2-3 times. 800 mg of light yellow solid was obtained by

suction filtration. The product was purified by column chromatography to obtain 628 mg of product with purity of 94.8% and yield of 59.9%.

8. Synthesis of Intermediate 8

In a dry and clean 100 ml single-neck reaction flask, 10 ml of dichloromethane and 872 mg of intermediate 7 were added in turn.After uniform stirring, 3 ml of TFA was added dropwise, and the reaction was carried out at room temperature for 2 hours, and the completion of the reaction of the raw materials was monitored by TLC.The solvent was removed by vacuum distillation, and the solid was obtained by pulping with methyl tert-butyl ether and filtration. The solid was purified by silica gel. The eluate containing the product was collected and dried under vacuum to constant weight to obtain 459 mg of the product with a yield of 60.3%.

9. Synthesis of Intermediate 9

Add 15 ml THF, 459 mg intermediate 8 and 434 mg DEPBT into a dry and clean 250 ml single-neck reaction flask in turn, and react at room temperature for 10 min.Then 457.8 mg of intermediate 4 was added, and the reaction was carried out at room temperature for 15 min under nitrogen purging. DIPEA 627 μl was added dropwise, the reaction was carried out at room temperature for 3 hours under the protection of nitrogen gas exchange, the solvent was evaporated under reduced pressure, water was added and the slurry was pulped for 2-3 times, 750 mg of light yellow solid was obtained by suction filtration, and 655 mg of product was obtained by column purification with a yield of 63.2%.

10. Synthesis of Intermediate 10

Sequentially added 25 ml of DMF, intermediate 9 (655 mg, 0.88 mmol) and Bis-PNP (804 mg, 2.64 mmol) into a 100 ml single-necked reaction flask, reacting for 15 min at room temperature under that protection of nitrogen gas exchange, dropwise adding 258μl of DIPEA, reacting for 3 h at room temperature under the protection of nitrogen gas exchange, monitor that 7% of raw materials remain by HPLC, terminating the reaction, evaporating the solvent under reduced pressure, and purifying by column chromatography to obtain 335 mg of the product with a yield of 42%.

11. Synthesis of Intermediate 11

214.3 mg of doxorubicin hydrochloride (1.0 eq, 0.369 mmol) and 335 mg of intermediate 10 (1.0 eq, 0.369 mmol) were added into a 100 mL reaction flask, and reacted at room temperature for 15 min under nitrogen protection. 190μl of DIPEA was further added dropwise, and that mixture was allowed to react at room temperature for 4 hour. The solvent was evaporate under reduce pressure, and that crude product was dissolved in methanol and purified by a reversed-phase high-pressure column chromatography to give intermediate 11 (115 mg of red solid, 23. 8% yield).

12. Synthesis of the Final Product

To a 100 mL reaction flask, 15 mL of THF, intermediate 11 (115 mg, 0.0877 mmol), and tri-n-butyltin hydride (76 mg, 0.2631 mmol) were added in turn, and the reaction solution was saturated with nitrogen. Tetrakis (triphenylphosphine) palladium (0)(14.2 mg, 0.012 mmol) was then added, and that mixture was stirred at room temperature overnight. Monitor by TLC until conversion was completed. The content of that flask were then filtered through celite and the residue was washed with THF. The filtrate was concentrated under reduced pressure. The result crude product was purified by column chromatography to give 100 mg (yield: 90%) of that target compound.

**Embodiment 3: Synthesis of N-CBP**

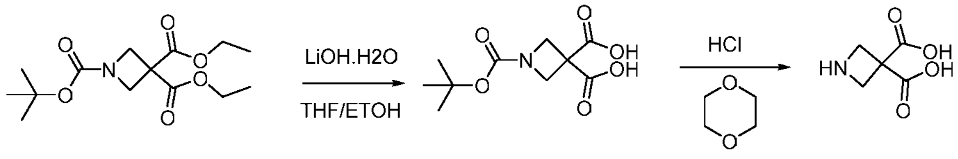


Raw material

Intermediate 1

Intermediate 2

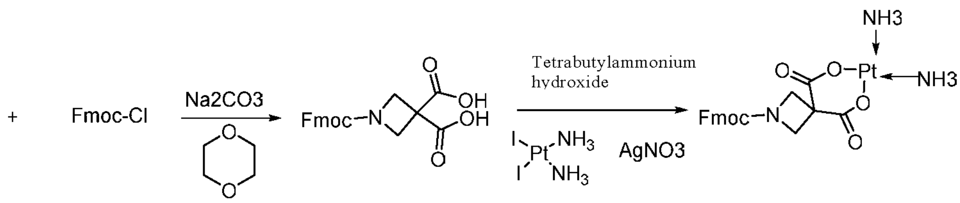


Intermediate 3

Intermediate 4

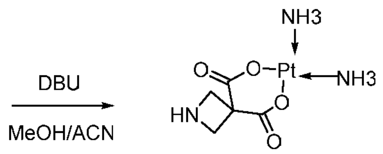


N-CBP

1. Synthesis of Intermediate 1

Add raw material 300 mg to 100 ml three-necked bottle, and add 15 ml THF/ETOH (4:1) dissolve. Cool to -5°C-0°C in an ice-salt bath, dropwise adding 210mg of LiOH (5ml) aqueous solution into that bottle in batch while stirring and controlling the temperature, and naturally raising the temperature to react for 1h after dropwise adding. The reaction liquid is sent to HPLC, that temperature is control to be -5°C-0°C after the raw material are completely reacted, the pH value of the reaction liquid is adjusted to be 3-4 by using 1mol/L HCl, the temperature is control to be 25-30 °C, and the solvent is removed to obtain a crude product of the intermediate 1 which is directly used for the next step.

2. Synthesis of Intermediate 2

Add 15 ml of 1 mol/L dioxane hydrochloride solution into the intermediate 1, stir at room temperature to react for 1 h, send the reaction solution to HPLC, and wait for the complete reaction of the intermediate 1. Controlling the temperature at 25-30 °C to remove the solvent to obtain a crude product of the intermediate 2 which is directly used for the next step.

3. Synthesis of Intermediate 3

Add the crude product of intermediate 2 into a 100ml three-necked flask, add 20ml dioxane to dissolve, and cool to -5°C-0°C in ice-salt bath.Then 159 mg of sodium carbonate aqueous solution (pH is about 8) was added dropwise into the flask under controlled temperature, and 311 mg of Fmoc-Cl dioxane solution was added dropwise under nitrogen protection and controlled temperature of -5°C-0°C. After dropping, the temperature was naturally raised to react for 1 h, and the reaction was sent to HPLC for detection until the intermediate 2 was completely reacted. The solvent was removed by spinning, and that crude silica gel was mixed and purified by a reversed-phase high-pressure column chromatography to obtain 107 mg of intermediate 3.

4. Synthesis of Intermediate 4

Add 107 mg of intermediate 3 into a 50 ml single-necked flask, and add 15 ml of methanol to dissolve. Cool to -20 °C with liquid nitrogen, dropwise add 302 ul of tetrabutylammonium hydroxide (25% methanol solution) into the flask,

naturally heat up and react for 1 h after dropping, and this reaction solution is the standby solution 1.

Add 140.8 mg of diiododiammine platinum into a 50 ml single-neck flask, and add 10 ml of ultrapure water to dissolve, heat to 50°C, keep away from light, dropwise add 49.5 mg of silver nitrate aqueous solution into the flask under the protection of nitrogen, react for 15 min, and then continue to dropwise add 49.5 mg of silver nitrate aqueous solution into the flask. After 15 min of reaction after dropping, the reaction solution was filtered with a filter membrane, and the filtrate was transferred to a 100 ml single-necked flask into which standby solution 1 was added dropwise at room temperature. After nitrogen replacement three times, the reaction solution was transferred to an oil bath and heated to 50°C. After reaction overnight (usually 16h) in that dark. The reaction solution was centrifuged, and the supernatant was directly passed through a high pressure reverse phase column, and the preparation was lyophilized to obtain 79 mg of intermediate 4 with a yield of 45.7%.

5. Synthesis of N-CBP

5 mg of intermediate 4 was added to a 10 ml single-necked flask, followed by 2 ml of MeOH/ACN (1:1) stir to dissolve. 2 μl of DBU was dropped into that reaction solution at room temperature, the reaction was performed for half an hour under nitrogen protection, and the detection was performed by HPLC. After the reaction of the intermediate 4 is completed, the reaction solution was dripped into 6 ml of methyl tert-butyl ether to precipitate an off-white solid, centrifuged, and the supernatant was removed. The solid was dissolved in water/tert-butyl alcohol and passed through a column to obtain 1.8 mg of the product N-CBP.

**Embodiment 4: Synthesis of QHL-140-N-CBP**

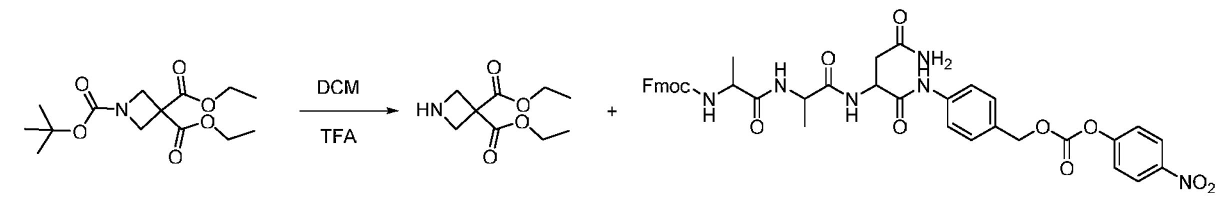


Intermediate 1

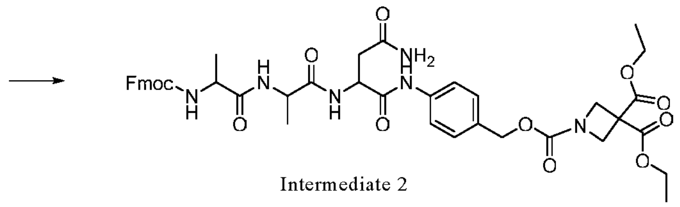

Intermediate 2

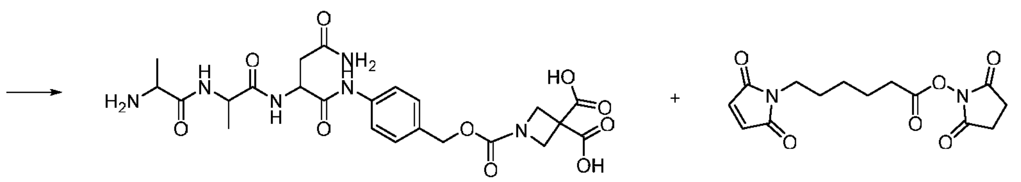

Intermediate 3

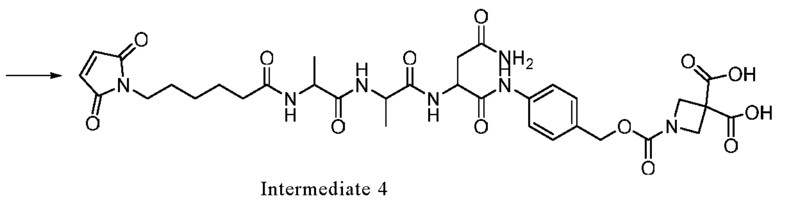

Intermediate 4

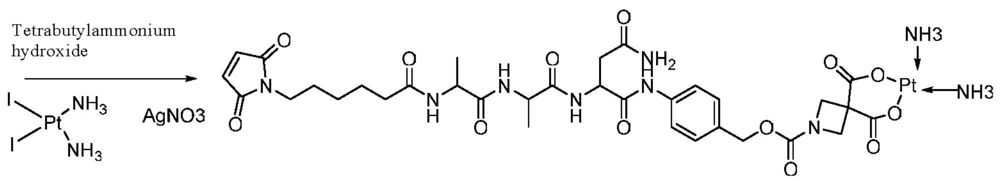


1. Synthesis of Intermediate 1

Add 500mg of raw material into a 100ml three-necked flask, and add 10ml of DCM for dissolution.When the temperature is reduced to -5 °C-0 °C, 5ml TFA was added dropwise under stirring, and after reacting for 1h, the raw materials was monitored by HPLC to react completely.The solvent in the reaction solution was removed by spinning, and the remaining oil was intermediate 1.

2. Synthesis of Intermediate 2

Intermediate 1 and 1.15 g of the starting material Fmoc-AAN-PABC-PNP were added to a 100 ml single-necked flask and dissolved in 20 ml of DMF. And activate for 10 minutes under that protection of nitrogen and stirring. 0.87ml DIPEA was added into the reaction flask, and the reaction was carried out for 0.5h. After the reaction of Fmoc-AAN-PABC-PNP was completed, the DMF in the reaction solution was removed by rotation, and the crude product was dissolved in water/DMF, and then passed through a high pressure reversed-phase column to obtain 975mg of intermediate

2 with a yield of 78.6%.

3. Synthesis of Intermediate 3

400 mg of intermediate 2 was added to a 250 ml three-necked flask, THF/ETOH (4:1) 35 ml dissolved.The temperature was reduced to -5 °C-0 °C by ice-salt bath, and 202 mg of LiOH aqueous solution was added dropwise in batches. After dropping, the reaction was carried out for 3h at controlled temperature, and then the reaction was detected by HPLC. After the reaction of intermediate 2 was completed, the temperature was controlled to-5 °C-0 °C. The PH of the reaction solution was adjusted to 6-7 with 1mol/L HCL. At 25 °C-30 °C, the solvent was removed by rotation. The crude product was beaten with methyl tert-butyl ether twice, and the solid was dissolved with methanol/water. After passing through a high-pressure reversed-phase column, 230mg of intermediate 3 was obtained, with a yield of 86.7%.

4. Synthesis of Intermediate 4

235 mg of intermediate 3 and 222 mg of EMC-OSU were added in a 100 ml single-neck flask, and 30 ml of DMF was added and stirred to dissolve. It was then heated to 50°C, reacted overnight (typically 16 h) under nitrogen protection, and detected by HPLC. After the intermediate 3 was completely reacted, the DMF was removed, the crude product was dissolved by methanol/water, and 200 mg of the intermediate 4 was obtained by passing through a high-pressure reversed-phase column, and the yield is 53.6%.

5. Synthesis of the Final Product QHL-140-N-CBP

Add 200 mg of intermediate 4 to a 100 ml single-necked flask and dissolve in 20 ml of methanol. It was cooled to -20°C with liquid nitrogen, and 279 μl of tetrabutylammonium hydroxide (25% solution in methanol) was added dropwise to that flask. After dropping, naturally raise the temperature to react for 1 h, and the reaction solution was the standby solution 1.

Add 130 mg of diiododiammine platinum into a 100 ml single-necked flask, and add 30 ml of ultrapure water to dissolve, and heat to 50°C. Dropwise adding 46mg of silver nitrate aqueous solution into that flask under the conditions of avoiding light and protecting nitrogen, react for 15min, and continuously dropwise adding 46mg of silver nitrate aqueous solution into the flask. After 15 min reaction, the reaction solution was filtered with a filter membrane and transferred to a 250 ml single-necked flask, into which standby solution 1 was added dropwise at room temperature. After nitrogen replacement three times, the reaction solution was transferred to an oil bath and heated to 50 °C. After reaction overnight (usually 16 h) in the dark, the reaction solution was centrifuged and the supernatant was directly passed through a high pressure reversed phase column. The preparation was lyophilized to obtain 90 mg of product QHL-140-N CBP, with a yield of 34.5%.

**Embodiment 5: Synthesis of QHL-086-N-CBP**

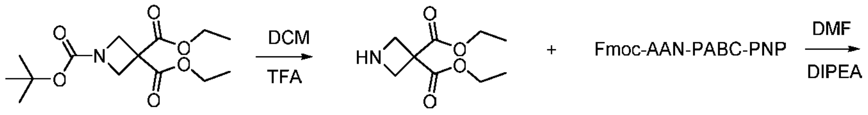


Intermediate 1

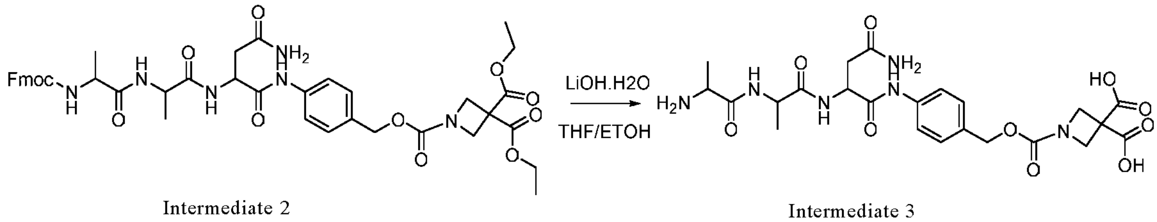


Intermediate 2

Intermediate 3

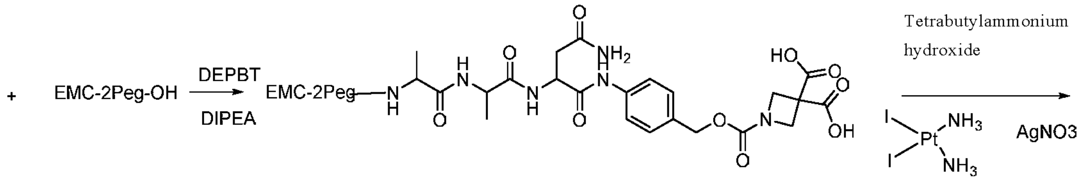


Intermediate 4

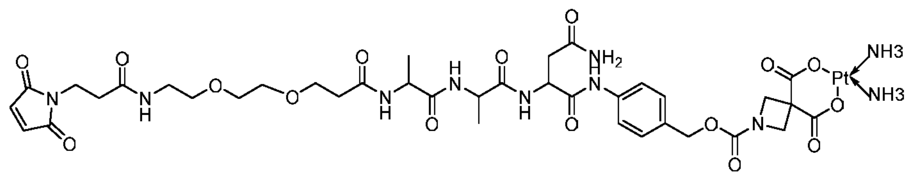

1. Synthesis of Intermediate 1

Add 500mg of raw material into a 100ml three-necked flask, and add 10ml of DCM for dissolution.When the temperature is reduced to -5 °C-0 °C, 5ml TFA was added dropwise under stirring, and after reacting for 1h, the raw materials was monitored by HPLC to react completely.The solvent in the reaction solution was removed by spinning, and the remaining oil was intermediate 1.

2. Synthesis of Intermediate 2

Intermediate 1 and 1.15 g of the raw material Fmoc-AAN-PABC-PNP were added to a 100 ml single-necked flask and dissolved in 20 ml of DMF. And activated for 10 minutes under that protection of nitrogen and stirring. Then 0.87ml DIPEA was added into the reaction flask and reacted for 0.5h. After the reaction of Fmoc-AAN-PABC-PNP was completed, DMF was removed from the reaction solution by rotation, and the crude product was dissolved in water/DMF. 975mg of intermediate 2 was obtained by high pressure reversed phase column chromatography with the yield of 78.6%.

3. Synthesis of Intermediate 3

400 mg of intermediate 2 was added to a 250 ml three-necked flask, THF/ETOH (4:1) 35 ml dissolved.The temperature was reduced to -5 °C-0 °C by ice-salt bath, and 202 mg of LiOH aqueous solution was added dropwise in batches. After dropping, the reaction was carried out for 3h at controlled temperature, and then the reaction was detected by HPLC. After the reaction of intermediate 2 was completed, the temperature was controlled to-5 °C-0 °C. The PH of the reaction solution was adjusted to 6-7 with 1mol/L HCL. At 25 °C-30 °C, the solvent was removed by rotation. The

crude product was beaten with methyl tert-butyl ether twice, and the solid was dissolved with methanol/water. After passing through a high-pressure reversed-phase column, 235mg of intermediate 3 was obtained, with a yield of 88.6%.

4. Synthesis of Intermediate 4

89 mg of EMC-2Peg-OH was added to a 100 ml single-necked flask and dissolved in DMF. Add 97 mg of DEPBT into the flask, stir and activate for 1 h at room temperature.Then add 95ul DEPBT into the flask, continue to stir for 1h, then add 150mg DMF solution of intermediate 3 into the flask in batches, stir at room temperature after dropping, detect by HPLC. After the reaction, remove DMF by rotation, dissolve the crude product with water/methanol and pass through reversed-phase high-pressure column to obtain 88mg product with the yield of 37.6%.

5. Synthesis of the Final Product QHL-086-N-CBP

Add 88 mg of intermediate 4 to a 50 ml single-necked flask and dissolve in 10 ml of methanol. It was cooled to -20°C with liquid nitrogen, and 106 μl of tetrabutylammonium hydroxide (25% solution in methanol) was added dropwise to that flask. After dropping, naturally raise the temperature to react for 1 h, and the reaction solution was the standby solution 1.

Add 49 mg of diiododiammine platinum into a 50 ml single-necked flask, and add 10 ml of ultrapure water to dissolve, and heat to 50°C. Dropwise adding 17mg of silver nitrate aqueous solution into that flask under the conditions of avoiding light and protecting nitrogen, react for 15min, and continuously dropwise adding 17mg of silver nitrate aqueous solution into the flask. After 15 min reaction, the reaction solution was filtered with a filter membrane and transferred to a 100 ml single-necked flask, into which standby solution 1 was added dropwise at room temperature. After that, nitrogen replacement three times, then the reaction solution was transferred to an oil bath and heated to 50°C. The reaction was stopped overnight (typically 16 h) protected from light until about 20% of Intermediate 4 had not reacted completely, as detected by HPLC. The reaction solution was centrifuged and the supernatant was directly passed through a high pressure reversed phase column. The preparation was lyophilized to obtain 54 mg of product QHL-086-N-CBP, with a yield of 48.6%.

**Embodiment 6: Synthesis of QHL-095-N-CBP**

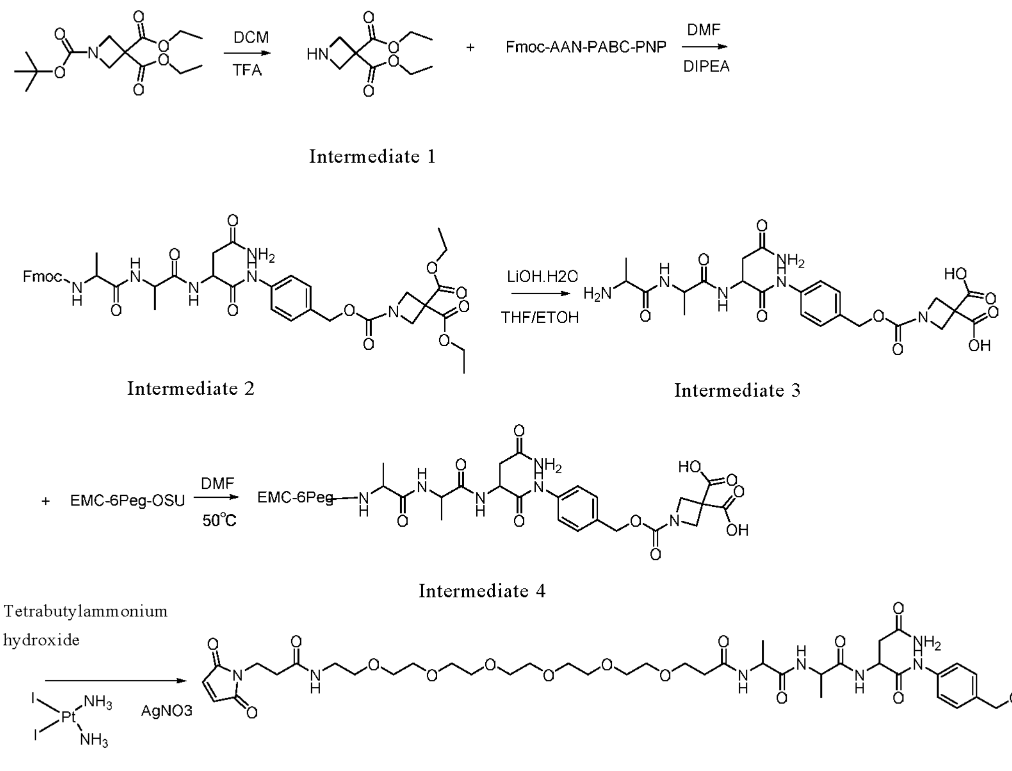

1. Synthesis of Intermediate 1

Add 500mg of raw material into a 100ml three-necked flask, and add 10ml of DCM for dissolution.When the temperature is reduced to -5 °C-0 °C, 5ml TFA was added dropwise under stirring, and after reacting for 1h, the raw materials was monitored by HPLC to react completely.The solvent in the reaction solution was removed by spinning, and the remaining oil was intermediate 1.

2. Synthesis of Intermediate 2

Intermediate 1 and 1.15 g of the raw material Fmoc-AAN-PABC-PNP were added to a 100 ml single-necked flask and dissolved in 20 ml of DMF. And activated for 10 minutes under that protection of nitrogen and stirring. Then 0.87ml DIPEA was added into the reaction flask and reacted for 0.5h, and sent it to be detected by HPLC. After the reaction of Fmoc-AAN-PABC-PNP was completed, DMF was removed from the reaction solution, and the crude product was dissolved in water/DMF. 975mg of intermediate 2 was obtained by high pressure reversed phase column chromatography with the yield of 78.6%.

3. Synthesis of Intermediate 3

400 mg of intermediate 2 was added to a 250 ml three-necked flask, THF/ETOH (4:1) 35 ml dissolved.The temperature was reduced to -5 °C-0 °C by ice-salt bath, and 202 mg of LiOH aqueous solution was added dropwise in batches. After dropping, the reaction was carried out for 3h at controlled temperature, and then the reaction was detected by HPLC. After the reaction of intermediate 2 was completed, the temperature was controlled to-5 °C-0 °C. The PH of the reaction solution was adjusted to 6-7 with 1mol/L HCL. At 25 °C-30 °C, the solvent was removed by rotation. The crude product was beaten with methyl tert-butyl ether twice, and the solid was dissolved with methanol/water. After passing through a high-pressure reversed-phase column, 235mg of intermediate 3 was obtained, with a yield of 88.6%.

4. Synthesis of Intermediate 4

180mg of intermediate 3 and 240mg of EMC-6Peg-OSU were added to a 100ml single-neck bottle, and then 20ml of DMF was added, stirred and dissolved, and then heated to 50°C. The reaction was carried out overnight (usually 16h) under nitrogen protection, and sent to HPLC for detection until the reaction of intermediate 3 was completed. DMF was spun off, the crude product was dissolved in methanol/water, and passed through a high-pressure reverse-phase column to obtain 234 mg of intermediate 4 with a yield of 69.2%.

5. Synthesis of the Final Product QHL-095-N-CBP

Add 234 mg of intermediate 4 to a 100 ml single-necked flask and dissolve in 15 ml of methanol. It was cooled to -20°C with liquid nitrogen, and 234 μl of tetrabutylammonium hydroxide (25% solution in methanol) was added dropwise to that flask. After dropping, naturally raise the temperature to react for 1 h, and the reaction solution was the standby solution 1.

Add 109 mg of diiododiammine platinum into a 100 ml single-necked flask, and add 20 ml of ultrapure water to dissolve, and heat to 50°C. Dropwise adding 38mg of silver nitrate aqueous solution into that flask under the conditions of avoiding light and protecting nitrogen, react for 15min, and continuously dropwise adding 38mg of silver nitrate aqueous solution into the flask. After 15 min reaction, the reaction solution was filtered with a filter membrane and transferred to a 250 ml single-necked flask, into which standby solution 1 was added dropwise at room temperature. After that, nitrogen replacement three times, then the reaction solution was transferred to an oil bath and heated to 50 °C. The reaction was stopped overnight (typically 16 h) protected from light. The reaction solution was centrifuged and the supernatant was directly passed through a high pressure reversed phase column. The preparation was lyophilized to obtain 138 mg of final product with a yield of 48%.

**Embodiment 7: Synthesis of QHL-006-DOX**

The synthetic route of the MI-S group in QHL-006 is shown below:

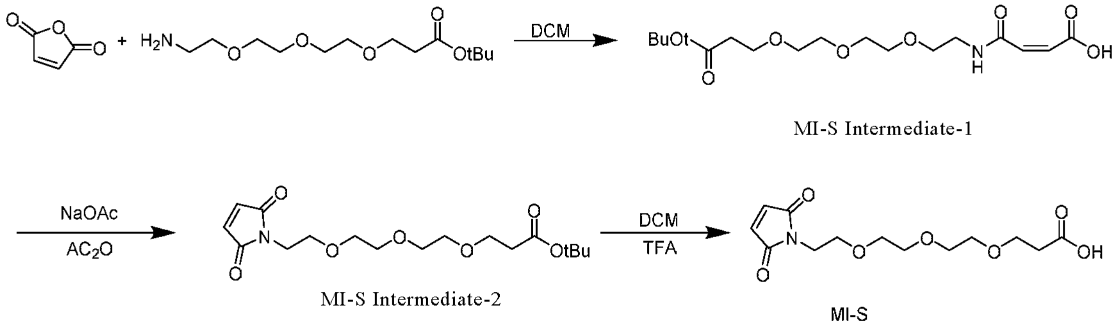


MI-S Intermediate-1

MI-S Intermediate-2

MI-S

1. Synthesis of MI-S Intermediate-1 in QHL-006-DOX

Maleic anhydride (245 mg, 2.5 mmol) was weighed into a dry and clean 100 ml single-neck reaction flask, and then 10 ml of dichloromethane was added, stirred and dissolved. NH2H2H2-3Peg-COOtBu (624mg, 2.25mmol) was added and reacted for 6 hours at room temperature. The reaction was monitored by LC-MS until the maleic anhydride was completely reacted. The reaction solution was dried by spin drying and the silica gel was stirred and passed through a column to give MI-S intermediate-1 (456 mg, yield 48.6%).

2. Synthesis of MI-S Intermediate-2 in QHL-006-DOX

Add 456 mg of MI-S intermediate-1 obtained in the above step into a 100 ml single-necked reaction flask, and then add 10 ml of acetic anhydride and stir for dissolution. NaOAC (98.7 mg, 1.216 mmol) was added slowly in batches and heated to 110 °C in oil bath to react for 3 h. The reaction was monitored by LC-MS until the MI-S intermediate-1 was completely reacted. After the reaction solution was cooled to room temperature, MI-S intermediate-2 (312, yield 70%) was obtained by spin-drying and purification by column chromatography.

3. Synthesis of MI-S in QHL-006-DOX

The MI-S intermediate-2 (312 mg, 0.87 mmol) obtained in the previous step was added to a 100 ml single-necked reaction flask, and 10 ml of dichloromethane was added to dissolve it. 2 ml of TFA and 0.15 ml of water were added dropwise, the reaction was carried out at room temperature for 30 min, and the completion of the reaction was monitored by TLC. The solvent was evaporated under reduced pressure, slurried by adding methyl tert-butyl ether, and suction filtered to obtain a solid, which was mixed with silica gel and passed through a reversed-phase column to obtain 196 mg of the product. The yield thereof was found to be 75%.

The final product was prepared by a method similar to the synthesis of QHL-095-DOX, using different MI-S for ligation (the preparation of MI-S refers to the synthesis process of MI-S in QHL-006-DOX).

**Embodiments 8: Synthesis of QHL-096-DOX**

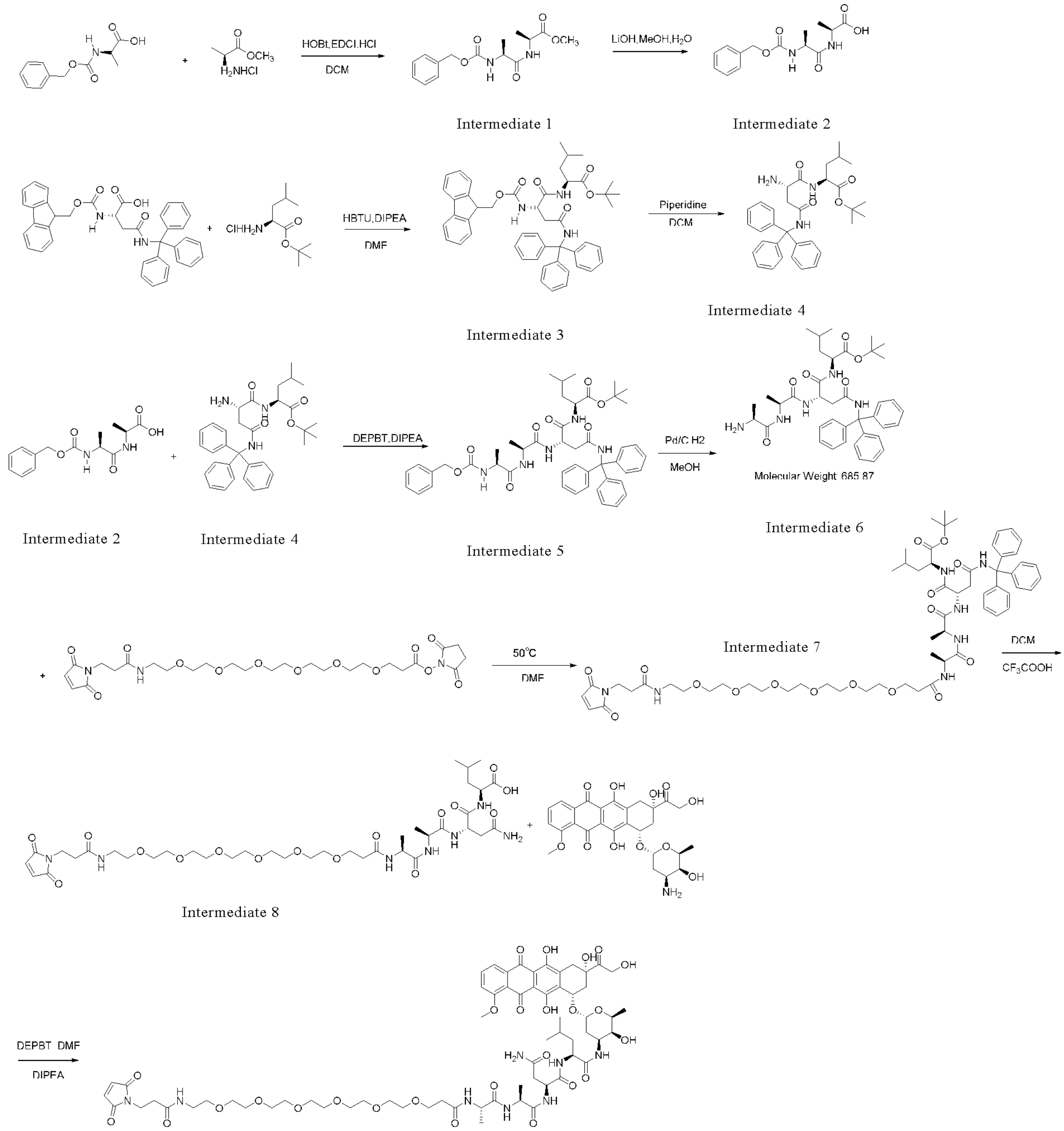

Intermediate 1
Intermediate 2
Intermediate 3
Intermediate 4
Intermediate 2
Intermediate 4
Intermediate 5
Intermediate 6
Intermediate 7
Intermediate 8

1) Synthesis of Intermediate 1

Dissolve N-benzyloxycarbonyl-L-alanine (100g, 0.45mol) in dry N,N-dimethylformamide (3L), add 1-hydroxybenzotriazole (72.6g, 0.54mol)) and 1-ethyl-(3-dimethylaminopropyl)carbodiimide hydrochloride (103.3g, 0.54mol) with stirring. After 1 hour of reaction, N, N-dimethylformamide (1 L) solution of L-alanine methyl ester (46.2 g, 0.45 mol) and N, N-diisopropylethylamine (173.8 g, 1.34 mol) was added dropwise at 0°C in an ice bath , after that, stir at room temperature for 10 hours. Then, evaporating the solvent under reduced pressure, dissolving the crude product in dichloromethane (2 L), washing with saturated ammonium chloride solution, water and saturated sodium chloride solution in turn, drying the organic phase with anhydrous sodium sulfate, evaporating the solvent under reduced pressure, recrystallizing the crude product with ethyl acetate/petroleum ether to obtain the pure product, namely intermediate 1 (101 g of white solid with a yield of 73.1%).

2) Synthesis of Intermediate 2

Intermediate 1 (100 g, 0.34 mol) was dissolved in a mixed solution of tetrahydrofuran (2 L) and water (1 L),

cooled to 0 °C, and 1 mol/L lithium hydroxide solution (400 mL) was added dropwise. It was stirred and reacted for 10 hours, and then concentrated hydrochloric acid was added dropwise to neutralize to pH<6. The tetrahydrofuran was evaporated under reduced pressure, the remaining aqueous phase was extracted with dichloromethane (1L × 3), the organic phase was dried over anhydrous sodium sulfate, and evaporated to dryness under reduced pressure to obtain Intermediate 2 (88 g white solid with a yield of 92.2%).

3) Synthesis of Intermediate 3

In a three-necked flask, L-leucine tert-butyl ester (22.4g, 0.1mol), N-Fmoc-N'-trityl asparagine (59.6g, 0.1mol) were dissolved in N,N-bismuth methylformamide (1000mL), stirred and added 1-hydroxybenzotriazole (14.85g, 0.11mol) and 1-ethyl-(3-dimethylaminopropyl)carbodiimide hydrochloride (23g, 0.12mol). After ice bathed to 0°C, additional N, N-diisopropylethylamine (25.8 g, 0.2 mol) was added. After stirring for 10 hour, that solvent was distilled off under reduced pressure, the crude product was dissolved in chloroform (1000 ml), washed successively with saturated ammonium chloride solution, saturated sodium chloride solution and water, the organic phase was dried over anhydrous sodium sulfate, filtered and the solvent was distilled off under reduced pressure.The obtained crude product was recrystallized (dichloromethane: ethyl acetate =1:1) to give intermediate 3 (42.4 g of a white solid with a yield of 55.4%).

4) Synthesis of Intermediate 4

Intermediate 3 (7.65 g, 0.01 mol) was dissolved in a mixture of dichloromethane (100 mL) and N,N-dimethylformamide (100 mL).Piperidine (40 ml) was added and after stirred at room temperature for 5 hours, the solvent was distilled off under reduced pressure and then dried in a vacuum oven under high vacuum to remove a small amount of piperidine to give Intermediate 4 as a pale yellow solid which was used in the next step without purification.

5) Synthesis of Intermediate 5

The crude intermediate 4 obtained in the previous step was dissolved in N,N-dimethylformamide (200 mL), followed by the addition of intermediate 2 (2.94 g, 0.012 mol), benzotriazole-N,N,N', N'-tetramethylurea hexafluorophosphate (HBTU) (6.07 g, 0.016 mol). After ice bathing to 0°C, N, N-diisopropylethylamine (2.6 g, 0.02 mol) was added, and the mixture was stirred at room temperature overnight. The solvent was evaporated under reduced pressure, the residue was dissolved in chloroform (100 ml), washed successively with saturated ammonium chloride solution and saturated sodium chloride solution, dried over anhydrous sodium sulfate and filtered, and the solvent was evaporated. The obtained crude product was subjected to silica gel column chromatography Intermediate 5 (3.1 g white solid, total yield of the first two steps: 37.8%) was obtained.

6) Synthesis of Intermediate 6

Cbz-AAN(trt)-L-Otbu (3.00 g, 3.65 mmol) was dissolved in methanol (100 mL), 10% palladium on charcoal (0.3 g) was added thereto, and hydrogen gas was introduced. The reaction was stirred at normal temperature and normal pressure for 4 hours, palladium on charcoal was removed by filtration, washed with methanol, that filtrate and the washings were combined, and the solvent was distilled off under reduced pressure to obtain intermediate 6 (2.38 g of a white solid with a yield of 95.2%).

7) Synthesis of Intermediate 7

Intermediate 6 (2.38 g, 3.4 mmol) and EMC-6Peg-OSu (2.4 g, 4.08 mmol) were added to a 250 ml single-necked flask, and DMF (30 ml) was added to dissolve, and heated to 50 °C for 6 h. The solvent was distilled off under reduced pressure, the crude product was dissolved in methanol, and purified by a reverse-phase high-pressure column chromatography to obtain Intermediate 7 (2.5 g with a yield of 63.2%).

8) Synthesis of Intermediate 8

Intermediate 7 (1.00 g, 0.852 mmol) was dissolved in DCM (20 mL) and trifluoroacetic acid (10 mL) was added dropwise at room temperature. It was stirred and reacted for 2 hours, and the reaction solution was monitored by HPLC. When the reaction of intermediate 1 was complete, the solvent was removed by distillation under reduced pressure. The crude product was washed twice with methyl tert-butyl ether, and the solid was dissolved in methanol and purified by a reverse-phase high-pressure column chromatography to obtain Intermediate 8 (721 mg of white solid with a yield of 96.8%).

9) Synthesis of the final product QHL-096-DOX

In a 100 mL reaction flask, add 63 mg of doxorubicin hydrochloride (1.0 eq), 95 mg of intermediate 8 (1 eq), 39 mg of DEPBT (1.2 eq) and 10 mL of DMF. Under nitrogen protection, 60ul of DIPEA (3eq) was added to the reaction mixture. After 4 hours of reaction at room temperature, the solvent was evaporated under reduced pressure. The crude product was dissolved in methanol and purified by a reverse-phase high-pressure column chromatography to obtain QHL-096-DOX (52 mg of red solid with a yield of 34.2%). 7) Synthesis of Intermediate 7 Intermediate 6 (1.00 g, 1.46 mmol) was dissolved in DCM (20 mL) and trifluoroacetic acid (10 mL) was added dropwise at room temperature. It was stirred and reacted for 2 hours, and the reaction solution was monitored by HPLC. When the reaction of intermediate 1 was complete, the solvent was removed by distillation under reduced pressure. The crude product was washed twice with methyl tert-butyl ether, and the solid was dissolved in methanol and purified by a reverse-phase high-pressure column chromatography to obtain Intermediate 7 (546 mg of white solid with a yield of 96.8%).

**Embodiment 9: Synthesis of QHL-117-DOX**

The synthetic route of QHL-117 is shown below:

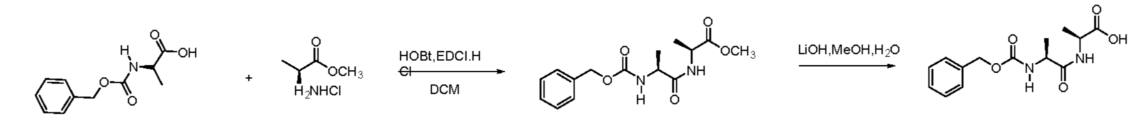


Intermediate 1

Intermediate 2

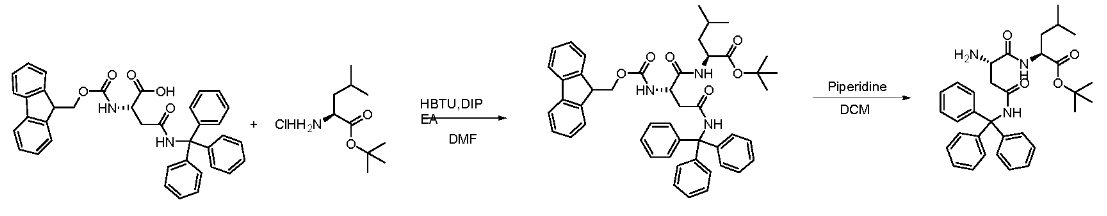


Intermediate 4

Intermediate 3

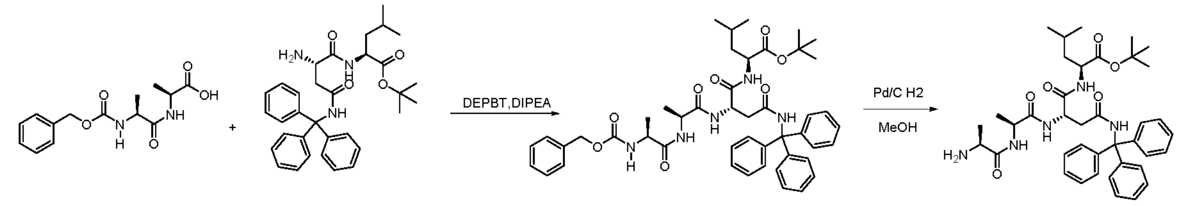


Intermediate 2 Intermediate 4 Intermediate 5 Intermediate 6

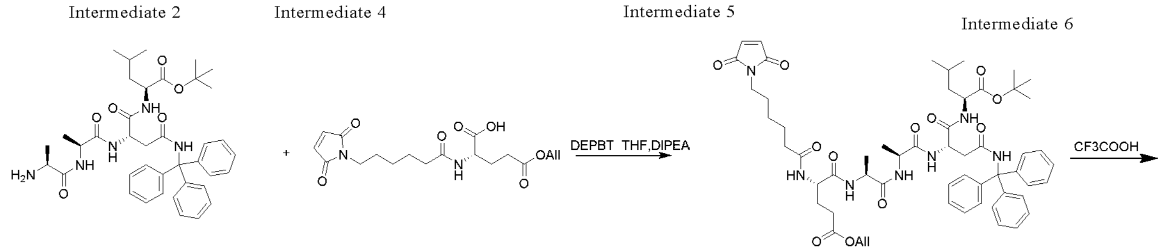


Intermediate 6

Intermediate 7

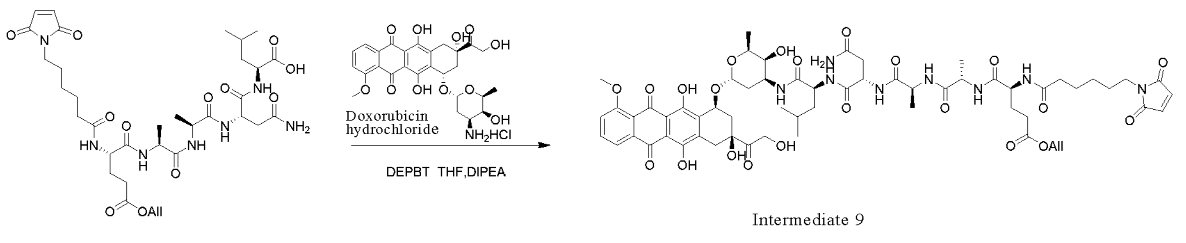


Intermediate 9

Intermediate 8

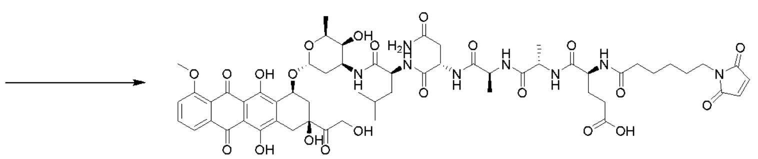

1) Synthesis of Intermediate 1

Dissolve N-benzyloxycarbonyl-L-alanine (100g, 0.45mol) in dry N, N-dimethylformamide (3L), add 1-hydroxybenzotriazole (72.6g, 0.54mol)) and 1-ethyl-(3-dimethylaminopropyl) carbodiimide hydrochloride (103.3g, 0.54mol) with stirring. After 1 hour of reaction, N, N-dimethylformamide (1 L) solution of L-alanine methyl ester (46.2 g, 0.45 mol) and N, N-diisopropylethylamine (173.8 g, 1.34 mol) was added dropwise at 0°C in an ice bath, after that, stir at room temperature for 10 hours. Then, evaporating the solvent under reduced pressure, dissolving the crude product in dichloromethane (2 L), washing with saturated ammonium chloride solution, water and saturated sodium chloride solution in turn, drying the organic phase with anhydrous sodium sulfate, evaporating the solvent under reduced pressure, recrystallizing the crude product with ethyl acetate/petroleum ether to obtain the pure product, namely intermediate 1 (101 g

of white solid with a yield of 73.1%).

2) Synthesis of Intermediate 2

Intermediate 1 (100 g, 0.34 mol) was dissolved in a mixed solution of tetrahydrofuran (2 L) and water (1 L), cooled to 0°C, and 1 mol/L lithium hydroxide solution (400 mL) was added dropwise. It was stirred and reacted for 10 hours, and then concentrated hydrochloric acid was added dropwise to neutralize to pH<6. The tetrahydrofuran was evaporated under reduced pressure, the remaining aqueous phase was extracted with dichloromethane (1L × 3), the organic phase was dried over anhydrous sodium sulfate, and evaporated to dryness under reduced pressure to obtain Intermediate 2 (88 g white solid with a yield of 92.2%).

3) Synthesis of Intermediate 3

In a three-necked flask, L-leucine tert-butyl ester (22.4g, 0.1mol), N-Fmoc-N'-trityl asparagine (59.6g, 0.1mol) were dissolved in N,N-bismuth methylformamide (1000mL), stirred and added 1-hydroxybenzotriazole (14.85g, 0.11mol) and 1-ethyl-(3-dimethylaminopropyl)carbodiimide hydrochloride (23g, 0.12mol). After ice bathed to 0°C, additional N, N-diisopropylethylamine (25.8 g, 0.2mol) was added. After stirring for 10 hour, that solvent was distilled off under reduced pressure, the crude product was dissolved in chloroform (1000 ml), washed successively with saturated ammonium chloride solution, saturated sodium chloride solution and water, the organic phase was dried over anhydrous sodium sulfate, filtered and the solvent was distilled off under reduced pressure.The obtained crude product was recrystallized (dichloromethane: ethyl acetate =1:1) to give intermediate 3 (42.4 g of a white solid with a yield of 55.4%).

4) Synthesis of Intermediate 4

Intermediate 3 (7.65 g, 0.01 mol) was dissolved in a mixture of dichloromethane (100 mL) and N, N-dimethylformamide (100 mL). Piperidine (40 ml) was added and after stirred at room temperature for 5 hours, the solvent was distilled off under reduced pressure and then dried in a vacuum oven under high vacuum to remove a small amount of piperidine to give Intermediate 4 as a pale yellow solid which was used in the next step without purification.

5) Synthesis of Intermediate 5

The crude intermediate 4 obtained in the previous step was dissolved in N, N-dimethylformamide (200 mL), followed by the addition of intermediate 2 (2.94 g, 0.012 mol), benzotriazole-N, N, N', N'-tetramethylurea hexafluorophosphate (HBTU) (6.07 g, 0.016 mol). After ice bathing to 0°C, N, N-diisopropylethylamine (2.6 g, 0.02mol) was added, and the mixture was stirred at room temperature overnight. The solvent was evaporated under reduced pressure, the residue was dissolved in chloroform (100 ml), washed successively with saturated ammonium chloride solution and saturated sodium chloride solution, dried over anhydrous sodium sulfate and filtered, and the solvent was evaporated. The obtained crude product was subj ected to silica gel column chromatography Intermediate 5 (3.1 g white solid, total yield of the first two steps: 37.8%) was obtained.

6) Synthesis of Intermediate 6

Cbz-AAN (trt)-L-Otbu (3.00 g, 3.65 mmol) was dissolved in methanol (100 mL), 10% palladium on charcoal (0.3 g) was added thereto, and hydrogen gas was introduced. The reaction was stirred at normal temperature and normal pressure for 4 hours, palladium on charcoal was removed by filtration, washed with methanol, that filtrate and the washings were combined, and the solvent was distilled off under reduced pressure to obtain intermediate 6 (2.38 g of a white solid with a yield of 95.2%).

7) Synthesis of Intermediate 7

15ml THF, (2.387g, 3.4mmol) intermediate 6 and 1.35g DEPBT were sequentially added to a dry and clean 250ml single-necked reaction flask. The reaction was carried out at room temperature for 10 min, and thenEMC-Glu (OAll)-COOH (1.3 g, 3.4 mmol) was added. Protected by nitrogen ventilation, and reacted at room temperature for 15 min. After adding DIPEA 1.8ml dropwise, nitrogen ventilation protection, the reaction was carried out at room temperature for 3 hours, the solvent was evaporated under reduced pressure, water was added for 2-3 times beating, suction filtration to obtain 700mg of light yellow solid, which was purified by column to obtain product 2.2g with a yield of 63.2%.

8) Synthesis of Intermediate 8

Intermediate 7 (1.53 g, 1.46 mmol) was dissolved in DCM (20 mL) and trifluoroacetic acid (10 mL) was added dropwise at room temperature. It was stirred and reacted for 2 hours, and the reaction solution was monitored by HPLC. When the reaction of intermediate 1 was complete, the solvent was removed by distillation under reduced pressure. The crude product was washed twice with methyl tert-butyl ether, and the solid was dissolved in methanol and purified by a reverse-phase high-pressure column chromatography to obtain Intermediate 8 (928 mg of white solid with a yield of 84.8%).

9) Synthesis of Intermediate 9

In a 100 mL reaction flask, 510.4 mg of doxorubicin hydrochloride (1.0 eq, 0.88 mmol), 659 mg of intermediate 8 (1.0 eq, 0.88 mmol) were added. The reaction was carried out at room temperature for 15 min under nitrogen protection. 78 μl of DIPEA was added dropwise, and after 4 hours of reaction at room temperature, the solvent was evaporated under reduced pressure, the crude product was dissolved in methanol, and purified by a reverse-phase high-pressure column chromatography to obtain Intermediate 9 (258 mg of red solid with a yield of 23.8%). 10) Synthesis of the final product

In a 100 mL reaction flask, THF 15 ml, intermediate 9 (258 mg, 0.202 mmol), n-butyltin hydride (175.7 mg, 0.606 mmol) were successively added, and the reaction solution was saturated with nitrogen. Then tetrakis (triphenylphosphine) palladium(0) (32.7 mg, 0.028 mmol) was added and the mixture was stirred at room temperature overnight. Monitor by TLC until conversion was completed. The content of that flask were then filtered through celite and the residue was washed with THF. The filtrate was concentrated under reduced pressure. The obtained crude product was purified by column to obtain 224 mg (yield: 90%) of the target compound.

The other compounds in Table 1 below were prepared in a similar manner to embodiments 1-2, 4-9 using different MI, S, C, A and D parts.

The compounds were verified by mass spectrometry (MS) and their molecular weights are shown in Table 1, which are in agreement with the calculated molecular weights based on their structures.

Table 1

| Compound No. | Molecular weight | MS | Traits | Yield |
|---|---|---|---|---|
| QHL-001-DOX | 1143.16 | 1143 | Red solid | 71mg |
| QHL-002-DOX | 1187.22 | 1187 | Red solid | 49mg |
| QHL-003-DOX | 1231.27 | 1231 | Red solid | 112mg |
| QHL-004-DOX | 1319.37 | 1319 | Red solid | 93mg |
| QHL-005-DOX | 1144.15 | 1144 | Red solid | 37mg |
| QHL-006-DOX | 1188.21 | 1188 | Red solid | 46mg |
| QHL-007-DOX | 1232.26 | 1232 | Red solid | 158mg |
| QHL-008-DOX | 1320.36 | 1320 | Red solid | 102mg |
| QHL-009-DOX | 1152.17 | 1152 | Red solid | 34mg |
| QHL-010-DOX | 1196.23 | 1196 | Red solid | 28mg |
| QHL-011-DOX | 1240.28 | 1240 | Red solid | 18mg |
| QHL-012-DOX | 1328.38 | 1328 | Red solid | 31mg |
| QHL-013-DOX | 1272.27 | 1272 | Red solid | 180mg |
| QHL-014-DOX | 1316.33 | 1316 | Red solid | 105mg |
| QHL-Q15-DOX | 1360.38 | 1360 | Red solid | 214mg |
| QHL-016-DOX | 1448.48 | 1448 | Red solid | 54mg |
| QHL-Q17-DOX | 1273.26 | 1273 | Red solid | 189mg |
| QHL-018-DOX | 1317.32 | 1317 | Red solid | 167mg |
| QHL-019-DOX | 1361.37 | 1361 | Red solid | 102mg |

(continued)

| Compound No. | Molecular weight | MS | Traits | Yield |
|---|---|---|---|---|
| QHL-020-DOX | 1449.47 | 1449 | Red solid | 81mg |
| QHL-021-DOX | 1281.28 | 1281 | Red solid | 106mg |
| QHL-022-DOX | 1325.34 | 1325 | Red solid | 97mg |
| QHL-023-DOX | 1369.39 | 1369 | Red solid | 139mg |
| QHL-024-DOX | 1457.49 | 1457 | Red solid | 76mg |
| QHL-025-DOX | 1258.24 | 1258 | Red solid | 143mg |
| QHL-026-DOX | 1302.3 | 1302 | Red solid | 125mg |
| QHL-027-DOX | 1346.35 | 1346 | Red solid | 136mg |
| QHL-028-DOX | 1434.45 | 1434 | Red solid | 121mg |
| QHL-029-DOX | 1259.23 | 1259 | Red solid | 223mg |
| QHL-030-DOX | 1303.29 | 1303 | Red solid | 184mg |
| QHL-031-DOX | 1347.34 | 1347 | Red solid | 98mg |
| QHL-032-DOX | 1435.44 | 1435 | Red solid | 131mg |
| QHL-033-DOX | 1267.25 | 1267 | Red solid | 135mg |
| QHL-034-DOX | 1311.31 | 1311 | Red solid | 154mg |
| QHL-035-DOX | 1355.36 | 1355 | Red solid | 164mg |
| QHL-036-DOX | 1443.46 | 1443 | Red solid | 182mg |
| QHL-037-DOX | 1343.35 | 1343 | Red solid | 155mg |
| QHL-038-DOX | 1344.34 | 1344 | Red solid | 169mg |
| QHL-039-DOX | 1352.36 | 1352 | Red solid | 156mg |
| QHL-040-DOX | 1329.32 | 1329 | Red solid | 231mg |
| QHL-041-DOX | 1330.31 | 1330 | Red solid | 143mg |
| QHL-042-DOX | 1338.33 | 1338 | Red solid | 157mg |
| QHL-043-DOX | 1387.41 | 1387 | Red solid | 241mg |
| QHL-044-DOX | 1388.4 | 1388 | Red solid | 185mg |
| QHL-045-DOX | 1396.42 | 1396 | Red solid | 174mg |
| QHL-046-DOX | 1373.38 | 1373 | Red solid | 169mg |
| QHL-047-DOX | 1374.37 | 1374 | Red solid | 64mg |
| QHL-048-DOX | 1382.39 | 1382 | Red solid | 105mg |
| QHL-049-DOX | 1431.46 | 1431 | Red solid | 98mg |
| QHL-050-DOX | 1432.45 | 1432 | Red solid | 216mg |
| QHL-Q51-DOX | 1440.47 | 1440 | Red solid | 198mg |
| QHL-052-DOX | 1417.43 | 1417 | Red solid | 183mg |
| QHL-053-DOX | 1418.42 | 1418 | Red solid | 175mg |
| QHL-054-DOX | 1426.44 | 1426 | Red solid | 168mg |
| QHL-055-DOX | 1519.56 | 1520 | Red solid | 156mg |
| QHL-056-DOX | 1520.55 | 1521 | Red solid | 141mg |
| QHL-057-DOX | 1528.57 | 1529 | Red solid | 139mg |

(continued)

| Compound No. | Molecular weight | MS | Traits | Yield |
|---|---|---|---|---|
| QHL-058-DOX | 1505.53 | 1506 | Red solid | 145mg |
| QHL-059-DOX | 1506.52 | 1507 | Red solid | 182mg |
| QHL-060-DOX | 1514.54 | 1515 | Red solid | 163mg |
| QHL-061-DOX | 1357.38 | 1357 | Red solid | 196mg |
| QHL-062-DOX | 1358.37 | 1358 | Red solid | 175mg |
| QHL-063-DOX | 1366.39 | 1366 | Red solid | 154mg |
| QHL-064-DOX | 1343.35 | 1343 | Red solid | 139mg |
| QHL-065-DOX | 1344.34 | 1344 | Red solid | 28mg |
| QHL-066-DOX | 1352.36 | 1352 | Red solid | 18mg |
| QHL-067-DOX | 1401.44 | 1401 | Red solid | 31mg |
| QHL-068-DOX | 1402.43 | 1402 | Red solid | 164mg |
| QHL-069-DOX | 1410.45 | 1410 | Red solid | 84mg |
| QHL-070-DOX | 1387.41 | 1387 | Red solid | 115mg |
| QHL-071-DOX | 1388.4 | 1388 | Red solid | 54mg |
| QHL-072-DOX | 1396.42 | 1396 | Red solid | 189mg |
| QHL-073-DOX | 1445.49 | 1445 | Red solid | 167mg |
| QHL-074-DOX | 1446.48 | 1446 | Red solid | 102mg |
| QHL-075-DOX | 1454.5 | 1455 | Red solid | 81mg |
| QHL-076-DOX | 1431.46 | 1431 | Red solid | 106mg |
| QHL-077-DOX | 1432.45 | 1432 | Red solid | 97mg |
| QHL-078-DOX | 1440.47 | 1440 | Red solid | 139mg |
| QHL-079-DOX | 1533.59 | 1534 | Red solid | 76mg |
| QHL-080-DOX | 1534.58 | 1535 | Red solid | 143mg |
| QHL-081-DOX | 1542.6 | 1543 | Red solid | 125mg |
| QHL-082-DOX | 1519.56 | 1520 | Red solid | 136mg |
| QHL-083-DOX | 1520.55 | 1521 | Red solid | 121mg |
| QHL-084-DOX | 1528.57 | 1529 | Red solid | 223mg |
| QHL-085-DOX | 1214.24 | 1214 | Red solid | 184mg |
| QHL-086-DOX | 1215.23 | 1215 | Red solid | 74mg |
| QHL-087-DOX | 1223.25 | 1223 | Red solid | 121mg |
| QHL-088-DOX | 1258.3 | 1258 | Red solid | 157mg |
| QHL-089-DOX | 1259.29 | 1259 | Red solid | 84mg |
| QHL-090-DOX | 1267.31 | 1267 | Red solid | 164mg |
| QHL-091-DOX | 1302.35 | 1302 | Red solid | 182mg |
| QHL-092-DOX | 1303.34 | 1303 | Red solid | 155mg |
| QHL-093-DOX | 1311.36 | 1311 | Red solid | 169mg |
| QHL-094-DOX | 1390.45 | 1390 | Red solid | 156mg |
| QHL-095-DOX | 1391.44 | 1391 | Red solid | 49mg |

(continued)

| Compound No. | Molecular weight | MS | Traits | Yield |
|---|---|---|---|---|
| QHL-096-DOX | 1399.46 | 1399 | Red solid | 52mg |
| QHL-097-DOX | 1228.27 | 1228 | Red solid | 157mg |
| QHL-098-DOX | 1229.26 | 1229 | Red solid | 137mg |
| QHL-099-DOX | 1237.28 | 1237 | Red solid | 49mg |
| QHL-100-DOX | 1272.33 | 1272 | Red solid | 67mg |
| QHL-101-DOX | 1273.32 | 1273 | Red solid | 71mg |
| QHL-102-DOX | 1281.34 | 1281 | Red solid | 49mg |
| QHL-103-DOX | 1316.38 | 1316 | Red solid | 86mg |
| QHL-104-DOX | 1317.37 | 1317 | Red solid | 93mg |
| QHL-105-DOX | 1325.39 | 1325 | Red solid | 37mg |
| QHL-106-DOX | 1404.48 | 1404 | Red solid | 46mg |
| QHL-107-DOX | 1405.47 | 1405 | Red solid | 158mg |
| QHL-108-DOX | 1413.49 | 1413 | Red solid | 102mg |
| QHL-109-DOX | 1184.17 | 1184 | Red solid | 34mg |
| QHL-110-DOX | 1185.16 | 1185 | Red solid | 28mg |
| QHL-111-DOX | 1193.18 | 1193 | Red solid | 38mg |
| QHL-112-DOX | 1170.14 | 1170 | Red solid | 31mg |
| QHL-113-DOX | 1171.13 | 1171 | Red solid | 104mg |
| QHL-114-DOX | 1179.15 | 1179 | Red solid | 170mg |
| QHL-115-DOX | 1226.25 | 1226 | Red solid | 118mg |
| QHL-116-DOX | 1227.24 | 1227 | Red solid | 100mg |
| QHL-117-DOX | 1235.26 | 1235 | Red solid | 224mg |
| QHL-118-DOX | 1212.22 | 1212 | Red solid | 167mg |
| QHL-119-DOX | 1213.21 | 1213 | Red solid | 102mg |
| QHL-120-DOX | 1221.23 | 1221 | Red solid | 81mg |
| QHL-121-DOX | 1163.18 | 1163 | Red solid | 106mg |
| QHL-122-DOX | 1177.22 | 1177 | Red solid | 97mg |
| QHL-123-DOX | 1205.28 | 1205 | Red solid | 139mg |
| QHL-124-DOX | 1219.31 | 1219 | Red solid | 76mg |
| QHL-125-DOX | 1219.31 | 1219 | Red solid | 143mg |
| QHL-126-DOX | 1237.34 | 1237 | Red solid | 125mg |
| QHL-127-DOX | 1239.3 | 1239 | Red solid | 136mg |
| QHL-128-DOX | 1278.33 | 1278 | Red solid | 121mg |
| QHL-129-DOX | 1193.22 | 1193 | Red solid | 64mg |
| QHL-130-DOX | 1207.25 | 1207 | Red solid | 184mg |
| QHL-131-DOX | 1209.28 | 1209 | Red solid | 164mg |
| QHL-132-DOX | 1269.32 | 1269 | Red solid | 144mg |
| QHL-133-DOX | 1220.25 | 1220 | Red solid | 104mg |

(continued)

| Compound No. | Molecular weight | MS | Traits | Yield |
|---|---|---|---|---|
| QHL-134-DOX | 1234.28 | 1234 | Red solid | 95mg |
| QHL-135-DOX | 1234.32 | 1234 | Red solid | 164mg |
| QHL-136-DOX | 1262.33 | 1262 | Red solid | 182mg |
| QHL-137-DOX | 1243.29 | 1243 | Red solid | 155mg |
| QHL-140-N-CBP | 970.86 | 971 | Light yellow solid | 90mg |
| QHL-143-N-CBP | 942.8 | 943 | Light yellow solid | 41mg |
| QHL-095-N-CBP | 1264.17 | 1264 | Light yellow solid | 138mg |
| QHL-092-N-CBP | 1176.07 | 1176 | Light yellow solid | 88mg |
| QHL-089-N-CBP | 1132.01 | 1132 | Light yellow solid | 43mg |
| QHL-107-N-CBP | 1278.2 | 1278 | Light yellow solid | 55mg |
| QHL-104-N-CBP | 1204.12 | 1204 | Light yellow solid | 150mg |
| QHL-101-N-CBP | 1146.04 | 1146 | Light yellow solid | 142mg |
| QHL-098-N-CBP | 1101.99 | 1102 | Light yellow solid | 95mg |
| QHL-146-N-CBP | 914.75 | 915 | Light yellow solid | 116mg |
| QHL-086-N-CBP | 1087.96 | 1088 | Light yellow solid | 54mg |
| QHL-086 - Dovitinib | 1108.16 | 1108 | Light yellow solid | 65mg |
| QHL-089 - Dovitinib | 1152.21 | 1152 | Light yellow solid | 86mg |
| QHL-086-Epirubicin | 1259.24 | 1259 | Red solid | 78mg |
| QHL-089-Epirubicin | 1303.3 | 1303 | Red solid | 90mg |
| QHL-086-Compound a | 1223.62 | 1224 | Light yellow solid | 134mg |
| QHL-089-Compound a | 1267.67 | 1268 | Light yellow solid | 45mg |
| QHL-086-Compound b | 1241.61 | 1242 | Light yellow solid | 98mg |
| QHL-089-Compound b | 1285.66 | 1286 | Light yellow solid | 41mg |
| QHL-086-Mitomycin | 1050.05 | 1050 | Grayish blue solid powder | 85mg |
| QHL-089-Mitomycin | 1094.1 | 1094 | Grayish blue solid powder | 58mg |
| QHL-086-Dabrafenib | 1235.28 | 1235 | Light yellow solid | 73mg |
| QHL-089-Dabrafenib | 1279.33 | 1279 | Light yellow solid | 81mg |
| QHL-086-Motesani | 1133.23 | 1133 | Light yellow solid | 118mg |
| QHL-089-Motesani | 1089.18 | 1089 | Light yellow solid | 44mg |
| QHL-138-N-CBP | 934.86 | 935 | Light yellow solid | 95mg |
| QHL-141-N-CBP | 906.8 | 907 | Light yellow solid | 53mg |
| QHL-096-N-CBP | 1228.17 | 1228 | Light yellow solid | 70mg |
| QHL-093-N-CBP | 1140.07 | 1140 | Light yellow solid | 135mg |
| QHL-090-N-CBP | 1096.01 | 1096 | Light yellow solid | 90mg |
| QHL-108-N-CBP | 1242.2 | 1242 | Light yellow solid | 37mg |
| QHL-105-N-CBP | 1168.12 | 1168 | Light yellow solid | 65mg |
| QHL-102-N-CBP | 1110.04 | 1110 | Light yellow solid | 136mg |
| QHL-099-N-CBP | 1065.99 | 1066 | Light yellow solid | 41mg |

(continued)

| Compound No. | Molecular weight | MS | Traits | Yield |
|---|---|---|---|---|
| QHL-144-N-CBP | 878.75 | 879 | Light yellow solid | 118mg |
| QHL-087-N-CBP | 1051.96 | 1052 | Light yellow solid | 53mg |
| QHL-087- Dovitinib | 1072.16 | 1072 | Light yellow solid | 38mg |
| QHL-090- Dovitinib | 1116.21 | 1116 | Light yellow solid | 82mg |
| QHL-087-Epirubicin | 1223.24 | 1223 | Red solid | 73mg |
| QHL-090-Epirubicin | 1267.3 | 1267 | Red solid | 69mg |
| QHL-087-Compound a | 1187.62 | 1188 | Light yellow solid | 117mg |
| QHL-090-Compound a | 1231.67 | 1232 | Light yellow solid | 115mg |
| QHL-087-Compound b | 1205.61 | 1206 | Light yellow solid | 115mg |
| QHL-090-Compound b | 1249.66 | 1250 | Light yellow solid | 116mg |
| QHL-087-mitomycin | 1014.05 | 1014 | Grayish blue solid powder | 55mg |
| QHL-090-mitomycin | 1058.1 | 1058 | Grayish blue solid powder | 78mg |
| QHL-087-Dabrafenib | 1199.28 | 1199 | Light yellow solid | 61mg |
| QHL-090-Dabrafenib | 1243.33 | 1243 | Light yellow solid | 102mg |
| QHL-087-Motseni | 1097.23 | 1097 | Light yellow solid | 40mg |
| QHL-090-Motseni | 1053.18 | 1053 | Light yellow solid | 85mg |
| QHL-140- Resiquimod | 913.00 | 913 | White solid | 57mg |
| QHL-086- Resiquimod | 1030.11 | 1030 | White solid | 82mg |
| QHL-089- Resiquimod | 1074.16 | 1074 | Off-white solid | 49mg |
| QHL-092- Resiquimod | 1118.21 | 1118 | Off-white solid | 76mg |
| QHL-095- Resiquimod | 1206.32 | 1206 | Off-white solid | 47mg |
| QHL-005-Resiquimod | 959.03 | 959 | White solid | 41mg |
| QHL-006- Resiquimod | 1003.08 | 1003 | White solid | 47mg |
| QHL-008- Resiquimod | 1135.24 | 1135 | White solid | 45mg |
| QHL-147- Resiquimod | 1399.56 | 1400 | White solid | 91mg |
| QHL-116- Resiquimod | 1042.12 | 1042 | White solid | 85mg |
| QHL-119- Resiquimod | 1028.09 | 1028 | Off-white solid | 95mg |
| QHL-140-Prednisone | 956.33 | 956 | Off-white solid | 65mg |
| QHL-086-Prednisone | 1073.44 | 1073 | Off-white solid | 75mg |
| QHL-089-Prednisone | 1117.49 | 1117 | White solid | 76mg |
| QHL-092-Predni sone | 1161.54 | 1162 | White solid | 80mg |
| QHL-095-Prednisone | 1249.65 | 1250 | White solid | 57mg |
| QHL-005-Prednisone | 1002.36 | 1002 | White solid | 80mg |
| QHL-006-Prednisone | 1046.41 | 1046 | White solid | 88mg |
| QHL-008-Prednisone | 1178.57 | 1179 | White solid | 81mg |
| QHL-147-Prednisone | 1442.89 | 1443 | White solid | 86mg |
| QHL-116-Prednisone | 1085.45 | 1085 | White solid | 40mg |
| QHL-119-Prednisone | 1071.42 | 1071 | Off-white solid | 37mg |

(continued)

| Compound No. | Molecular weight | MS | Traits | Yield |
|---|---|---|---|---|
| QHL-150-T3 | 1100.44 | 1100 | Off-white solid | 68mg |
| QHL-157-T3 | 1217.55 | 1218 | Off-white solid | 95mg |
| QHL-158-T3 | 1261.6 | 1262 | Off-white solid | 86mg |
| QHL-159-T3 | 1305.65 | 1306 | Off-white solid | 76mg |
| QHL-160-T3 | 1393.76 | 1394 | Off-white solid | 55mg |
| QHL-153-T3 | 1146.47 | 1146 | Off-white solid | 76mg |
| QHL-154-T3 | 1190.52 | 1191 | Off-white solid | 80mg |
| QHL-155-T3 | 1322.68 | 1323 | White solid | 91mg |
| QHL-156-T3 | 1587 | 1587 | White solid | 42mg |
| QHL-161-T3 | 1229.56 | 1230 | White solid | 87mg |
| QHL-162-T3 | 1215.53 | 1216 | White solid | 65mg |

The present disclosure also provides the following comparative compounds of the formula:

Compound C1: Doxorubicin

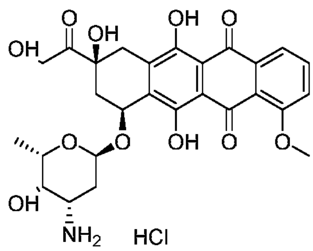

Compound C2: AANL-DOX

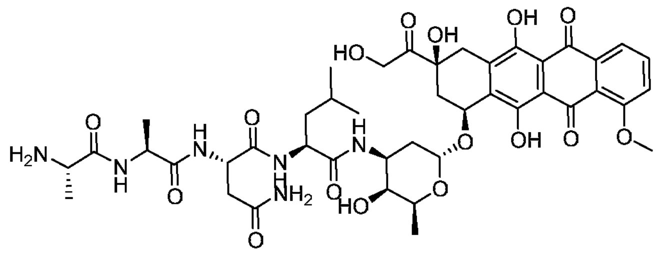

Compound C3: EMC-AANL-DOX

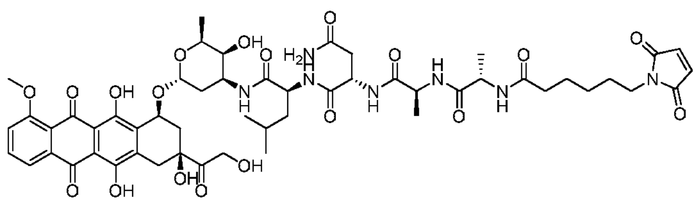

Compound C4: Peg-AANL-DOX

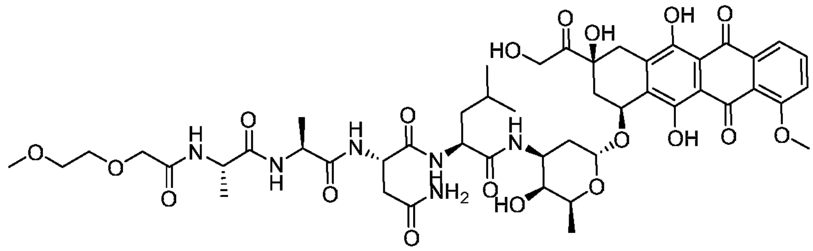

**Embodiment** 10: Preparation of Human Albumin Conjugated HSA-EMC-AANL-DOX, HSA-QHL-087- DOX and HSA-QHL-087-N-CBP Drugs

EMC-AANL-DOX, QHL-087-DOX and QHL-087-N-CBP are prepared, wherein EMC-AANL-DOX was dissolved by DMSO, and QHL-087-DOX and QHL-087-N-CBP were dissolved by sterile water. HSA was dissolved in sterile water. The compound was combined with HSA at a ratio of 3:1 (4.8 umol/mL, 1.6 umol/mL), and reacted in a water bath at 37°C for 3 h. The reaction solution was taken out, and the unbound compound was filtered by pressurized ultrafiltration membrane, diluted with normal saline and filtered for 3 times to obtain the semi-finished product. The human albumin conjugated doxorubicin antitumor drug is isolated by, for example, chromatographic methods such as DEAE ion exchange, gel filtration, and hydroxyapatite chromatography. The semi-finished products were packed, frozen and freeze-dried in time. The freeze-drying technology of the products could be determined according to the performance of the machine, but the preparation quality and storage quality of the products should be guaranteed to meet the requirements. The binding of Legubicin with HSA in different proportions and at different times was compared. The results showed that the mass ratio of EMC AANL DOX, QHL 087 DOX and QHL 087 N CBP with HSA was 3:1 and 37 °C for 3h, the binding rates of HSA were 62%, 99.6% and 99.7% respectively.

**Embodiment 11: Selection of Chemically Modify Linker for Optimal Activation Efficiency**

S-C-A is a chemically modified linker and shows a high activation efficiency compared to the native peptide sequence linker cleaved by Legumain. When C is AAN, the activation of the different S-C-A linkers and the control linker is evaluated in an activation assay. They were dissolved and diluted tenfold using S-C-A conjugates to a concentration of 0.1 mM/ml. The sample compounds were added at a concentration of 1 mg/ml to 100 μg of acidified human breast cancer (MDA - MB435) tumor tissue homogenate (pH 6.0) at 37°C. The enzyme in the tumor tissue homogenate was released and detected by HPLC to compare the efficiency of activation of the linker by the tumor tissue. The results was shown in Table 2-1, 2-2, 2-3 and 2-4.

Table 2-1

| Linker No. | S | A | Activation | | |
|---|---|---|---|---|---|
| | S1 | S2 | S3 | | efficiency (%) |
| EMC-AANL-DOX | $-(CH_2)_6$- CONH- | / | / | Leu | 56.4 |
| AANL-DOX | / | / | / | Leu | 42.1 |
| QHL-087 | - $CH_2CH_2$- CONH- | 2peg | / | Leu | 96.4 |
| QHL-090 | - $CH_2CH_2$- CONH- | 3peg | / | Leu | 93.4 |
| QHL-093 | - $CH_2CH_2$- CONH- | 4peg | / | Leu | 90.1 |
| QHL-096 | - $CH_2CH_2$- CONH- | 6peg | / | Leu | 82.6 |

Table 2-2

| Linker No. | S | | | A | Activatio n efficienc y |
|---|---|---|---|---|---|
| | S1 | S2 | S3 | | |
| EMC-AAN-PABC-DOX | $-(CH_2)_6$-CONH- | / | / | PABC-OH | 62.4 |

(continued)

| Linker No. | S | | | A | Activatio n efficienc y |
|---|---|---|---|---|---|
| | S1 | S2 | S3 | | |
| QHL-085 | $-CH_2CH_2$-CONH- | 2pe g | / | $H_2$PABC-$NH_2H_2$ | 93.5 |
| QHL-088 | $-CH_2CH_2$-CONH- | 3pe g | / | $H_2$PABC-$NH_2H_2$ | 99.6 |
| QHL-091 | $-CH_2CH_2$-CONH- | 4pe g | / | $H_2$PABC-$NH_2H_2$ | 94.1 |
| QHL-086 | $-CH_2CH_2$-CONH- | 2pe g | / | PABC-OH | 94.5 |
| QHL-089 | $-CH_2CH_2$-CONH- | 3pe g | / | PABC-OH | 98.6 |
| QHL-092 | $-CH_2CH_2$-CONH- | 4pe g | / | PABC-OH | 92.1 |
| QHL-087 | $-CH_2CH_2$-CONH- | 2pe g | / | Leu | 96.4 |
| QHL-090 | $-CH_2CH_2$-CONH- | 3pe g | / | Leu | 93.4 |
| QHL-093 | $-CH_2CH_2$-CONH- | 4pe g | / | Leu | 90.1 |

Table 2-3

| Compound No. | S | | | A | Activation efficiency (%) |
|---|---|---|---|---|---|
| | S1 | S2 | S3 | | |
| C4 | / | Peg | / | Leu | 66.9 |
| QHL-085 | $-CH_2CH_2$- CONH- | 2peg | / | $H_2$PABC- $NH_2H_2$ | 93.5 |
| QHL-088 | $-CH_2CH_2$- CONH- | 3peg | / | $H_2$PABC- $NH_2H_2$ | 99.6 |
| QHL-086 | $-CH_2CH_2$- CONH- | 2peg | / | PABC-OH | 94.5 |
| QHL-089 | $-CH_2CH_2$- CONH- | 3peg | / | PABC-OH | 98.6 |
| QHL-087 | $-CH_2CH_2$- CONH- | 2peg | / | Leu | 82.4 |
| QHL-090 | $-CH_2CH_2$- CONH- | 3peg | / | Leu | 93.4 |
| QHL-037 | $-CH_2CH_2$- CONH- | 2peg | Glu | $H_2$PABC- $NH_2H_2$ | 76.1 |
| QHL-043 | $-CH_2CH_2$- CONH- | 3peg | Glu | $H_2$PABC- $NH_2H_2$ | 88.4 |
| QHL-038 | $-CH_2CH_2$- CONH- | 2peg | Glu | PABC-OH | 91.5 |
| QHL-044 | $-CH_2CH_2$- CONH- | 3peg | Glu | PABC-OH | 92.4 |
| QHL-039 | $-CH_2CH_2$- CONH- | 2peg | Glu | Leu | 85.4 |
| QHL-045 | $-CH_2CH_2$- CONH- | 3peg | Glu | Leu | 84.6 |

The effect of the D-type tripeptide on the activation efficiency was examined in comparison to the native peptide sequence linker cleaved by Legumain, S1 in MI-S is $-CH_2CH_2$-CONH-, S2: 2peg. The results were shown in Table 2-4 below.

Table 2-4

| C | A | Activation efficiency (%) |
|---|---|---|
| Ala-Ala-Asn | Leu | 96.8 |
| Thr-Ala-Asn | Leu | 90.2 |
| Val-Ala-Asn | Leu | 78.9 |
| D-Thr-L-Val-L-Asn | Leu | 73.5 |
| D-Thr-L-Ala-L-Asn | Leu | 89.6 |

(continued)

| C | A | Activation efficiency (%) |
|---|---|---|
| D-Ala-L-Val-L-Asn | Leu | 93.5 |
| L-Thr-D-Val-L-Asn | Leu | 90.6 |
| L-Thr-D-Ala-L-Asn | Leu | 72.4 |
| L-Ala-D-Val-L-Asn | Leu | 83.4 |
| D-Thr-D-Val-L-Asn | Leu | 66.1 |
| D-Thr-D-Ala-L-Asn | Leu | 78.4 |
| D-Ala-D-Val-L-Asn | Leu | 61.5 |
| L-Ala-L-Val-D-Asn | Leu | 22.4 |

From the data in the tables, it can be seen that under the condition of high activation of S and A, different amino acid selection and configuration have influence on the activation efficiency when the variable is different tripeptides, especially D-Asn leads to the loss of activation ability, while the other two positions of amino acid are adjusted to D-type and still have activation activity.

**Embodiment 12: Comparison of Enzymatic Digestion Kinetic Rates of Preferred Compounds**

Accurately weigh 10 mg of C3, QHL-087-DOX, QHL-090-DOX, QHL-093-DOX, QHL-094-DOX, QHL-093-DOX and QHL-096-DOX samples respectively, add appropriate amount of water to prepare 4umol/mL sample stock solution, and add water to gradually dilute into the sample solutions with various concentrations in Table 3 below; 20ul of sample solutions with different concentrations was respectively measured, 80ul of Legumain was added, and water bath was carried out in a water bath kettle at 37°C; Take out after water bath for 2h, inject 10ul of sample, and detect by HPLC; Read out the area of each corresponding product, calculate the product concentration according to the linear equation of the product, and substitute it into the formula to obtain the corresponding V:

$$V(umoL/mL/min)=C(umoL/mL)/120min$$

Plotting [C] according to the formula V, the intercept Km/Vmax and the intersection point of the straight line with the x-axis can be obtained, which is -Km, and [C] is the concentration of each substrate, i.e. the concentration of the sample solution, in umoL/mL.

Table 3

| $[C_1]$ | $[C_2]$ | $[C_3]$ | $[C_4]$ | $[C_5]$ | $[C_6]$ | $[C_7]$ |
|---|---|---|---|---|---|---|
| 2 | 1.6 | 0.8 | 0.7 | 0.35 | 0.175 | 0.0875 |

As shown in Figure 3, the 2PEG group of QHL-087 significantly increased the activation efficiency under the condition of other structures being consistent, but the efficiency decreased with the increase of PEG amount. Compared with the same conditions of 6PEG group connection, the $H_2PABC-NH_2H_2$ substitution of leu significantly improves the activation efficiency.

**Embodiment 13: Isolation and Culture of Mouse Spleen and CD8+ T Cells, Isolation of Mouse Bone Marrow Mononuclear Cells and Differentiation of M2 Macrophages**

**1. Isolation of mouse spleen cell**

1) Take the spleen of C57BL/6 mouse, place it on the culture dish with 40uM screen (ice bath), add about 10mL of normal saline, and gently grind with sterile syringe core.

2) Transfer the ground cell suspension into a 50mL centrifuge tube, add about 5mL of normal saline to rinse the petri dish, and also transfer it into the 50mL centrifuge tube, and combine the suspensions.

3) Centrifuge the cell suspension at 1000 r/min for 10 min, discard the supernatant, and add an appropriate volume of normal saline to resuspend. Then add 3 times volume of ammonium chloride erythrocyte lysate, pipetting evenly. After lysing on ice for about 10 min, add 10 mL of normal saline to stop the lysing, and centrifuge at 1000 r/min for 5 min.

4) After centrifugation, discard the supernatant, add 10 mL of physiological saline, pipette evenly, centrifuge at 1000 r/min for 5 min, and repeat the above operation once. Cells were then resuspended in 10% RMPI 1640 medium for subsequent culture or in 0.5% BSA for subsequent T cell sorting.

**2. CD8+ T cell sorting**

The mouse splenocytes isolated above were resuspended to 1E8/mL. Add 100ul Miltenyi biotec CD8a(Ly-2) microBeads per 1E8 cells, mix well, and incubate at 4°C for 15 minutes in the dark. Add 5-10 times the volume of PBS, mix and wash thoroughly, centrifuge at 300g for 5 minutes, remove the supernatant, and repeat the washing once. Resuspend the cells to 2E8/mL for separation on the column, place the cell suspension on the magnet plate on the LS column, which has been pre-equilibrated with wash buffer (pH7.2 PBS + 0.5% BSA + 2mM EDTA). After the cell suspension slowly flowed through the LS column and the CD8+ T cells were bound to the magnetic particles in the LS column, the LS column was washed with 3 times the volume of cell suspension wash buffer. After washing, the LS column was taken out from the magnet plate and placed in a 15mL centrifuge tube. Add 5 mL of washing buffer to the LS column, and then use the LS cartridge to quickly squeeze and elute the bound cells in the LS into a centrifuge tube, collect all the cells that pass through the column, centrifuge the obtained cells to remove the supernatant, and repeat the washing with the washing buffer once. Resuspend the cells with an appropriate volume of 10% RMPI1640 medium, and count the cells for use.

**3. Activation and expansion of CD8+ T positive cells**

A. Washing of CD3/CD28 magnetic beads: a. Shake and suspend the immunomagnetic beads in the small tube (vortex for more than 30s, or tilt and rotate for 5min). b. Take the required amount of immunomagnetic beads into a 1.5ml test tube, add 1ml of 1640 containing serum and suspend well, vortex for more than 30s, or keep rolling for at least 5min.

B. Activation of T cells: a. Inoculate the appropriate number of cells in the culture plate (such as 6-well plate, 1E6/ml, 2ml culture medium, keep the T cell density above 2E6/ml, but not more than 2E6/ml). b. Add the washed magnetic beads to make the ratio of magnetic beads and cells to be 1:1. c. Put it into the incubator for 3 days.

C. Amplification: a. The culture medium does not need to be replaced within 3 days. On the third day, 30U/ml IL-2 was added (it can be appropriately increased according to the actual situation), and the medium was changed at the same time (the number of cells would approximately double after 48h, and the size and shape of the cells should be monitored in real time). b. After 4-5 days of stimulation, remove the magnetic beads (avoid excessive activation), and move the cells up and down for 5-10 times (avoid air bubbles and turbulence during movement) to separate the cells from the magnetic beads. The cell was collected into a 1.5 ml tube and place on a magnet for 1 min until that beads were on the wall of the tube. The cells were transferred to another tube to remove the beads as completely as possible and cultured in medium supplemented with 30U/ml IL-2 (which could be increased according to the actual situation) and their status, proliferation and viability were monitored.

**4. Isolation of mouse bone marrow mononuclear cells and induction of M2 macrophage differentiation**

The bilateral femur and tibiae of two C57BL/6 mice were taken out under aseptic conditions, and that metaphysis was cut off in a super-clean table. the marrow cavity was was gently washed with serum-free MEM culture solution by a 5 mL sterile syringe for 4 times, and all cell suspensions were collect; Centrifuge at 1000 r/min for 10 min, discard the supernatant to obtain the cell precipitate, resuspend with an appropriate volume of serum-free MEM culture medium, repeatedly blow and homogenize, filter with a 40uM filter screen, add 3 times the volume of erythrocyte lysate, and lyse on ice for 10 min; Centrifuge at 1000 r/min for 5 min, discard the supernatant to obtain the cell precipitate, wash with serum-free MEM culture medium for 2 times, and collect the cell precipitate; Resuspend the cells with MEM complete

culture medium containing 10% FBS and 1%PS in volume fraction, and count the cells for subsequent differentiation; 100uL, 20000 cells/well were inoculated into 96-well cell culture plate, 100ng M-CSF was added into the culture medium for differentiation of M2 macrophages, and the cells were statically cultured at 37 °C in 5% CO2 incubator, and induced to differentiate for 7 days, and the cell morphology was observed. The morphology of induced cells was shown in Figure 4. M2 macrophages were different from monocytes, DC and GM-macrophages, and were confirmed to be M2 macrophages.

**Embodiment 14: Determination of the Inhibitory Effect of Drugs on Cell Growth by MTT Assay**

After the cell count in Example 13, the cell concentration was adjusted with the culture medium, and the cells were seeded in a 96-well culture plate at 100μl of cell suspension per well, wherein the seeding concentration of CD8+ T cells was 100000 cells/well, and the seeding concentration of M2 macrophages was 20000 cells/well. The 96-well plate was incubated overnight for 24 hour at 37°C in a carbon dioxide (5%) incubator. After 24 hours, 100ul of cell culture solutions containing different concentrations of drugs were added to the 96-well culture plate. At the same time, a control well (0.1% DMSO) with no drug added and only the corresponding drug solvent and a zeroadjusted well (Blank) with only medium and no cells added was set. Each set was prepared in triplicate and the plates were incubated for 48 hours at 37 °C in a 5% $CO_2$ incubator. After 48 hours, 20 μl MTT (5 mg/ml) was added to each well and the incubation was continued for 4 hours. The culture medium was then gently aspirated, and 150 μl DMSO was added to each well as solvent for dissolution. After dissolution, the absorbance at 490 nm was measured with a microplate reader.

The cell survival rate and the 50% inhibitory concentration of the drug on the cells were calculated. Cell survival rate (%) = (ODtest-ODblank)/(ODtest control-ODblank)*100%. The cell survival rate (%) was calculated by Excel software, and the dose-response curve of drug to cells was drawn by Prism 5, in which each index was expressed by mean value, and the coefficient of variation (CV) was used to evaluate the consistency of data.

According to the schematic diagram of the experiment and the setting of the dosing concentration of the cells in the above experimental method, the maximum initial concentration of the drug to be tested was set to 14uM, and the gradient was diluted in a ratio of 1:3 into 9 dose groups (3 replicates in each group). The concentration of drug solvent (DMSO) in all the wells dosed was controlled at 0.1%. The Control group was dosed only with drug solvent (0.1%DMSO), and the Blank group was dosed only with culture medium without cells. Then the survival rate (%) of tumor cells in each dose group relative to the Control group was calculated according to the following method.

Cell survival rate of each dose group (%) = (OD dose group-OD blank group)/(OD 0.1%DMSO - OD blank group)*100%

The experimental results are shown in Figures 5 and 6. Under otherwise structurally consistent conditions, QHL-087-DOX has significantly improved cytotoxicity against M2 macrophages and less cytotoxicity against CD8+ T cells relative to C3 (ie, EMC-AANL-DOX), resulting in selectivity for immunosuppressive cells.

**Embodiment 15: Screening of Various Agents of the Disclosure for Cytotoxicity on M2 Macrophages**

Cytotoxicity screening experiments for some compounds were performed for M2 macrophage inhibition as in embodiment 14. Each drug was tested in 3 wells, and 10 uM of the following drugs were added to each well to test the inhibition rate relative to the drug-free group. The experimental results are shown in Table 4.

**Embodiment 16: Comparison of Water-Solubility of the Water-Soluble Highly Efficient Targeted Activated Adriamycin Derivatives, etc. Prepared in the Embodiments of the Disclosure with the Reference Compound**

The compounds prepared in accordance with the examples of the disclosure and the reference compounds C1, C2, C3 and C4 were lyophilized (- 70°C). The compounds were dissolved in different concentrations of water and the water solubility was checked by observation and HPLC testing (> 95%). The result was shown in Table 4.

Table 4: Water solubility test data of screened drug and inhibition rate of M2 macrophage

| Compound No. | Linker | Solubility | Inhibition rate of M2 macrophages |
|---|---|---|---|
| DOX | / | <1mg/ml | 12.6% |
| AANL-DOX | / | <1mg/ml | 16.9% |

(continued)

| Compound No. | Linker | Solubility | Inhibition rate of M2 macrophages |
|---|---|---|---|
| EMC-AANL-DOX | / | <5mg/ml | 34.6% |
| PEG-AANL-DOX | / | <5mg/ml | 10.5% |
| QHL-001-DOX | QHL-001 | >10mg/ml | 84.5% |
| QHL-002-DOX | QHL-002 | >10mg/ml | |
| QHL-003-DOX | QHL-003 | >15mg/ml | 75.8% |
| QHL-004-DOX | QHL-004 | >20mg/ml | 63.7% |
| QHL-005-DOX | QHL-005 | >10mg/ml | |
| QHL-006-DOX | QHL-006 | >10mg/ml | |
| QHL-007-DOX | QHL-007 | >15mg/ml | |
| QHL-008-DOX | QHL-008 | >20mg/ml | |
| QHL-009-DOX | QHL-009 | >10mg/ml | |
| QHL-010-DOX | QHL-010 | >10mg/ml | |
| QHL-011-DOX | QHL-011 | >15mg/ml | |
| QHL-012-DOX | QHL-012 | >20mg/ml | |
| QHL-013-DOX | QHL-013 | >20mg/ml | 78.4% |
| QHL-014-DOX | QHL-014 | >20mg/ml | |
| QHL-015-DOX | QHL-015 | >25mg/ml | |
| QHL-016-DOX | QHL-016 | >30mg/ml | |
| QHL-017-DOX | QHL-017 | >20mg/ml | |
| QHL-018-DOX | QHL-018 | >20mg/ml | |
| QHL-019-DOX | QHL-019 | >25mg/ml | |
| QHL-020-DOX | QHL-020 | >30mg/ml | |
| QHL-021-DOX | QHL-021 | >20mg/ml | 68.6% |
| QHL-022-DOX | QHL-022 | >20mg/ml | |
| QHL-023-DOX | QHL-023 | >25mg/ml | |
| QHL-024-DOX | QHL-024 | >30mg/ml | |
| QHL-025-DOX | QHL-025 | >20mg/ml | |
| QHL-026-DOX | QHL-026 | >20mg/ml | |
| QHL-027-DOX | QHL-027 | >25mg/ml | |
| QHL-028-DOX | QHL-028 | >30mg/ml | |
| QHL-029-DOX | QHL-029 | >20mg/ml | 46.4% |
| QHL-030-DOX | QHL-030 | >20mg/ml | |
| QHL-031-DOX | QHL-031 | >25mg/ml | |
| QHL-032-DOX | QHL-032 | >30mg/ml | |
| QHL-033-DOX | QHL-033 | >20mg/ml | |
| QHL-034-DOX | QHL-034 | >20mg/ml | |
| QHL-035-DOX | QHL-035 | >25mg/ml | |
| QHL-036-DOX | QHL-036 | >30mg/ml | |

(continued)

| Compound No. | Linker | Solubility | Inhibition rate of M2 macrophages |
|---|---|---|---|
| QHL-037-DOX | QHL-037 | >20mg/ml | 87.8% |
| QHL-038-DOX | QHL-038 | >20mg/ml | 76.4% |
| QHL-039-DOX | QHL-039 | >20mg/ml | 78.9% |
| QHL-040-DOX | QHL-040 | >20mg/ml | 46.7% |
| QHL-041-DOX | QHL-041 | >20mg/ml | |
| QHL-042-DOX | QHL-042 | >20mg/ml | |
| QHL-043-DOX | QHL-043 | >20mg/ml | |
| QHL-044-DOX | QHL-044 | >20mg/ml | |
| QHL-045-DOX | QHL-045 | >20mg/ml | |
| QHL-046-DOX | QHL-046 | >20mg/ml | |
| QHL-047-DOX | QHL-047 | >20mg/ml | |
| QHL-048-DOX | QHL-048 | >20mg/ml | |
| QHL-049-DOX | QHL-049 | >25mg/ml | |
| QHL-050-DOX | QHL-050 | >25mg/ml | |
| QHL-051-DOX | QHL-051 | >25mg/ml | |
| QHL-052-DOX | QHL-052 | >25mg/ml | |
| QHL-053-DOX | QHL-053 | >25mg/ml | |
| QHL-054-DOX | QHL-054 | >25mg/ml | |
| QHL-055-DOX | QHL-055 | >25mg/ml | |
| QHL-056-DOX | QHL-056 | >25mg/ml | |
| QHL-057-DOX | QHL-057 | >25mg/ml | |
| QHL-058-DOX | QHL-058 | >25mg/ml | |
| QHL-059-DOX | QHL-059 | >25mg/ml | |
| QHL-060-DOX | QHL-060 | >25mg/ml | |
| QHL-061-DOX | QHL-061 | >15mg/ml | |
| QHL-062-DOX | QHL-062 | >15mg/ml | |
| QHL-063-DOX | QHL-063 | >15mg/ml | |
| QHL-064-DOX | QHL-064 | >15mg/ml | |
| QHL-065-DOX | QHL-065 | >15mg/ml | |
| QHL-066-DOX | QHL-066 | >15mg/ml | |
| QHL-067-DOX | QHL-067 | >15mg/ml | |
| QHL-068-DOX | QHL-068 | >15mg/ml | |
| QHL-069-DOX | QHL-069 | >25mg/ml | |
| QHL-070-DOX | QHL-070 | >25mg/ml | |
| QHL-071-DOX | QHL-071 | >25mg/ml | |
| QHL-072-DOX | QHL-072 | >25mg/ml | |
| QHL-073-DOX | QHL-073 | >25mg/ml | |
| QHL-074-DOX | QHL-074 | >25mg/ml | |

(continued)

| Compound No. | Linker | Solubility | Inhibition rate of M2 macrophages |
|---|---|---|---|
| QHL-075-DOX | QHL-075 | >25mg/ml | |
| QHL-076-DOX | QHL-076 | >25mg/ml | |
| QHL-077-DOX | QHL-077 | >25mg/ml | |
| QHL-078-DOX | QHL-078 | >25mg/ml | |
| QHL-079-DOX | QHL-079 | >25mg/ml | 87.5% |
| QHL-080-DOX | QHL-080 | >25mg/ml | 87.7% |
| QHL-081-DOX | QHL-081 | >30mg/ml | 78.5% |
| QHL-082-DOX | QHL-082 | >30mg/ml | |
| QHL-083-DOX | QHL-083 | >30mg/ml | |
| QHL-084-DOX | QHL-084 | >30mg/ml | |
| QHL-085-DOX | QHL-085 | >10mg/ml | 92.2% |
| QHL-086-DOX | QHL-086 | >15mg/ml | 92.9% |
| QHL-087-DOX | QHL-087 | >10mg/ml | 99.8% |
| QHL-088-DOX | QHL-088 | >10mg/ml | 98.4% |
| QHL-089-DOX | QHL-089 | >15mg/ml | 82.2% |
| QHL-090-DOX | QHL-090 | >10mg/ml | 72.9% |
| QHL-091-DOX | QHL-091 | >20mg/ml | 63.8% |
| QHL-092-DOX | QHL-092 | >25mg/ml | 94.4% |
| QHL-093-DOX | QHL-093 | >20mg/ml | 77.4% |
| QHL-094-DOX | QHL-094 | >20mg/ml | 72.6% |
| QHL-095-DOX | QHL-095 | >20mg/ml | 74.7% |
| QHL-096-DOX | QHL-096 | >20mg/ml | 70.4% |
| QHL-097-DOX | QHL-097 | >20mg/ml | |
| QHL-098-DOX | QHL-098 | >10mg/ml | |
| QHL-099-DOX | QHL-099 | >20mg/ml | |
| QHL-100-DOX | QHL-100 | >25mg/ml | |
| QHL-101-DOX | QHL-101 | >20mg/ml | |
| QHL-102-DOX | QHL-102 | >10mg/ml | |
| QHL-103-DOX | QHL-103 | >15mg/ml | |
| QHL-104-DOX | QHL-104 | >20mg/ml | |
| QHL-105-DOX | QHL-105 | >10mg/ml | |
| QHL-106-DOX | QHL-106 | >20mg/ml | |
| QHL-107-DOX | QHL-107 | >25mg/ml | |
| QHL-108-DOX | QHL-108 | >20mg/ml | |
| QHL-109-DOX | QHL-109 | >10mg/ml | 54.8% |
| QHL-110-DOX | QHL-110 | >15mg/ml | 46.8% |
| QHL-111-DOX | QHL-111 | >10mg/ml | |
| QHL-112-DOX | QHL-112 | >10mg/ml | |

(continued)

| Compound No. | Linker | Solubility | Inhibition rate of M2 macrophages |
|---|---|---|---|
| QHL-113-DOX | QHL-113 | >15mg/ml | |
| QHL-114-DOX | QHL-114 | >15mg/ml | |
| QHL-115-DOX | QHL-115 | >10mg/ml | |
| QHL-116-DOX | QHL-116 | >15mg/ml | |
| QHL-117-DOX | QHL-117 | >10mg/ml | |
| QHL-118-DOX | QHL-118 | >15mg/ml | |
| QHL-119-DOX | QHL-119 | >15mg/ml | |
| QHL-120-DOX | QHL-120 | >10mg/ml | |
| QHL-121-DOX | QHL-121 | >10mg/ml | |
| QHL-122-DOX | QHL-122 | >10mg/ml | |
| QHL-123-DOX | QHL-123 | >2mg/ml | |
| QHL-124-DOX | QHL-124 | >5mg/ml | |
| QHL-125-DOX | QHL-125 | >2mg/ml | |
| QHL-126-DOX | QHL-126 | >2mg/ml | |
| QHL-127-DOX | QHL-127 | >10mg/ml | |
| QHL-128-DOX | QHL-128 | >2mg/ml | |
| QHL-129-DOX | QHL-129 | >5mg/ml | |
| QHL-130-DOX | QHL-130 | >2mg/ml | |
| QHL-131-DOX | QHL-131 | >10mg/ml | |
| QHL-132-DOX | QHL-132 | >2mg/ml | |
| QHL-133-DOX | QHL-133 | >2mg/ml | |
| QHL-134-DOX | QHL-134 | >5mg/ml | |
| QHL-135-DOX | QHL-135 | >5mg/ml | |
| QHL-136-DOX | QHL-136 | >2mg/ml | |
| QHL-137-DOX | QHL-137 | >5mg/ml | |
| QHL-140-N-CBP | QHL-140 | >10mg/ml | 89.5% |
| QHL-143-N-CBP | QHL-143 | >10mg/ml | 78.4% |
| QHL-095-N-CBP | QHL-095 | >10mg/ml | 68.7% |
| QHL-092-N-CBP | QHL-092 | >10mg/ml | 75.8% |
| QHL-089-N-CBP | QHL-089 | >10mg/ml | 97.4% |
| QHL-107-N-CBP | QHL-107 | >10mg/ml | |
| QHL-104-N-CBP | QHL-104 | >20mg/ml | |
| QHL-101-N-CBP | QHL-101 | >20mg/ml | |
| QHL-098-N-CBP | QHL-098 | >20mg/ml | |
| QHL-146-N-CBP | QHL-146 | >20mg/ml | |
| QHL-086-N-CBP | QHL-086 | >20mg/ml | |
| QHL-086 - Dovitinib | QHL-086 | >20mg/ml | |
| QHL-089 - Dovitinib | QHL-089 | >20mg/ml | |

(continued)

| Compound No. | Linker | Solubility | Inhibition rate of M2 macrophages |
|---|---|---|---|
| QHL-086-Epirubicin | QHL-086 | >20mg/ml | |
| QHL-089-Epirubicin | QHL-089 | >20mg/ml | |
| QHL-086-Compound a | QHL-086 | >20mg/ml | |
| QHL-089-Compound a | QHL-089 | >20mg/ml | |
| QHL-086-Compound b | QHL-086 | >20mg/ml | |
| QHL-089-Compound b | QHL-089 | >20mg/ml | |
| QHL-086-Mitomycin | QHL-086 | >20mg/ml | |
| QHL-089-Mitomycin | QHL-089 | >20mg/ml | |
| QHL-086-Dabrafenib | QHL-086 | >20mg/ml | |
| QHL-089-Dabrafenib | QHL-089 | >20mg/ml | |
| QHL-086-Motesani | QHL-086 | >20mg/ml | |
| QHL-089-Motesani | QHL-089 | >20mg/ml | |
| QHL-138-N-CBP | QHL-138 | >20mg/ml | |
| QHL-141-N-CBP | QHL-141 | >20mg/ml | |
| QHL-096-N-CBP | QHL-096 | >20mg/ml | |
| QHL-093-N-CBP | QHL-093 | >10mg/ml | |
| QHL-090-N-CBP | QHL-090 | >10mg/ml | |
| QHL-108-N-CBP | QHL-108 | >10mg/ml | |
| QHL-105-N-CBP | QHL-105 | >10mg/ml | |
| QHL-102-N-CBP | QHL-102 | >10mg/ml | |
| QHL-099-N-CBP | QHL-099 | >10mg/ml | |
| QHL-144-N-CBP | QHL-144 | >10mg/ml | |
| QHL-087-N-CBP | QHL-087 | >10mg/ml | |
| QHL-087 - Dovitinib | QHL-087 | >10mg/ml | |
| QHL-090 - Dovitinib | QHL-090 | >10mg/ml | |
| QHL-087-Epirubicin | QHL-087 | >10mg/ml | |
| QHL-090-Epirubicin | QHL-090 | >10mg/ml | |
| QHL-087-Compound a | QHL-087 | >10mg/ml | |
| QHL-090-Compound a | QHL-090 | > 1 0mg/ml | |
| QHL-087-Compound b | QHL-087 | >10mg/ml | |
| QHL-090-Compound b | QHL-090 | >10mg/ml | |
| QHL-087-mitomycin | QHL-087 | >10mg/ml | |
| QHL-090-mitomycin | QHL-090 | >10mg/ml | |
| QHL-087-Dabrafenib | QHL-087 | >10mg/ml | |
| QHL-090-Dabrafenib | QHL-090 | >10mg/ml | |
| QHL-087-Motseni | QHL-087 | >10mg/ml | |
| QHL-090-Motseni | QHL-090 | >10mg/ml | |
| QHL-140- Resiquimod | QHL-140 | >10mg/ml | |

(continued)

| Compound No. | Linker | Solubility | Inhibition rate of M2 macrophages |
|---|---|---|---|
| QHL-086- Resiquimod | QHL-086 | >10mg/ml | |
| QHL-089- Resiquimod | QHL-089 | >10mg/ml | |
| QHL-092- Resiquimod | QHL-092 | >10mg/ml | |
| QHL-095- Resiquimod | QHL-095 | >10mg/ml | |
| QHL-005- Resiquimod | QHL-005 | >10mg/ml | |
| QHL-006- Resiquimod | QHL-006 | >10mg/ml | |
| QHL-008- Resiquimod | QHL-008 | >10mg/ml | |
| QHL-147- Resiquimod | QHL-147 | >10mg/ml | |
| QHL-116- Resiquimod | QHL-116 | >10mg/ml | |
| QHL-119- Resiquimod | QHL-119 | >10mg/ml | |
| QHL-140-Prednisone | QHL-140 | >10mg/ml | |
| QHL-086-Prednisone | QHL-086 | > 1 0mg/ml | |
| QHL-089-Prednisone | QHL-089 | >10mg/ml | |
| QHL-092-Prednisone | QHL-092 | >10mg/ml | |
| QHL-095 -Prednisone | QHL-095 | >10mg/ml | |
| QHL-005 -Prednisone | QHL-005 | >10mg/ml | |
| QHL-006-Prednisone | QHL-006 | >10mg/ml | |
| QHL-008-Prednisone | QHL-008 | >10mg/ml | |
| QHL-147-Prednisone | QHL-147 | >10mg/ml | |
| QHL-116-Prednisone | QHL-116 | >10mg/ml | |
| QHL-119-Prednisone | QHL-119 | >10mg/ml | |
| QHL-150-T3 | QHL-150 | >10mg/ml | |
| QHL-157-T3 | QHL-157 | >10mg/ml | |
| QHL-158-T3 | QHL-158 | >10mg/ml | |
| QHL-159-T3 | QHL-159 | >10mg/ml | |
| QHL-160-T3 | QHL-160 | >10mg/ml | |
| QHL-153-T3 | QHL-153 | >10mg/ml | |
| QHL-154-T3 | QHL-154 | >10mg/ml | |
| QHL-155-T3 | QHL-155 | >10mg/ml | |
| QHL-156-T3 | QHL-156 | >10mg/ml | |
| QHL-161-T3 | QHL-161 | >10mg/ml | |
| QHL-162-T3 | QHL-162 | >10mg/ml | |

The results showed that the water solubility of 2peg group was improved obviously, and the solubility increased with the increase of PEG amount. Under that same conditions for PEG attachment, increase Glu and Asp can increase water solubility. The water solubility of the coupling drug was changed through the change of the group, and the water solubility of the coupling drug was greatly influenced on the vascular membrane of the drug and the permeability of the tumor cell membrane, thereby influencing the drug effect of treatment. The enhancement of the water solubility of the compound provides a necessary condition for the preparation of medicaments and the production of coupled medicaments.

**Embodiment 17: Pharmacodynamic Study of C3, QHL-085-DOX, QHL-087-DOX, QHL-091-DOX, QHL-094-DOX Injections in Nude Mice HT1080 Model**

Objective: To investigate the antitumor effects of C3, QHL-085-DOX, QHL-087-DOX, QHL-091-DOX and QHL-94-DOX in mouse models during tumor therapy.

Test drugs: C3, QHL-085-DOX, QHL-087-DOX, QHL-091-DOX and QHL-094-DOX were used as injections and diluted to the corresponding concentrations with normal saline during the test.

Method and results:

1. Animals: nude mice aged 6-8 weeks, all female.

2. Preparation of tumor models

1) HT1080 cells were purchased from ATCC and characterized according to the instructions provided. Cells were cultured in DMEM medium containing 10% fetal bovine serum at 37°C in 5% $CO_2$. Passages were performed every three days and cells up to passage 15 were used.

2) Tumor generation: $5\times10^6$ HT1080 cells were injected subcutaneously into the back of nude mice. Randomization was performed when the tumor size reached 100 $mm^3$. Treatment was then initiated, and the day of initiation of treatment was counted as Day 1.

3) Treatment process
According to the clinical application of C3, QHL-085-DOX, QHL-087-DOX, QHL-091-DOX and QHL-094-DOX, the drug was administered intravenously (IV). C3, QHL-085-DOX, QHL-087-DOX, QHL-091-DOX, and QHL-094-DOX were administered at low and the same dose of 18 umol/kg, respectively. The control group was given normal saline once a week for 3 weeks.

4) Results and discussion: The grouping and test results are shown in Figure 7. Compared with the equimolar dose low-dose treatment group, the tumor-inhibitory effects of the 4peg and 2peg groups were sequentially enhanced.

**Embodiment 18: Pharmacodynamic Study of C1, C2, C3, QHL-086-DOX, QHL-092-DOX, QHL-095-DOX, QHL-087-DOX, QHL-010-DOX, QHL-117-DOX Injections in Nude Mice HT1080 Model**

Objective: To investigate the anti-tumor efficacy of the above compounds in mouse models during tumor therapy.

Test drug: C1, C2, C3, and corresponding compound were used as injections and diluted to the corresponding concentrations with normal saline during the test.

Method and results:

1. Animals: nude mice aged 6-8 weeks, all female.

2. Preparation of tumor models

1) HT1080 cells were purchased from ATCC and characterized according to the instructions provided. Cells were cultured in DMEM medium containing 10% fetal bovine serum at 37°C in 5% $CO_2$. Passages were performed every three days and cells up to passage 15 were used.

2) Tumor generation: $5\times10^6$ HT1080 cells were injected subcutaneously into the back of nude mice. Randomization was performed when the tumor size reached 100 $mm^3$. Treatment was then initiated, and the day of initiation of treatment was counted as Day 1.

3) Treatment process

According to the clinical application of the corresponding compound, the drug was administered intravenously (IV). The compounds indicated in the table were administered at a low dose and at the same dose of 36 umol/kg. The control group was given normal saline once a week for 3 weeks.

5) The grouping and test results are shown in Table 5.

Table 5: C1, C2, C3 and part of that compound of the present disclosure and mitomycin for tumor treatment in nude mice

| Group | Number of animals | Tumor volume ($mm^3$) | Tumor inhibition rate |
|---|---|---|---|
| | | Day 28 | Day 28 |
| Normal saline | 6 | 1746.6± 673.4 | 0 |
| QHL-086-DOX | 6 | 0 | 100% |
| QHL-092-DOX | 6 | 0 | 100% |
| QHL-087-DOX | 6 | 0 | 100% |
| QHL-010-DOX | 6 | 0 | 100% |
| QHL-117-DOX | 6 | 0 | 100% |
| DOX | 6 | 754.4± 587.4 | 56.8% |
| AANL-DOX | 6 | 318.5±197.6 | 81.8% |
| EMC-AANL-DOX | 6 | 138. 3±124.6 | 92.1% |

5) Result and discussion: As shown in Table 5, compared with equimolar dose AANL-DOX treatment group, QHL-086-DOX, QHL-092-DOX, QHL-095-DOX, QHL-087-DOX, QHL-010-DOX, QHL-117-DOX high dose treatment greatly improve that growth inhibition of tumor, and finally the tumor disappeared, thus achieving the effect of tumor cure.

**Embodiment** 19: **Tissue Distribution Study of** QHL-087-DOX **and** EMC-AANL-DOX **in Orthotopic Liver Transplantation CT26 Tumors.**

Objective: To investigate the tissue distribution of activating drugs in liver tumors.
Test animals: BALB/c mice, 6-8 weeks old, all female.
Preparation of tumor models

1) CT26 cells were purchased from ATCC. Cells were cultured in DMEM medium containing 10% fetal bovine serum at 37°C in 5% $CO_2$. Passages were performed every three days and cells up to passage 15 were used.

2) Tumor generation: $5\times10^6$ CT26 cells were injected subcutaneously into the back of nude mice. Randomization was performed when the tumor size reached 800- 1000 $mm^3$. Tumor tissue was then extracted and cut into 100 $mm^3$ tumor tissue pieces and transplanted orthotopically into BALB/c mouse livers.

3) Administration process: After 14 days, when the orthotopically transplanted tumor grew, a group of 36 orthotopically transplanted tumor mice were treated with drugs. Different tissues were then collected at 1, 6, 12, 24, 36, 72 hr to detect the concentration of doxorubicin released in the different tissues. AUClast h*nmol/g was calculated and the mean and SEM are shown in Figures 8 and 9.

4) Results and discussion: As shown in Figures 8 and 9, the active doxorubicin of QHL-087-DOX and EMC-AANL-DOX were mainly distributed in the liver and liver orthotopic tumors. The previously published use of EMC-AANL-DOX was for breast cancer treatment. Further studies found that QHL-087-DOX had the property of delivering more drugs to the liver, and the activation of tumors in situ in the liver led to higher doxorubicin exposure. Compared with EMC-AANL-DOX, QHL-087-DOX showed enhanced doxorubicin delivery and activation in situ liver cancer.

**Embodiment 20: Pharmacodynamic Study of QHL-087-DOX in Orthotopic Liver Transplantation CT26 Tumors.**

Objective: To study the efficacy of QHL-087-DOX, PD-1 and their combination in orthotopic transplantation of CT26 tumor.
Test drug: 18 μmol/kg of QHL-087-DOX, 5 mg/kg of mouse PD-1.
Test animals: BALB/c mice, 6-8 weeks old, all female.

1) Preparation of tumor models: CT26 cells were purchased from ATCC. Cells were cultured in DMEM medium containing 10% fetal bovine serum at 37°C in 5% $CO_2$. Passages were performed every three days and cells up to

passage 15 were used. $5\times10^5$ CT26 cells were injected subcutaneously into the back of nude mice. Randomization was performed when the tumor size reached 800- 1000 $mm^3$. Tumor tissue was then extracted and cut into 100 $mm^3$ tumor tissue pieces and transplanted orthotopically into BALB/c mouse livers. After one week, when the orthotopically transplanted tumors grew, the mice with orthotopically transplanted tumors were randomized.

2) Treatment course: 6 mice in one group were treated with drugs. The day of treatment was Day 1. According to the clinical application of QHL-087-DOX, the drug was administered intravenously (IV) once weekly for 3 weeks. PD-1 antibody were injected intravenously (IV) twice weekly for 3 week. Group assignment and test results are shown in Figure 10.

3) Results and discussion: It was found for the first time that QHL-087-DOX combined with PD-1 had a synergistic immunotherapeutic effect on hepatic tumors in situ. The combination of QHL-087-DOX and PD-1 was more effective than QHL-087-DOX alone and had immunotherapeutic characteristics.

**Embodiment 21: Effect of EMC-AANL-DOX (legubicin), lenvatinib and PD-1 combination therapy for orthotopic live cancer**

Objective: To evaluate the effect of EMC-AANL-DOX (legubicin), lenvatinib and PD-1 combination therapy for orthotopic live cancer

Test drug: 18 μmol//kg of EMC-AANL-DOX, 5 mg/kg of mouse PD-1.

Test animals: BALB/c mice, 6-8 weeks old, all female.

Preparation of tumor models: CT26 cells were purchased from ATCC. Cells were cultured in DMEM medium containing 10% fetal bovine serum at 37°C in 5% $CO_2$. Passages were performed every three days and cells up to passage 15 were used. $5\times10^5$ CT26 cells were injected subcutaneously into the back of nude mice. Randomization was performed when the tumor size reached 800- 1000 $mm^3$. Tumor tissue was then extracted and cut into 100 $mm^3$ tumor tissue pieces and transplanted orthotopically into BALB/c mouse livers. After one week, when the orthotopically transplanted tumors grew, the mice with orthotopically transplanted tumors were randomized.

2) Treatment course: 6 mice in one group were treated with drugs. The day of treatment was Day 1. According to the clinical application of EMC-AANL-DOX, the drug was administered intravenously (IV) once weekly for 3 weeks. PD-1 antibody were injected intravenously (IV) twice weekly for 3 week. The grouping and test results are shown in Figure 11.

Results and Discussion: It was found for the first time that the efficacy of EMC-AANL-DOX combined with PD-1 was superior to that of lenvatinib combined with PD-1, and the efficacy of EMC-AANL-DOX combined with lenvatinib was superior to that of each monotherapy.

**Embodiment 22: Study on the Therapeutic Effect of Injections of Some Compounds of the Disclosure on Human Liver Cancer HepG2 Cells in Nude Mice**

Test objective: To study the antitumor efficacy of some compounds of the present disclosure in mouse tumor treatment models.

Test drug: The corresponding compound injection and control group injection in the table are diluted to the corresponding concentration with normal saline during the test.

Method and results:

1. Animals: nude mice aged 6-8 weeks, all female.

2. Preparation of tumor models

Human hepatoma HepG2 cells were purchased from ATCC and identified according to the instructions provided. Cells were cultured in DMEM medium containing 10% fetal bovine serum at 37°C in 5% $CO_2$. Passages were performed every three days and cells up to passage 15 were used.

2) Tumor generation: $5\times10^6$ HepG2 cells were injected subcutaneously into the back of nude mice. Randomization was performed when the tumor size reached 100 $mm^3$. Treatment was then initiated, and the day of initiation of treatment was counted as Day 1.

3) Treatment process

According to the clinical application of the corresponding compound, the drug was administered intravenously

(IV). The compound and the control drug were administered at a dose of 54 umol/kg, and DOX could only be administered at a dose of 18 umol/kg due to toxicity limitations. The control group was given normal saline once a week for 4 weeks.

4) The grouping and test results are shown in Table 6.

Table 6: Effect of some compounds of the present disclosure and control group on tumor treatment in nude mice

| Group | Number of animals | Tumor volume ($mm^3$) | Tumor inhibition rate (%) |
|---|---|---|---|
| | | Day 28 | Day 28 |
| Normal saline | 6 | 2897.9±1948.6 | 0 |
| QHL-095-DOX | 6 | 208.7±164.7 | 92.8% |
| QHL-008-DOX | 6 | 148.1±84.6 | 94.9% |
| QHL-086-DOX | 6 | 0 | 100% |
| QHL-116-DOX | 6 | 0 | 100% |
| QHL-119-DOX | 6 | 292.68±196.80 | 89.9% |
| QHL-092-DOX | 6 | 69.5±46.7 | 97.6% |
| QHL-006-DOX | 6 | 0 | 100% |
| QHL-089-DOX | 6 | 0 | 100% |
| QHL-005-DOX | 6 | 0 | 100% |
| QHL-007-DOX | 6 | 148.1±84.6 | 94.9% |
| QHL-096-DOX | 6 | 0 | 100% |
| QHL-012-DOX | 6 | 197.4±104.5 | 93.2% |
| QHL-087-DOX | 6 | 0 | 100% |
| QHL-117-DOX | 6 | 0 | 100% |
| QHL-120-DOX | 6 | 208.1±164.8 | 92.8% |
| QHL-093-DOX | 6 | 168.49±98.4 | 94.2% |
| QHL-010-DOX | 6 | 0 | 100% |
| QHL-090-DOX | 6 | 0 | 100% |
| QHL-009-DOX | 6 | 0 | 100% |
| QHL-011-DOX | 6 | 98.1±48.4 | 96.6% |
| C1 DOX | 6 | 1683.4±1087.4 | 41.9% |
| C2 AANL-DOX | 6 | 1564 ± 689.4 | 46.0% |
| C3 EMC-AANL-DOX | 6 | 218. 3±167.7 | 92.5% |
| C4 PEG-AANL-DOX | 6 | 848.7± 347.5 | 70.7% |

5) Results and discussion: As shown in Table 6, EMC-AANL-DOX has a good effect on the treatment of liver cancer. The preferred compounds of the present disclosure have an increased therapeutic effect on tumor growth compared to EMC-AANL-DOX at the same molar dose.

**Embodiment 23: Pharmacodynamic Study of QHL-096-DOX, QHL-087-DOX, QHL-090-DOX, QHL-093-DOX, QHL-117-DOX in the Treatment of CT26 Tumor Immune Model**

Objective: To study the antitumor effect of the above compounds in immunotherapy of CT26 tumor model.

Test drug: QHL-096-DOX, QHL-087-DOX, QHL-090-DOX, QHL-093-DOX, QHL-117-DOX and control group, dose 36 umol/kg, mouse PD-1 antibody, 5 mg/kg.

Test animals: BALB/c mice aged 6-8 weeks, all female.

Preparation of tumor models:

1) CT26 cells were purchased from ATCC. Cells were cultured in DMEM medium containing 10% fetal bovine serum at 37°C in 5% $CO_2$. Passages were performed every three days and cells up to passage 15 were used.

2) The generation of tumor. The corresponding $5\times10^6$ cells were injected subcutaneously into the back of nude mice. After the size of tumors reached at least 100 $mm^3$, the mice were randomized into groups. Treatment was then initiated, with the day of treatment initiation being Day 1.

3) The course of treatment. The drug was administered at an equimolar dose of 36 umol/kg. The control group was given normal saline. The drug was administered once a week for three week.

4) Tumor CD8+ T cell analysis. Tumor tissue was homogenized, individual cells in the tumor were filtered, dissociated and washed twice with buffer, and then incubated with leukocyte common antigen CD45-PE and CD8-FITC-labeled antibodies for 1 hour at room temperature. The cells were washed twice with phosphate buffered saline containing 1% fetal bovine serum, and then the proportion of T lymphocyte antigen (CD8)-positive cells in leukocyte common antigen (CD45)-positive cells was analyzed by flow cytometry. An increase in the ratio indicates an increase in T lymphocytes and therefore an improvement in the immunity of the animal to the tumor.

4) The grouping and test results are shown in Table 7.

Table 7: Effects of corresponding compounds and controls on tumor suppression and immune activation

| Group | Number of animals | Tumor volume ($mm^3$) | Tumor inhibition rate (%) | CD8: CD45 (%) |
|---|---|---|---|---|
| | | Day 28 | Day 28 | |
| Normal saline | 6 | 1887.6±646.8 | 0 | 5.2 |
| PD-1 | 6 | 1574.6± 474.5 | 16.6% | 6.1 |
| C3: EMC-AANL-DOX | | 624.5± 313.6 | 66.9% | 8.9 |
| QHL-096-DOX | 6 | 347.7± 207.1 | 81.6% | 11.8 |
| QHL-087-DOX | 6 | 214.8±134.2 | 88.6% | 12.5 |
| QHL-090 -DOX | 6 | 335.7± 257.8 | 82.2% | 15.2 |
| QHL-093-DOX | 6 | 323.7± 242.8 | 82.9% | 11.3 |
| QHL-117-DOX | 6 | 306.4± 197.8 | 83.8% | 9.5 |
| C3 +PD-1 | 6 | 74.3±45.8 | 96.1% | 11.7 |
| QHL-087-Dabrafenib | 6 | 678.4±348.7 | 64.0% | 6.4 |
| QHL-090-Dabrafenib | 6 | 589.7±247.4 | 68.7% | 7.4 |
| QHL-140-Resiquimod | 6 | 342.8±112.9 | 81.9% | 15.4 |
| QHL-096-DOX+PD-1 | 6 | 44.3±25.6 | 97.7% | 18.4 |
| QHL-087-DOX+PD-1 | 6 | 0 | 100% | 19.7 |
| QHL-090-DOX+PD-1 | 6 | 0 | 100% | 21.7 |
| QHL-093-DOX+PD-1 | 6 | 0 | 100% | 18.4 |
| QHL-117-DOX+PD-1 | 6 | 64.6±42.6 | 96.6% | 20.2 |
| QHL-087-dabrafenib + PD-1 | 6 | 178.6±67.4 | 90.6% | 13.2 |
| QHL-090-dabrafenib + PD-1 | 6 | 104.7±78.4 | 94.5% | 17.8 |
| QHL-140-Resiquimod +PD-1 | 6 | 59.7±32.7 | 96.9% | 19.4 |

5) Results and discussion: The therapeutic effect of C3, QHL-096-DOX, QHL-087-DOX, QHL-090-DOX, QHL-093-DOX, QHL-117-DOX combined with PD-1 was improved compared with single drug and could be cured tumor.

QHL-090-dabrafenib, QHL-090-dabrafenib combined with PD-1 also has a certain synergistic effect.

**Embodiment 24: Study on the Efficacy of QHL-087-DOX Injection in Various Tumor Models**

Objective: To study the antitumor spectrum of QHL-087 in several mouse tumor models.

Test drug: QHL-087-DOX was injected and diluted to the corresponding concentration with normal saline during the test.

Method and results:

1. Animals: Nude mice aged 6-8 weeks, all female.

2. Preparation of tumor models:

1) The corresponding cells were purchased from ATCC and identified according to the instructions provided. Cells were cultured in DMEM medium containing 10% fetal bovine serum at 37°C in 5% $CO_2$. Passages were performed every three days and cells up to passage 15 were used.

2) The generation of tumor. The corresponding $5\times10^6$ cells were injected subcutaneously into the back of nude mice. After the size of tumors reached at least 100 $mm^3$, the mice were randomized into groups. Treatment was then initiated, with the day of treatment initiation being Day 1.

3) The course of treatment. According to its clinical application, QHL-087-DOX was administered at a dose of 36 umol/kg. The control group was given normal saline. The drug was administered once a week for three week.

4) The grouping and test results are shown in Table 8.

Table 8: Therapeutic Effects of QHL-087-DOX in Multiple Tumor Models

| Group | Tumor cell | Tumor Inhibition Rate (Day 26) |
|---|---|---|
| Human breast cancer | MDA-MB435 | 91.5% |
| Human ovarian carcinoma | SK-OV-3 | 78.7% |
| Human colon cancer | HT-29 | 85.3% |
| Human chronic leukemia | K562 | 79.4% |
| Human colon cancer | HT1080 | 90.5% |
| Human pancreatic carcinoma | Panc-1 | 75.7% |
| Human non-small cell lung cancer | A549 | 75.8% |
| Human live cancer | Hepg2 | 100% |
| Human renal carcinoma | OS-RC-2 | 87.4% |

5) Results and discussion: QHL-087-DOX showed excellent efficacy in many tumor models, indicating that the drug has a broad anti-tumor spectrum.

**Embodiment 25: Solubility Comparison of HSA-EMC-AANL-DOX, HSA-QHL-087-DOX, and HSA-QHL-087-N-CBP with Reference Compounds**

The freeze-dried products EMC-AANL-DOX, HSA-EMC-AANL-DOX, HSA-QHL-087-DOX and HSA-QHL-087-N-CBP prepared by the embodiment of the disclosure are subpackaged in a sterile room and redissolved by water for injection. HSA-EMC-AANL-DOX, HSA-QHL-087-DOX and HSA-QHL-087-N-CBP were all able to dissolve completely as shown in Table 9.

Table 9

| Compounds | Water for injection |
|---|---|
| EMC-AANL-DOX | Insolubility |
| HSA-EMC-AANL-DOX | Dissolved, 5mg/ml |
| HSA-QHL-087-DOX | Dissolved, 35mg/ml |
| HSA-QHL-087-N-CBP | Dissolved, 35mg/ml |

As can be seen from Table 9, human albumin coupled to the compounds further improved the solubility. HSA-EMC-AANL-DOX, HSA-QHL-087-DOX and HSA-QHL-087-N-CBP as macromolecular protein medicine may be dissolved directly in water for injection or physiological saline solution to high concentration without use of irritant organic solvent for EMC-AANL-DOX dissolution. Different from the waterinsoluble EMC-AANL-DOX small molecule compound drugs, the change of solubility characteristics has great influence on the distribution and metabolism of drugs and the mode of action of drugs.

**Embodiment 26: Comparison of Solution Stability of the Water-Soluble Highly Efficient Targeted Activated Adriamycin Derivative Prepared in the Embodiment of the Disclosure with that of a Control Compound**

Accurately weigh compounds EMC-AANL-DOX, HSA-EMC-AANL-DOX, QHL-087-DOX, HSA-QHL-087-DOX, QHL-087-N-CBP and HSA-QHL-087-N-CBP separately, aliquot 5.0 mg of each sample in a sterile room. Add 0.5ml of sterile water for injection to prepare a 10mg/ml mother solution. EMC-AANL-DOX needs 50% ethanol to dissolve. Take 30ul of the mother solution, add 570ul of buffer solutions with different pH values of 5.5, and prepare a 0.5mg/ml sample solution. After the sample was clarified, it was placed in a 25°C/37°C water bath, and after 8 hours, the sample was sampled by HPLC and electrophoresis to detect the content of the sample relative to 0 hours, and the solution stability data of different compounds could be obtained. The results were shown in Table 10.

Table 10: Stability data of the solution

| Compounds | 8hr |
|---|---|
| EMC-AANL-DOX | 94.3% |
| HSA-EMC-AANL-DOX | 97.8% |
| QHL-087-DOX | 98.2% |
| HSA-QHL-087-DOX | 100.2% |
| QHL-087-N-CBP | 85.3% |
| HSA-QHL-087-N-CBP | 99.8% |

It can be seen from the data in the above table that the stability of albumin conjugated compounds is increased at 25 °C and pH=5.5, which is more obvious for QHL-087-N-CBP and HSA-QHL-087-N-CBP.

**Embodiment 27: Activation Efficiency Assay of HSA-EMC-AANL-DOX, HSA-QHL-087-DOX and HSA-QHL-087-N-CBP**

EMC-AANL-DOX was dissolved in solvent (50% water for injection +50% alcohol), HSA-EMC-AANL-DOX, HSA-QHL-087-DOX and HSA-QHL-087-N-CBP were uniformly dissolved in water for injection and diluted 10 times to 1 mg/ml with water. In the experiments of the present disclosure, 1 mg/ml of the sample compound was added to 100 μg of acidified tumor tissue homogenate (pH 6.0) at 37°C. The enzyme in the tumor tissue homogenate can cause the release of doxorubicin, and the reduction of compound and the increase of doxorubicin could be detected by HPLC to compare the activation efficiency of the drug in the tumor tissue. The compounds EMC-AANL-DOX, HSA-EMC-AANL-DOX, HSA-QHL-087-DOX, and HSA-QHL-087-N-CBP linkages of the present disclosure were found to have the highest activation efficiency among the screened compounds by screening. The results were shown in Table 11. Table 11: Compound activation ratios (%) of EMC-AANL-DOX, HSA-EMC-AANL-DOX, HSA-QHL-087-DOX, and HSA-QHL-087-N-CBP in different tumor tissue homogenates

| | Tumorproducing cells | EMC-AANL-DOX | HSA-EMC-AANL-DOX | HSA-QHL-087-DOX | HSA-QHL-087-N-CBP |
|---|---|---|---|---|---|
| Human fibrosarcoma | HT-1080 | 44.7 | 75.4 | 87.9 | 74.6 |
| Human breast cancer | MDA-MB435 | 42.3 | 71.4 | 90.4 | 92.8 |
| Human ovarian carcinoma | SK-OV-3 | 58.4 | 64.6 | 99.3 | 63.8 |
| Human colon cancer | HT-29 | 49.4 | 59.9 | 91.4 | 90.6 |

(continued)

| | Tumorproducing cells | EMC-AANL-DOX | HSA-EMC-AANL-DOX | HSA-QHL-087-DOX | HSA-QHL-087-N-CBP |
|---|---|---|---|---|---|
| Human pancreatic carcinoma | Panc-1 | 54.8 | 73.8 | 91.5 | 93.1 |
| Human nonsmall cell lung cancer | A549 | 46.4 | 69.4 | 81.4 | 83.6 |
| Human prostate cancer | PC-3 | 37.3 | 58.4 | 96.3 | 93.5 |
| Human live cancer | Hep G2 | 75.3 | 84.6 | 93.5 | 94.2 |
| Human renal carcinoma | OS-RC-2 | 46.4 | 61.5 | 96.4 | 90.5 |
| Human heart | | 0.2 | / | 0.3 | / |

**Embodiment 28: Toxicity Assay of EMC-AANL-DOX, HSA-EMC-AANL-DOX, QHL-087-DOX, HSA-QHL-087-DOX, QHL-087-CBP and HSA-QHL-087-CBP**

Objective: To understand the acute toxicity of the drug organism of the disclosure through the test of determining the MTD of intravenous administration in mice.

Test drug: EMC-AANL-DOX was dissolved with solvent (50% water for injection, 50% alcohol), HSA-EMC-AANL-DOX, QHL-087-DOX, HSA-QHL-087-DOX, QHL-087-N-CBP and HSA-QHL-087-N-CBP were dissolved with water for injection, and diluted with normal saline to the corresponding dose during the test.

Animals: Grade I BALB/C mice (purchased from Shanghai Slac Laboratory Animal Co., Ltd.), weighing 19-21g, all female.

Methods and Results: Thirty-six female BALB/C mice weighing 19- 21g were used. They were randomly divided into 7 groups according to body weight, 6 mice in each group. EMC-AANL-DOX, HSA-EMC-AANL-DOX, QHL-087-DOX, HSA-QHL-087-DOX, QHL-087-N-CBP and HSA-QHL-087-N-CBP were administered intravenously at the doses shown in Table 12. The control test of saline group and paclitaxel group injection (commercially available, Beijing Youcare pharmaceutical) was carried out, the volume of administration was 0.2ml for each mouse. The mice were observed for 17 consecutive days, whether they appeared piloerection, disorder and lackluster, lethargy, hunchback, overreaction, etc., and the body weight and death situation were recorded. Blood samples were collected for whole blood count on the 3rd, 5th and 14th day, and the animals were dissected on the 14th day to take heart, liver, kidney, lung, spleen and pancreas for HE staining observation.

Table 12: Comparison of mortality results of mice receiving different doses of compound injection, normal saline and paclitaxel injection respectively

| Group | | Dose ($\mu$mol/kg) | Number of mice | Number of dead mice |
|---|---|---|---|---|
| 1 | Normal saline | 0 | 10 | 0 |
| 2 | EMC-AANL-DOX | 38.4 | 10 | 8 |
| 3 | HSA-EMC-AANL-DOX | 38.4 | 10 | 0 |
| 4 | QHL-087-DOX | 38.4 | 10 | 4 |
| 5 | HSA-QHL-087-DOX | 38.4 | 10 | 0 |
| 6 | QHL-087-N-CBP | 19.2 | 10 | 7 |
| 7 | HSA-QHL-087-N-CBP | 19.2 | 10 | 0 |

Result and discussion: when HSA-EMC-AANL-DOX, HSA-QHL-087-DOX and HSA-QHL-087-N-CBP were injected, the tested mice do not have the conditions of piloerection, disorder and lackluster, lethargy, hunching, overreaction and death, which shows that the toxicity of the albumin couple medicine is obviously lower than that of the uncoupled medicine.

**Embodiment 29: Therapeutic effect of HSA-EMC-AANL-DOX, HSA-QHL-087-DOX in combination with Anti-PD-1 antibody**

Objective: To compare the therapeutic effects of EMC-AANL-DOX, HSA-QHL-087-DOX and anti-PD-1 antibody.

Test drug: HSA-EMC-AANL-DOX and HSA-QHL-087-DOX were used at the dose of 18 $\mu$ mol/kg; 5 mg/kg of mouse PD-1 antibody.

Test animals: BALB/c mice aged 6-8 weeks, all female.

Preparation of tumor models: CT26 cells were purchased from ATCC. Cells were cultured in DMEM medium containing 10% fetal bovine serum at 37°C in 5% $CO_2$. Passages were performed every three days and cells up to passage 15 were used. Mice were injected subcutaneously with $5 \times 10^6$ CT26 cancer cells. Mice were injected with the drug 3 times a week and anti-PD-1 antibody twice a week for a total of 8 injections.

Results and discussion: The results are shown in Figure 12. The therapeutic effect of HSA-QHL-087-DOX combined with anti-PD-1 antibody is higher than that of HSA-EMC-AANL-DOX, and the cure rate is higher.

**Embodiment 30: The inhibitory effects of N-CBP and HSA-QHL-095-N-CBP on the growth of tumor cells were determined by MTT** assay

After counting the separated and cultured cells, adjust the cell concentration with culture medium, inoculate the cells on a 96-well culture plate with 100 $\mu$l cell suspension per well, 100000 cells/well of CD8+ T cells and 20000 cells/well of CT26 tumor cells. The 96-well plate was incubated overnight for 24 hour at 37°C in a carbon dioxide (5%) incubator. After 24 hours, 100 ul of cell culture medium containing different concentrations of drugs was added to the 96-well culture plate, and control wells (0.1%DMSO) without drugs but with corresponding drug solvent were set, and blank wells (Blank) with medium but without cells were set. Each set was prepared in triplicate and the plates were incubated for 48 hours at 37 °C in a 5% $CO_2$ incubator. After 48 hours, 20 $\mu$l MTT (5 mg/ml) was added to each well and the incubation was continued for 4 hours. The culture medium was then gently aspirated, and 150 $\mu$l DMSO was added to each well as solvent for dissolution. After dissolution, the absorbance at 490 nm was measured with a microplate reader.

Results and discussion: The test results are shown in Figures 13 and 14. The in vitro cytotoxicity of N-CBP was stronger than carboplatin and oxaliplatin and weaker than cisplatin (Figure 13); HSA-QHL-095-N-CBP showed reduced in vitro cytotoxicity relative to N-CBP and carboplatin (Figure 14).

**Embodiment 31: Therapeutic Effects of HSA-QHL-095-N-CBP Alone and in Combination with Anti-PD-1 Antibody**

Objective: To compare the efficacy of carboplatin, HSA-QHL-095-N-CBP, and combination therapy with anti-PD-1 antibody.

Test drug: Carboplatin and HSA-QHL-095-N-CBP were used at the dose of 18 $\mu$ mol/kg; 5 mg/kg of murine PD-1 antibody.

Test animals: BALB/c mice aged 6-8 weeks, all female.

Preparation of tumor models: CT26 cells were purchased from ATCC. Cells were cultured in DMEM medium containing 10% fetal bovine serum at 37°C in 5% $CO_2$. Passages were performed every three days and cells up to passage 15 were used. Mice were injected subcutaneously with $5 \times 10^6$ CT26 cancer cells.

Treatment process: The corresponding drug was injected once a week for 3 week; Anti-PD-1 antibody was administered weekly for 4 weeks.

Results and discussion: The test results are shown in Figure 15. The equimolar dose of HSA-QHL-095-N-CBP is significantly better than that of carboplatin. The therapeutic effect of HSA-QHL-095-N-CBP combined with anti-PD-1 antibody was improved compared with that of single drug, and the curative effect is obtained.

**Embodiment 32: Effect of Inflammatory Response in Nonalcoholic Fatty Liver Disease (NAFLD) Model Mice.**

Methods: C57 mice were randomly divided into normal group (standard diet), model group (high-fat diet), simvastatin group (positive control, 3 mg/kg) and drug group (50 mg/kg), with 6 mice in each group. The normal group was fed with standard diet, and the other groups were fed with high-fat diet to induce NAFLD model. At the same time of modeling, the mice in each group were given the corresponding dose of 48umol/kg medicine IV twice a week for 8 weeks in total. 12 hour after the last administration, the serum biochemical indicators were determined by automatic biochemical analyzer: High density lipoprotein cholesterol (HDL-C), and low density lipoprotein (LDL-C). The results were shown in Table 13.

Table 13

| Group | Change from normal group | Change from normal group |
|---|---|---|
| | LDL-C | HDL-C |
| Normal group (control group ) | 100% | 100% |
| Model group | 135.6% | 66.2% |
| Positive control group (simvastatin) | 118.3% | 86.3% |
| T3 compound | 129.3% | 71.9% |
| QHL-150-T3 | 119.32% | 86.8% |
| QHL-157-T3 | 110.7% | 94.8% |
| QHL-158-T3 | 103.6% | 91.9% |
| QHL-159-T3 | 105.3% | 97.7% |
| QHL-160-T3 | 117.5% | 89.4% |

Results: Compared with the normal group, the HDL-C content in the model group was significantly decreased by 66.2%, while the LDL-C content was significantly increased by 135.6%($P<0.05$). In the QHL-158-T3 and QHL-159-T3 groups, the levels of HDL-C and LDL-C returned to normal.

## Claims

1. A compound having the structure of Formula (I):

MI-S-C-A (I)

in that formula,
MI is a maleimide group;
S is a group for improving enzyme digestion efficiency or selectivity;
C is a proteolytic enzyme cleavable amino acid linker; and the
A is the auxiliary linker.

2. The compound of claim 1, wherein MI is a maleimide group represented by the following formula:

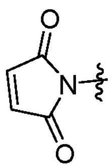

Among them, the wavy line indicates the connection position with S.

3. The compound of claim 1, wherein S is represented as S1-S2-S3, wherein S1 is selected from:

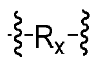

,

wherein $R_x$ is absent or selected from: $C_{1-6}$ alkylene, $C_{1-6}$ alkyleneamino, $C_{1-6}$ alkylenecarboxy and $C_{1-6}$ alkylene-carbonylamino, wavy lines indicate connections to adjacent moieties position; S2 is absent or $-[(CH_2)_pO]_q-$, wherein p is an integer of 1-4, preferably 2, and q is an integer of 0-15, preferably 1-15, more preferably 2-6; S3 is absent or selected from polar amino acid residues such as: Glu, Asp, Gly, Ala, Val, Leu, Ile, Met, Phe, Trp, Ser, Thr, Cys,

Tyr, Asn, Gln, Lys, Arg and His, preferably Glu and Asp;

wherein S is attached to C through a group selected from:

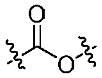 、 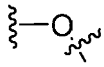 and 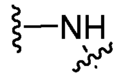 ,

The wavy line represents adjacent connecting parts.
And among them, at least one of S1, S2 and S3 exists.

4. The compound of claim 1, wherein S is $-R_1-[(CH_2)_pO]_q-R_2-R_3-$, wherein $R_1$ is attached to MI, absent or selected from $C_{1-6}$ alkylene group or $C_{1-6}$ alkylene carbonylamino; $R_2$ is selected from $C_{1-6}$ alkylene; $R_3$ is selected from -C(O)O-, -NH-, -O- or $-C(O)-R_4$, wherein, $R_4$ is an amino acid residue selected from the group consisting of Glu, Asp, Gly, Ala, Val, Leu, Ile, Met, Phe, Trp, Ser, Thr, Cys, Tyr, Asn, Gln, Lys, Arg and His, and preferably Glu and Asp group, and $R_4$ forms an amide bond with the -C(O)- through its amino group; p is an integer of 1-4; q is an integer of 0-15, preferably 1-15, more preferably 2-6.

5. The compound of any one of claims 1-4, wherein MI, S1, S2, S3, C and A are connected to each other by any of the following ways:

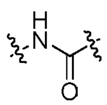 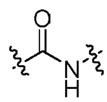 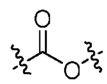 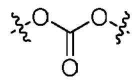 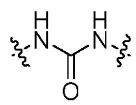 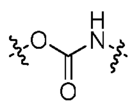 ,

The wavy line represents adjacent connecting parts.

6. The compound of claim 1, wherein MI-S is selected from:

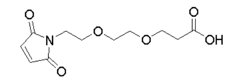 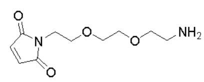 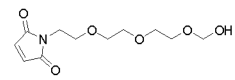

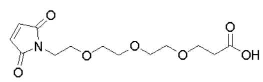 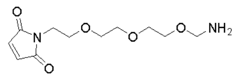 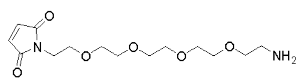

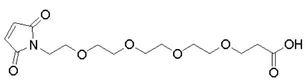 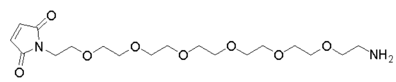

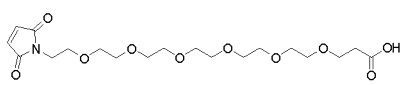 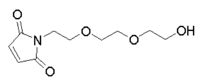

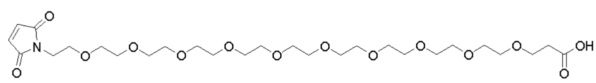 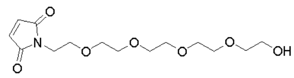

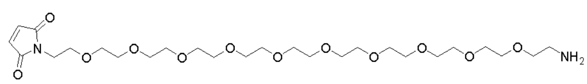

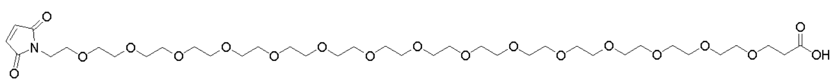

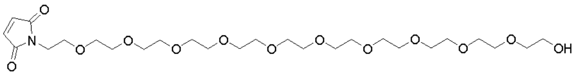
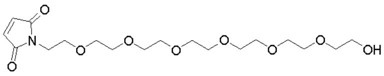

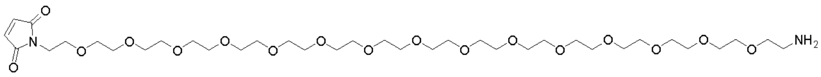

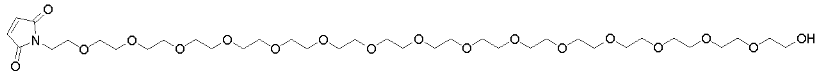

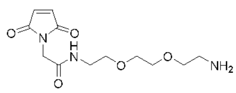
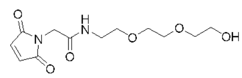
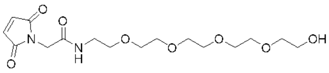

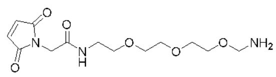
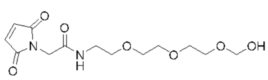
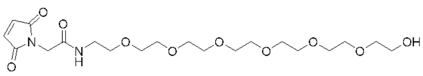

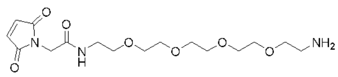
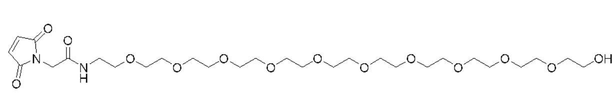

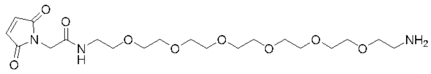
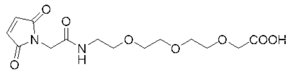

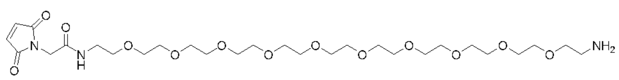
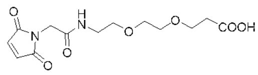

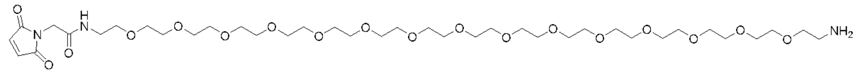

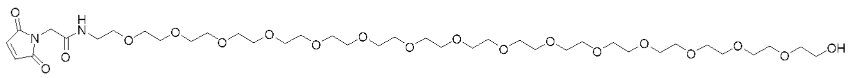

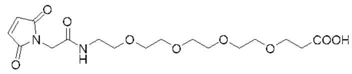
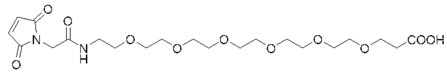

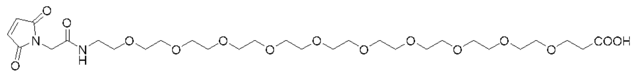

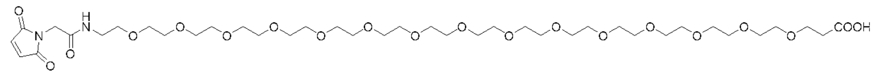

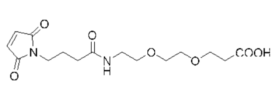
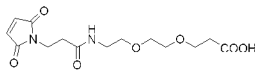

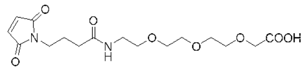
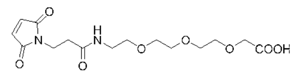
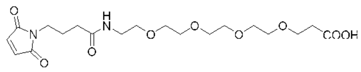
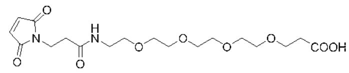
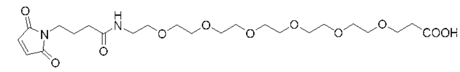
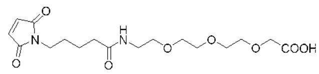
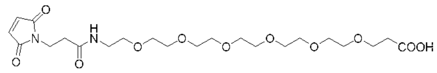
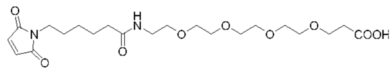
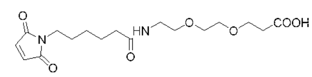
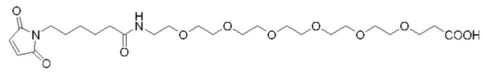
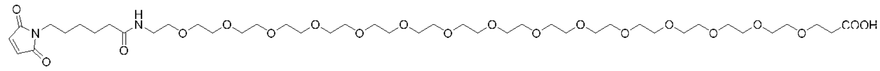
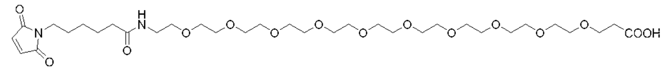
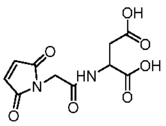
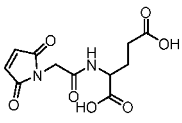
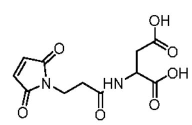
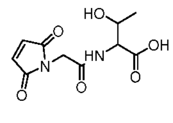
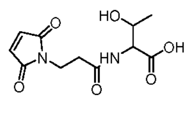
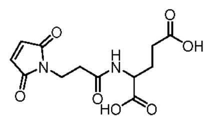
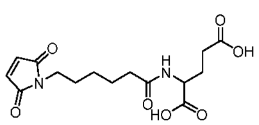
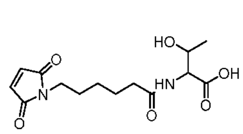
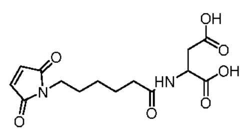
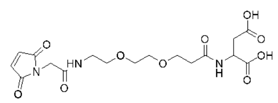
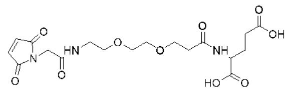
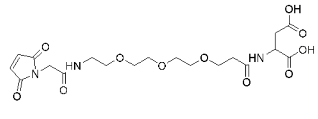
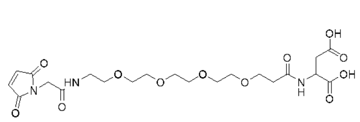
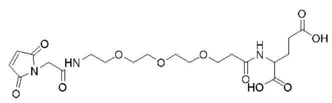
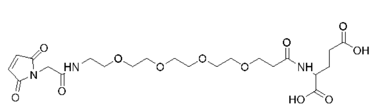
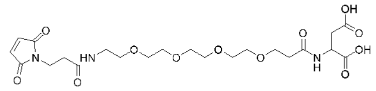
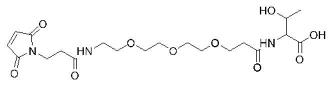
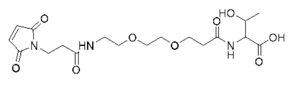
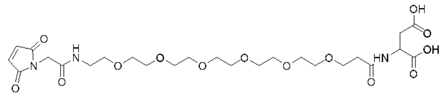
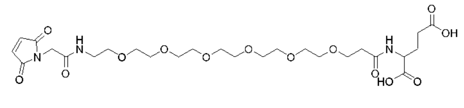

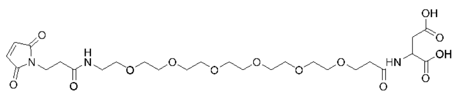
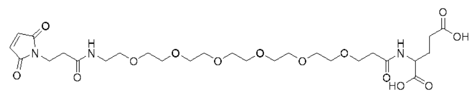

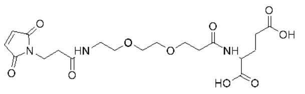
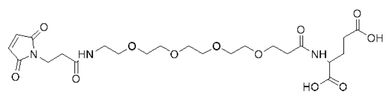
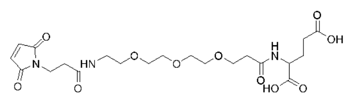
。

7. The compound of claim 1, wherein C is selected from a group that expresses asparagine endopeptidase cleavage in the tumor microenvironment, and the group contains an Asn residue.

8. The compound of claim 7, wherein C is $X_1X_2X_3$, wherein $X_1$ is selected from L or D type Ala, Thr, Val and Asn; $X_2$ is selected from L or D type Ala, Thr, Val and Ile; $X_3$ is Asn, preferably not D-Asn;
Preferably, C is selected from: Ala-Ala-Asn, Thr-Ala-Asn, Val-Ala-Asn, Asn-Ala-Asn, Thr-Thr-Asn, Val-Thr-Asn, Asn-Thr-Asn, Ala -Val-Asn, Thr-Val-Asn, Val-Val-Asn, Asn-Val-Asn, Ala-Ile-Asn, Thr-Ile-Asn, Val-Ile-Asn, Asn-Ile-Asn, Ala-Thr-Asn, D-Thr-L-Val-L-Asn, D-Thr-L-Ala-L-Asn, D-Ala-L-Val-L-Asn, L-Thr-D-Val-L-Asn , L-Thr-D-Ala-L-Asn, L-Ala-D-Val-L-Asn, D-Thr-D-Val-L-Asn, D-Thr-D-Ala-L-Asn, D -Ala-D-Val-L-Asn.

9. The compound of claim 1, wherein A is selected from:

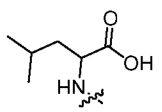
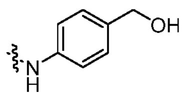
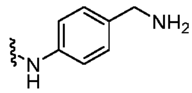
,

Where the wavy line indicates the location of the connection to C.

10. The compound of claim 1, wherein said S and A are selected from any of the following groups QHL-001 to QHL-162:

| Compound No. | S | | | A |
|---|---|---|---|---|
| | S1 | S2 | S3 | |
| QHL-001 | / | 2peg | / | $PABC-NH_2$ |
| QHL-002 | / | 3peg | / | $PABC-NH_2$ |
| QHL-003 | / | 4peg | / | $PABC-NH_2$ |
| QHL-004 | / | 6peg | / | $PABC-NH_2$ |
| QHL-005 | / | 2peg | / | PABC-OH |
| QHL-006 | / | 3peg | / | PABC-OH |
| QHL-007 | / | 4peg | / | PABC-OH |
| QHL-008 | / | 6peg | / | PABC-OH |
| QHL-009 | / | 2peg | / | Leu |
| QHL-010 | / | 3peg | / | Leu |
| QHL-011 | / | 4peg | / | Leu |
| QHL-012 | / | 6peg | / | Leu |
| QHL-013 | / | 2peg | Glu | $PABC-NH_2$ |
| QHL-014 | / | 3peg | Glu | $PABC-NH_2$ |
| QHL-015 | / | 4peg | Glu | $PABC-NH_2$ |
| QHL-016 | / | 6peg | Glu | $PABC-NH_2$ |

(continued)

| Compound No. | S | | | A |
|---|---|---|---|---|
| | S1 | S2 | S3 | |
| QHL-017 | / | 2peg | Glu | PABC-OH |
| QHL-018 | / | 3peg | Glu | PABC-OH |
| QHL-019 | / | 4peg | Glu | PABC-OH |
| QHL-020 | / | 6peg | Glu | PABC-OH |
| QHL-021 | / | 2peg | Glu | Leu |
| QHL-022 | / | 3peg | Glu | Leu |
| QHL-023 | / | 4peg | Glu | Leu |
| QHL-024 | / | 6peg | Glu | Leu |
| QHL-025 | / | 2peg | Asp | PABC-$NH_2$ |
| QHL-026 | / | 3peg | Asp | PABC-$NH_2$ |
| QHL-027 | / | 4peg | Asp | PABC-$NH_2$ |
| QHL-028 | / | 6peg | Asp | PABC-$NH_2$ |
| QHL-029 | / | 2peg | Asp | PABC-OH |
| QHL-030 | / | 3peg | Asp | PABC-OH |
| QHL-031 | / | 4peg | Asp | PABC-OH |
| QHL-032 | / | 6peg | Asp | PABC-OH |
| QHL-03 3 | / | 2peg | Asp | Leu |
| QHL-034 | / | 3peg | Asp | Leu |
| QHL-03 5 | / | 4peg | Asp | Leu |
| QHL-03 6 | / | 6peg | Asp | Leu |
| QHL-03 7 | $-CH_2CH_2-CONH-$ | 2peg | Glu | PABC-$NH_2$ |
| QHL-03 8 | $-CH_2CH_2-CONH-$ | 2peg | Glu | PABC-OH |
| QHL-03 9 | $-CH_2CH_2-CONH-$ | 2peg | Glu | Leu |
| QHL-040 | $-CH_2CH_2-CONH-$ | 2peg | Asp | PABC-$NH_2$ |
| QHL-041 | $-CH_2CH_2-CONH-$ | 2peg | Asp | PABC-OH |
| QHL-042 | $-CH_2CH_2-CONH-$ | 2peg | Asp | Leu |
| QHL-043 | $-CH_2CH_2-CONH-$ | 3peg | Glu | PABC-$NH_2$ |
| QHL-044 | $-CH_2CH_2-CONH-$ | 3peg | Glu | PABC-OH |
| QHL-045 | $-CH_2CH_2-CONH-$ | 3peg | Glu | Leu |
| QHL-046 | $-CH_2CH_2-CONH-$ | 3peg | Asp | PABC-$NH_2$ |
| QHL-047 | $-CH_2CH_2-CONH-$ | 3peg | Asp | PABC-OH |
| QHL-048 | $-CH_2CH_2-CONH-$ | 3peg | Asp | Leu |
| QHL-049 | $-CH_2CH_2-CONH-$ | 4peg | Glu | PABC-$NH_2$ |
| QHL-050 | $-CH_2CH_2-CONH-$ | 4peg | Glu | PABC-OH |
| QHL-051 | $-CH_2CH_2-CONH-$ | 4peg | Glu | Leu |
| QHL-052 | $-CH_2CH_2-CONH-$ | 4peg | Asp | PABC-$NH_2$ |
| QHL-053 | $-CH_2CH_2-CONH-$ | 4peg | Asp | PABC-OH |

(continued)

| Compound No. | S | | | A |
|---|---|---|---|---|
| | S1 | S2 | S3 | |
| QHL-054 | $-CH_2CH_2$-CONH- | 4peg | Asp | Leu |
| QHL-055 | $-CH_2CH_2$-CONH- | 6peg | Glu | PABC-$NH_2$ |
| QHL-056 | $-CH_2CH_2$-CONH- | 6peg | Glu | PABC-OH |
| QHL-057 | $-CH_2CH_2$-CONH- | 6peg | Glu | Leu |
| QHL-058 | $-CH_2CH_2$-CONH- | 6peg | Asp | PABC-$NH_2$ |
| QHL-059 | $-CH_2CH_2$-CONH- | 6peg | Asp | PABC-OH |
| QHL-060 | $-CH_2CH_2$-CONH- | 6peg | Asp | Leu |
| QHL-061 | $-CH_2CH_2CH_2$-CONH- | 2peg | Glu | PABC-$NH_2$ |
| QHL-062 | $-CH_2CH_2CH_2$-CONH- | 2peg | Glu | PABC-OH |
| QHL-063 | $-CH_2CH_2CH_2$-CONH- | 2peg | Glu | Leu |
| QHL-064 | $-CH_2CH_2CH_2$-CONH- | 2peg | Asp | PABC-$NH_2$ |
| QHL-065 | $-CH_2CH_2CH_2$-CONH- | 2peg | Asp | PABC-OH |
| QHL-066 | $-CH_2CH_2CH_2$-CONH- | 2peg | Asp | Leu |
| QHL-067 | $-CH_2CH_2CH_2$-CONH- | 3peg | Glu | PABC-$NH_2$ |
| QHL-068 | $-CH_2CH_2CH_2$-CONH- | 3peg | Glu | PABC-OH |
| QHL-069 | $-CH_2CH_2CH_2$-CONH- | 3peg | Glu | Leu |
| QHL-070 | $-CH_2CH_2CH_2$-CONH- | 3peg | Asp | PABC-$NH_2$ |
| QHL-071 | $-CH_2CH_2CH_2$-CONH- | 3peg | Asp | PABC-OH |
| QHL-072 | $-CH_2CH_2CH_2$-CONH- | 3peg | Asp | Leu |
| QHL-073 | $-CH_2CH_2CH_2$-CONH- | 4peg | Glu | PABC-$NH_2$ |
| QHL-074 | $-CH_2CH_2CH_2$-CONH- | 4peg | Glu | PABC-OH |
| QHL-075 | $-CH_2CH_2CH_2$-CONH- | 4peg | Glu | Leu |
| QHL-076 | $-CH_2CH_2CH_2$-CONH- | 4peg | Asp | PABC-$NH_2$ |
| QHL-077 | $-CH_2CH_2CH_2$-CONH- | 4peg | Asp | PABC-OH |
| QHL-078 | $-CH_2CH_2CH_2$-CONH- | 4peg | Asp | Leu |
| QHL-079 | $-CH_2CH_2CH_2$-CONH- | 6peg | Glu | PABC-$NH_2$ |
| QHL-080 | $-CH_2CH_2CH_2$-CONH- | 6peg | Glu | PABC-OH |
| QHL-081 | $-CH_2CH_2CH_2$-CONH- | 6peg | Glu | Leu |
| QHL-082 | $-CH_2CH_2CH_2$-CONH- | 6peg | Asp | PABC-$NH_2$ |
| QHL-083 | $-CH_2CH_2CH_2$-CONH- | 6peg | Asp | PABC-OH |
| QHL-084 | $-CH_2CH_2CH_2$-CONH- | 6peg | Asp | Leu |
| QHL-085 | $-CH_2CH_2$-CONH- | 2peg | / | PABC-$NH_2$ |
| QHL-086 | $-CH_2CH_2$-CONH- | 2peg | / | PABC-OH |
| QHL-087 | $-CH_2CH_2$-CONH- | 2peg | / | Leu |
| QHL-088 | $-CH_2CH_2$-CONH- | 3peg | / | PABC-$NH_2$ |
| QHL-089 | $-CH_2CH_2$-CONH- | 3peg | / | PABC-OH |
| QHL-090 | $-CH_2CH_2$-CONH- | 3peg | / | Leu |

(continued)

| Compound No. | S | | | A |
|---|---|---|---|---|
| | S1 | S2 | S3 | |
| QHL-091 | $-CH_2CH_2-CONH-$ | 4peg | / | $PABC-NH_2$ |
| QHL-092 | $-CH_2CH_2-CONH-$ | 4peg | / | PABC-OH |
| QHL-093 | $-CH_2CH_2-CONH-$ | 4peg | / | Leu |
| QHL-094 | $-CH_2CH_2-CONH-$ | 6peg | / | $PABC-NH_2$ |
| QHL-095 | $-CH_2CH_2-CONH-$ | 6peg | / | PABC-OH |
| QHL-096 | $-CH_2CH_2-CONH-$ | 6peg | / | Leu |
| QHL-097 | $-CH_2CH_2CH_2-CONH-$ | 2peg | / | $PABC-NH_2$ |
| QHL-098 | $-CH_2CH_2CH_2-CONH-$ | 2peg | / | PABC-OH |
| QHL-099 | $-CH_2CH_2CH_2-CONH-$ | 2peg | / | Leu |
| QHL-100 | $-CH_2CH_2CH_2-CONH-$ | 3peg | / | $PABC-NH_2$ |
| QHL-101 | $-CH_2CH_2CH_2-CONH-$ | 3peg | / | PABC-OH |
| QHL-102 | $-CH_2CH_2CH_2-CONH-$ | 3peg | / | Leu |
| QHL-103 | $-CH_2CH_2CH_2-CONH-$ | 4peg | / | $PABC-NH_2$ |
| QHL-104 | $-CH_2CH_2CH_2-CONH-$ | 4peg | / | PABC-OH |
| QHL-105 | $-CH_2CH_2CH_2-CONH-$ | 4peg | / | Leu |
| QHL-106 | $-CH_2CH_2CH_2-CONH-$ | 6peg | / | $PABC-NH_2$ |
| QHL-107 | $-CH_2CH_2CH_2-CONH-$ | 6peg | / | PABC-OH |
| QHL-108 | $-CH_2CH_2CH_2-CONH-$ | 6peg | / | Leu |
| QHL-109 | $-CH_2CH_2CH_2-CONH-$ | / | Glu | $PABC-NH_2$ |
| QHL-110 | $-CH_2CH_2CH_2-CONH-$ | / | Glu | PABC-OH |
| QHL-111 | $-CH_2CH_2CH_2-CONH-$ | / | Glu | Leu |
| QHL-112 | $-CH_2CH_2CH_2-CONH-$ | / | Asp | $PABC-NH_2$ |
| QHL-113 | $-CH_2CH_2CH_2-CONH-$ | / | Asp | PABC-OH |
| QHL-114 | $-CH_2CH_2CH_2-CONH-$ | / | Asp | Leu |
| QHL-115 | $-(CH_2)_6-CONH-$ | / | Glu | $PABC-NH_2$ |
| QHL-116 | $-(CH_2)_6-CONH-$ | / | Glu | PABC-OH |
| QHL-117 | $-(CH_2)_6-CONH-$ | / | Glu | Leu |
| QHL-118 | $-(CH_2)_6-CONH-$ | / | Asp | $PABC-NH_2$ |
| QHL-119 | $-(CH_2)_6-CONH-$ | / | Asp | PABC-OH |
| QHL-120 | $-(CH_2)_6-CONH-$ | / | Asp | Leu |
| QHL-121 | $-(CH_2)_6-CONH-$ | / | Gly | Leu |
| QHL-122 | $-(CH_2)_6-CONH-$ | / | Ala | Leu |
| QHL-123 | $-(CH_2)_6-CONH-$ | / | Val | Leu |
| QHL-124 | $-(CH_2)_6-CONH-$ | / | Leu | Leu |
| QHL-125 | $-(CH_2)_6-CONH-$ | / | Ile | Leu |
| QHL-126 | $-(CH_2)_6-CONH-$ | / | Met | Leu |
| QHL-127 | $-(CH_2)_6-CONH-$ | / | Phe | Leu |

(continued)

| Compound No. | S | | | A |
|---|---|---|---|---|
| | S1 | S2 | S3 | |
| QHL-128 | $-(CH_2)_6$-CONH- | / | Trp | Leu |
| QHL-129 | $-(CH_2)_6$-CONH- | / | Ser | Leu |
| QHL-130 | $-(CH_2)_6$-CONH- | / | Thr | Leu |
| QHL-131 | $-(CH_2)_6$-CONH- | / | Cys | Leu |
| QHL-132 | $-(CH_2)_6$-CONH- | / | Tyr | Leu |
| QHL-133 | $-(CH_2)_6$-CONH- | / | Asn | Leu |
| QHL-134 | $-(CH_2)_6$-CONH- | / | Gln | Leu |
| QHL-135 | $-(CH_2)_6$-CONH- | / | Lys | Leu |
| QHL-136 | $-(CH_2)_6$-CONH- | / | Arg | Leu |
| QHL-137 | $-(CH_2)_6$-CONH- | / | His | Leu |
| QHL-138 | $-(CH_2)_6$-CONH- | / | / | Leu |
| QHL-139 | $-(CH_2)_6$-CONH- | / | / | PABC-$NH_2$ |
| QHL-140 | $-(CH_2)_6$-CONH- | / | / | PABC-OH |
| QHL-141 | $-(CH_2)_4$-CONH- | / | / | Leu |
| QHL-142 | $-(CH_2)_4$-CONH- | / | / | PABC-$NH_2$ |
| QHL-143 | $-(CH_2)_4$-CONH- | / | / | PABC-OH |
| QHL-144 | $-CH_2CH_2$-CONH- | / | / | Leu |
| QHL-145 | $-CH_2CH_2$-CONH- | / | / | PABC-$NH_2$ |
| QHL-146 | $-CH_2CH_2$-CONH- | / | / | PABC-OH |
| QHL-147 | / | 12peg | / | Leu |
| QHL-148 | / | 12peg | / | PABC-$NH_2$ |
| QHL-149 | / | 12peg | / | PABC-OH |
| QHL-150 | $-(CH_2)_6$-CONH- | / | / | / |
| QHL-151 | $-(CH_2)_4$-CONH- | / | / | / |
| QHL-152 | $-CH_2CH_2$-CONH- | / | / | / |
| QHL-153 | / | 2peg | / | / |
| QHL-154 | / | 3peg | / | / |
| QHL-155 | / | 4peg | / | / |
| QHL-156 | / | 6peg | / | / |
| QHL-157 | $-CH_2CH_2$-CONH- | 2peg | / | / |
| QHL-158 | $-CH_2CH_2$-CONH- | 3peg | / | / |
| QHL-159 | $-CH_2CH_2$-CONH- | 4peg | / | / |
| QHL-160 | $-CH_2CH_2$-CONH- | 6peg | / | / |
| QHL-161 | $-(CH_2)_6$-CONH- | / | Glu | / |
| QHL-162 | $-(CH_2)_6$-CONH- | / | Asp | / |

Preferably, C is AAN.

**11.** A conjugate represented by the following formula (II) or a pharmaceutically acceptable salt thereof:

MI-S-C-A-D (II)

wherein MI, S, C and A are as defined in any one of claims 1 to 10; D is a drug, preferably an anticancer compound;

More preferably, D is selected from that group consisting of doxorubicin, daunorubicin, epirubicin, methotrexate, fludarabine, gemcitabine, cytarabine, melphalan, nimustine, mitoxantrone, mitomycin, camptothecin, 10-hydroxycamptothecin, topotecan, floxuridine, doxifluridine, etoposide, fludarabine, capecitabine, vincristine, epothilone B, paclitaxel, docetaxel, dabrafenib, dovitinib , motesanib, prednisone, triiodothyronine, resiquimod, and a platinum derivative represented by that following formula:

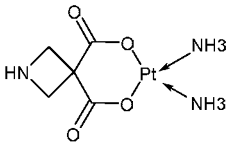
;

and the following compound a and compound b:

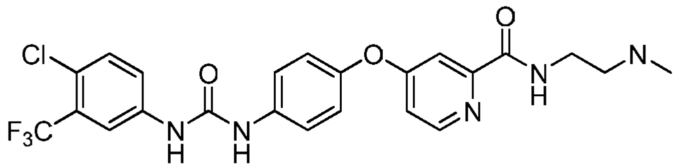

Compound a ;

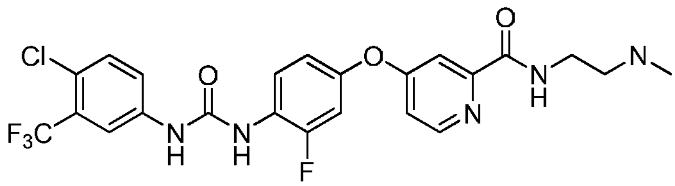

Compound b ;

Preferably, A and D are linked in any of the following ways:

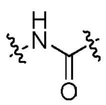 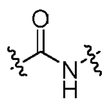 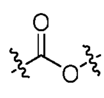 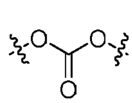 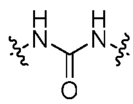 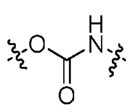 ,

The wavy line represents adjacent connecting parts.

**12.** The conjugate or pharmaceutically acceptable salt thereof of claim 11, wherein the compound is selected from the group consisting of:

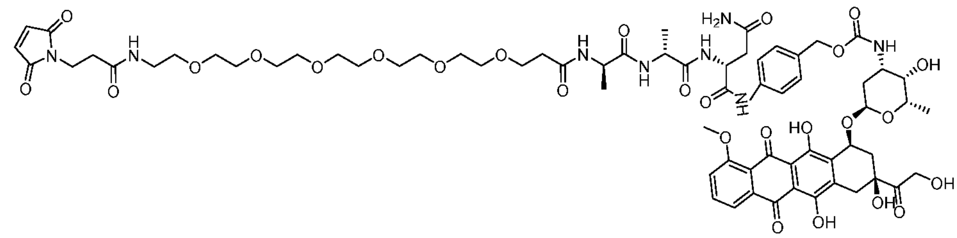

;

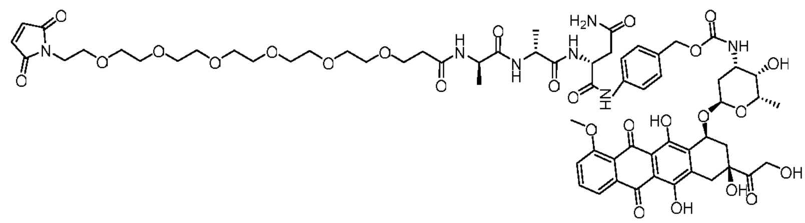

;

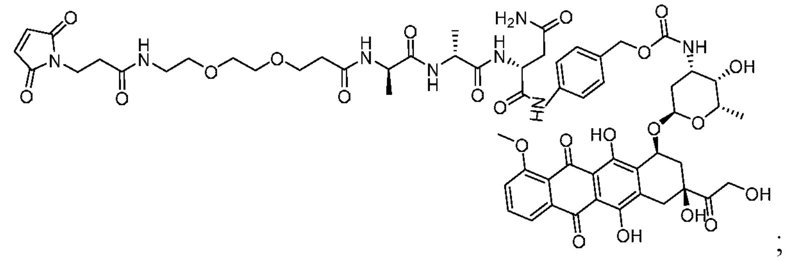

;

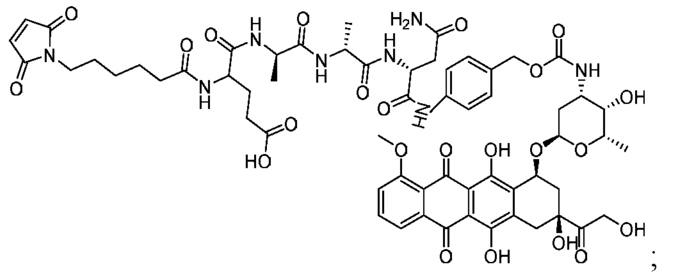

;

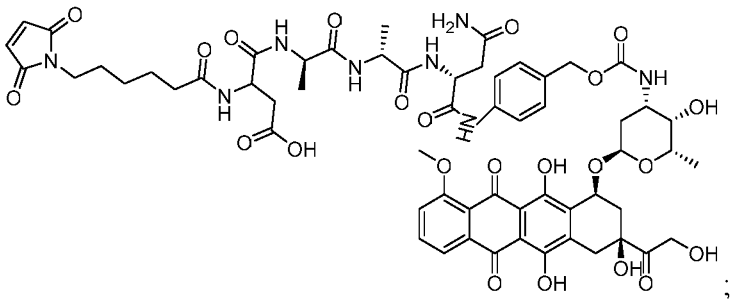

;

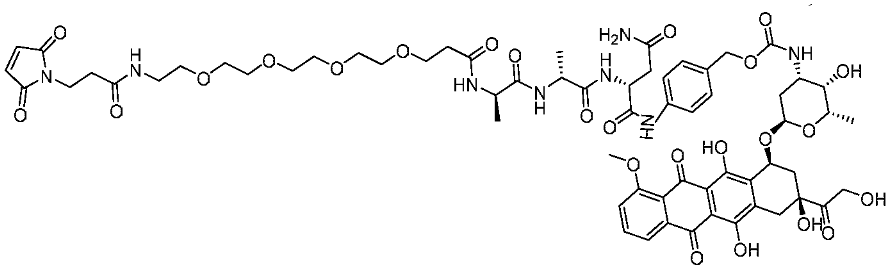

;

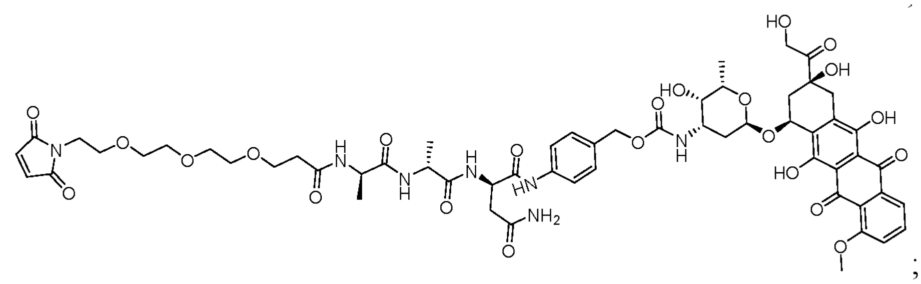

;

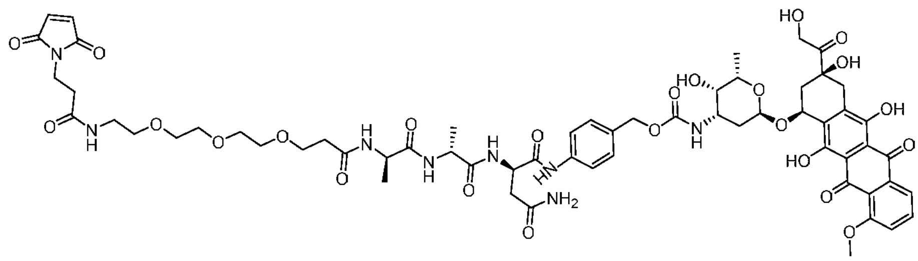

;

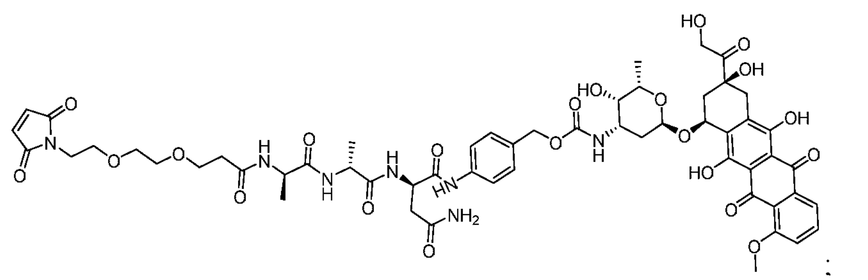

;

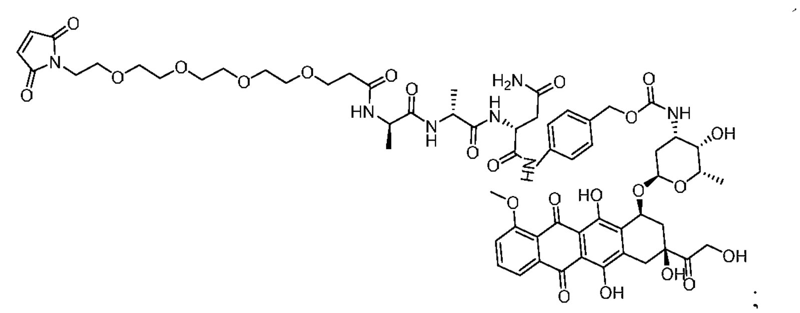

;

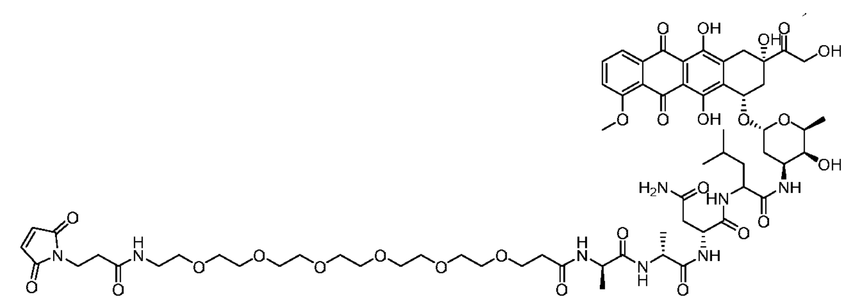

;

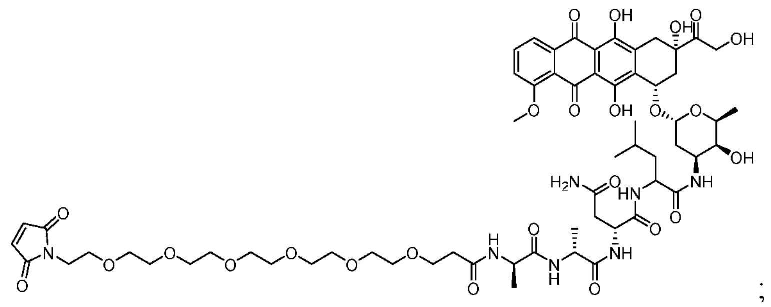

;

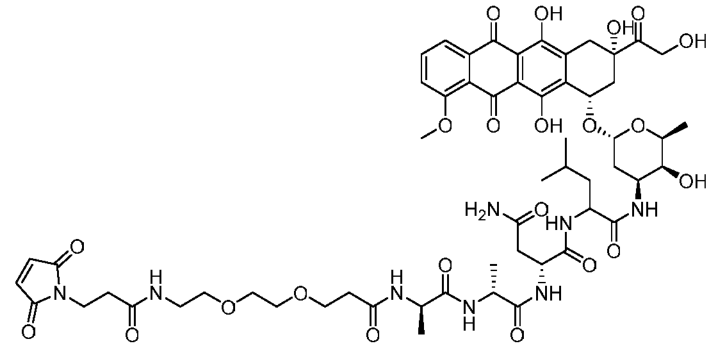

;

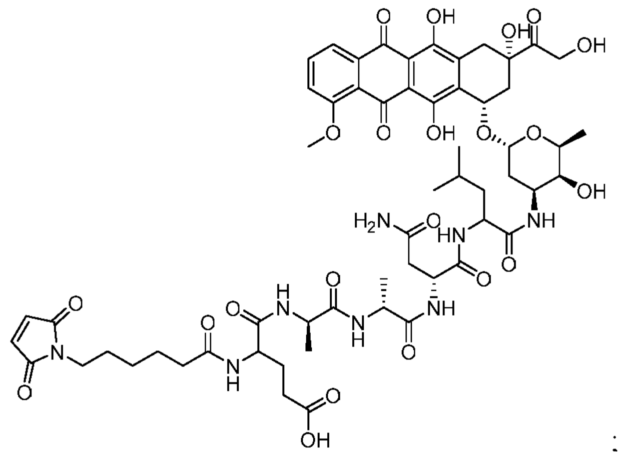

;

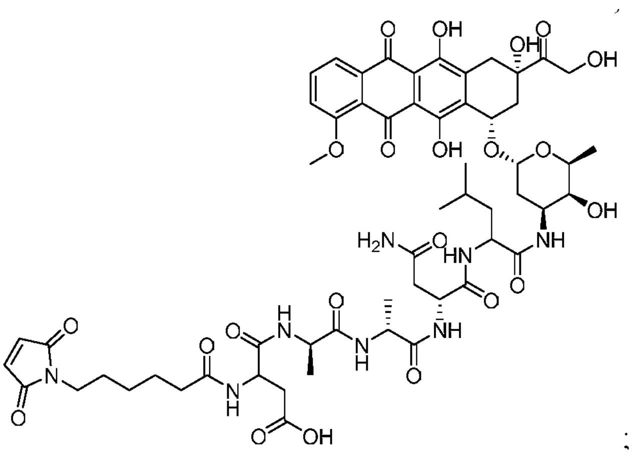

;

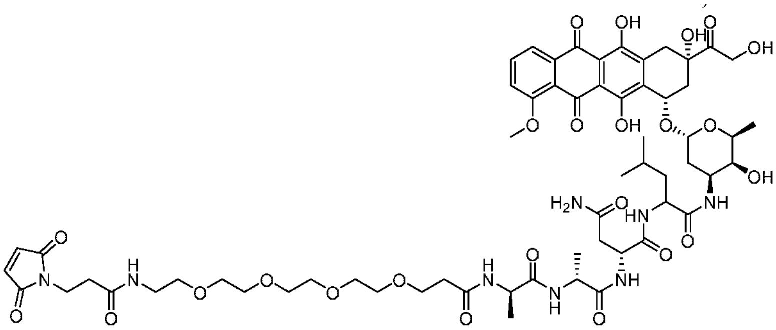

;

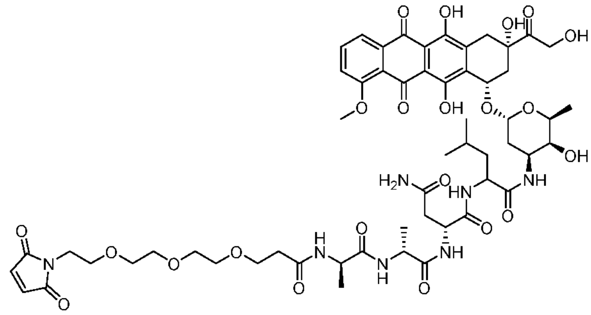

;

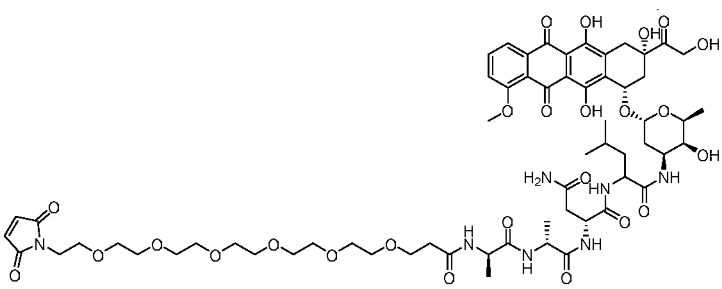

;

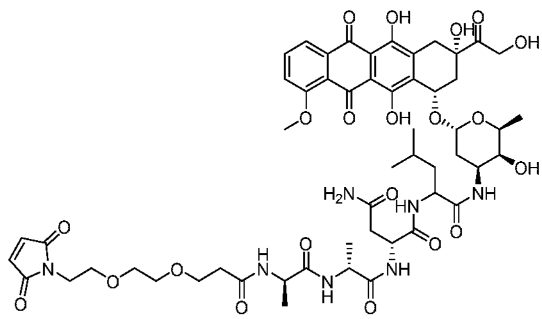

;

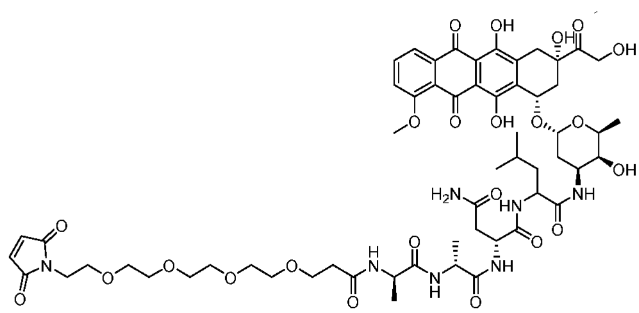

;

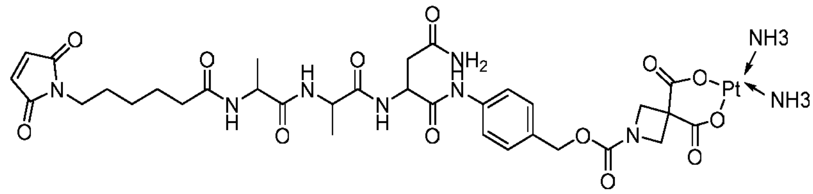

;

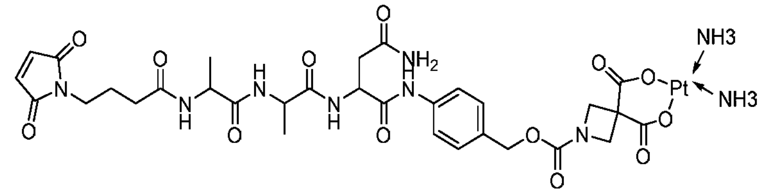

;

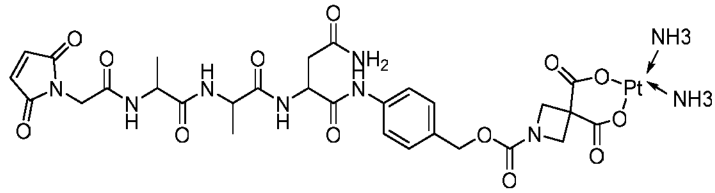

;

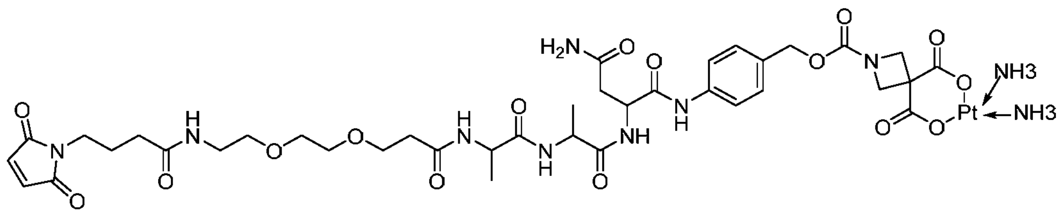

;

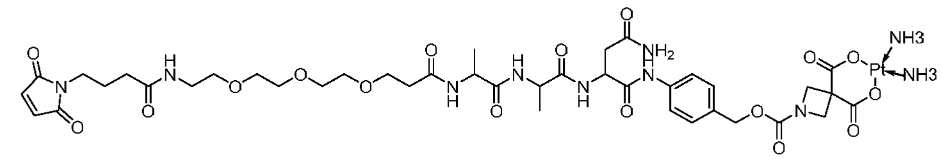

;

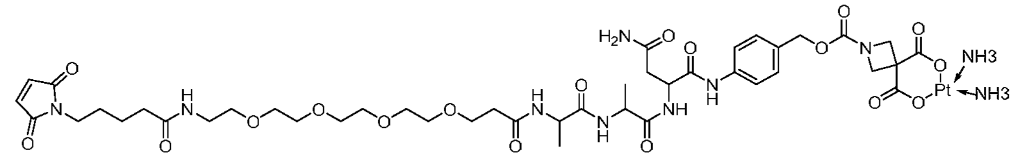

;

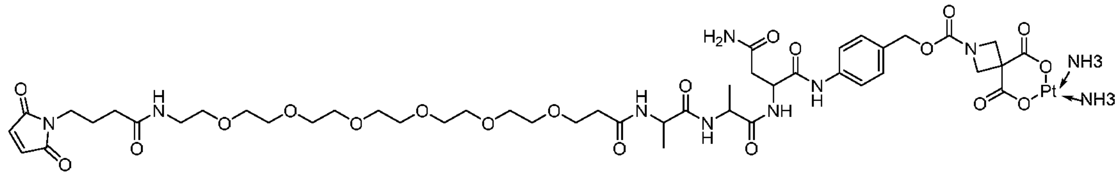

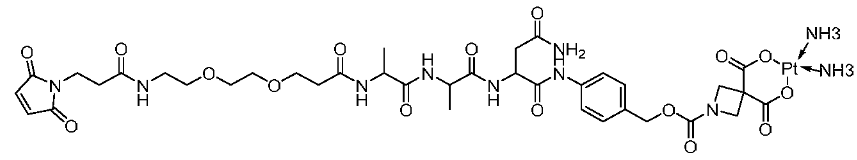

;

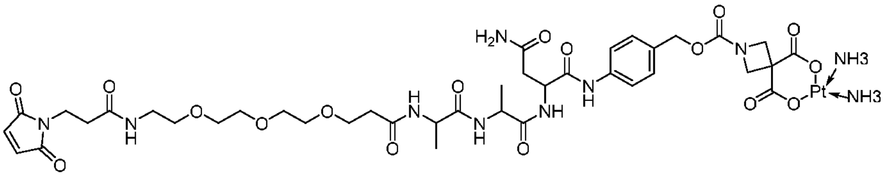

;

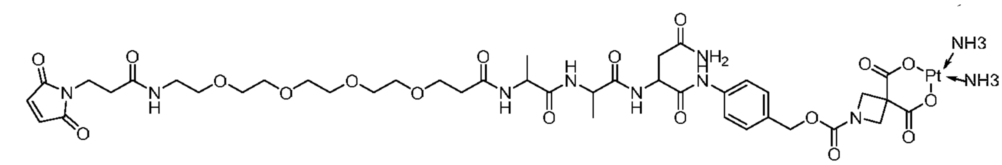

;

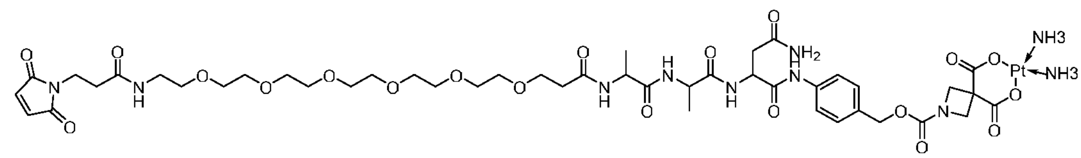

;

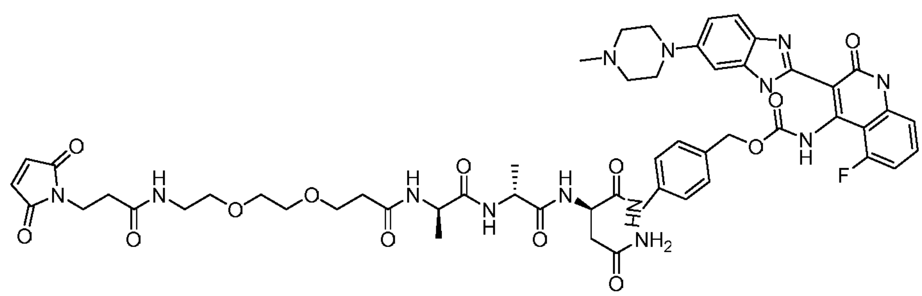

;

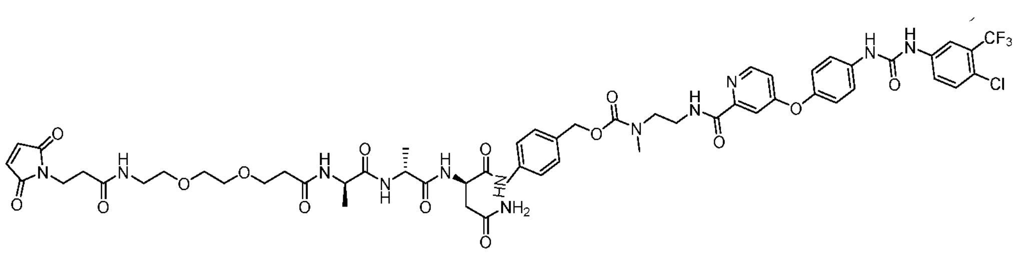

;

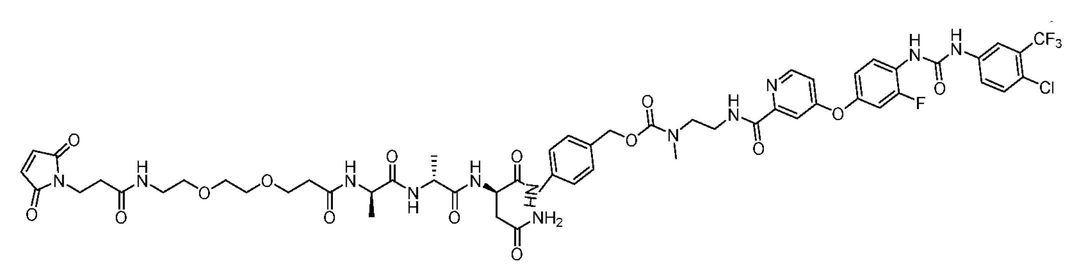

;

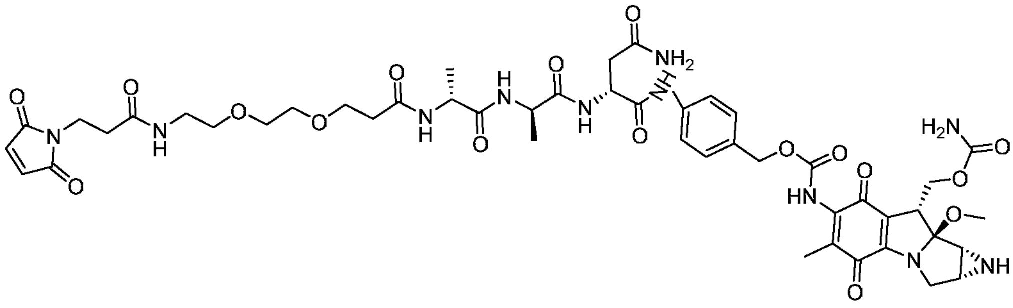

;

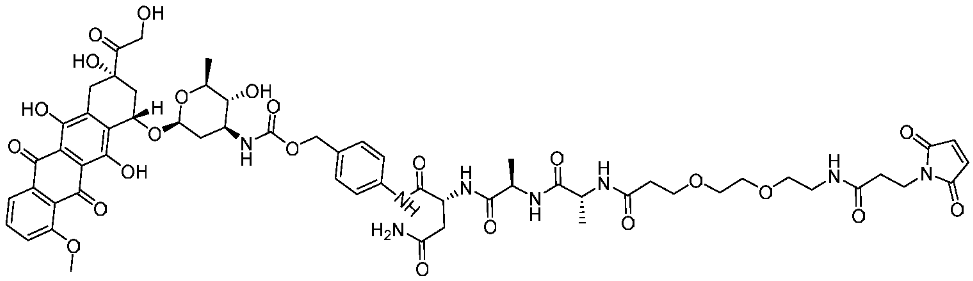

;

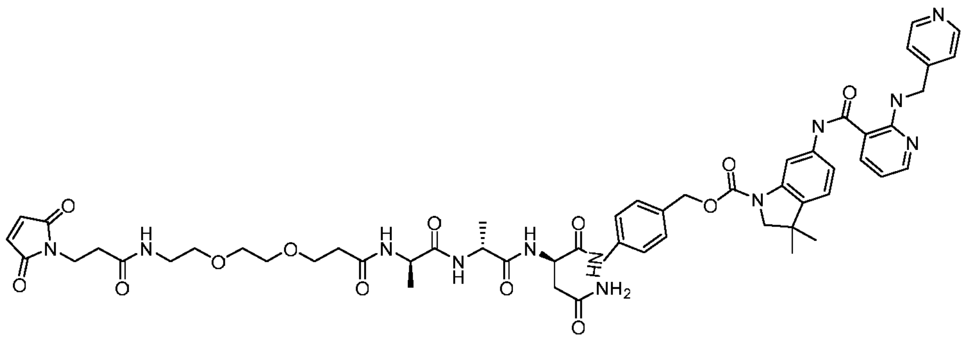

;

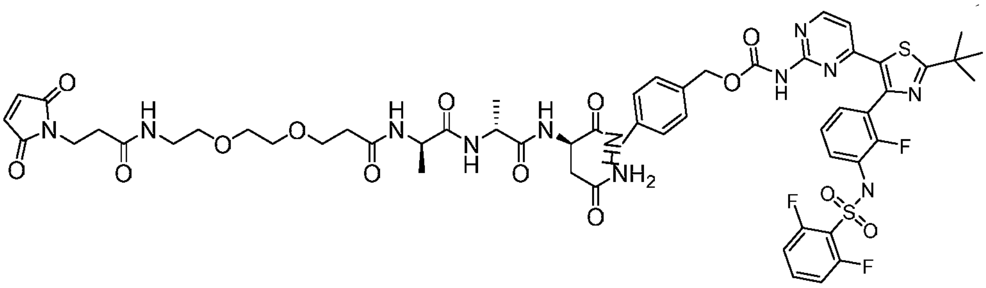

;

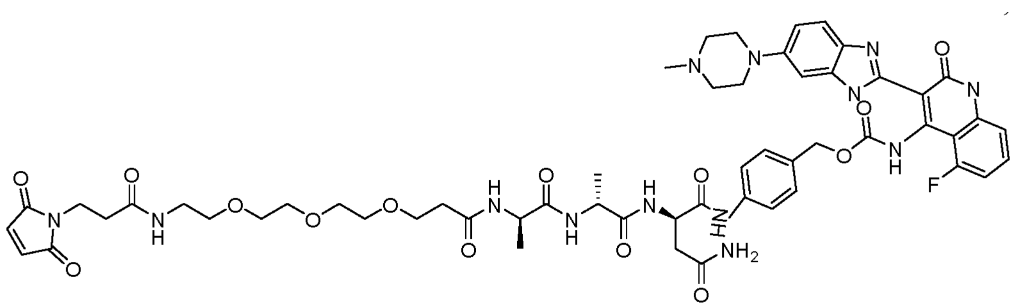

;

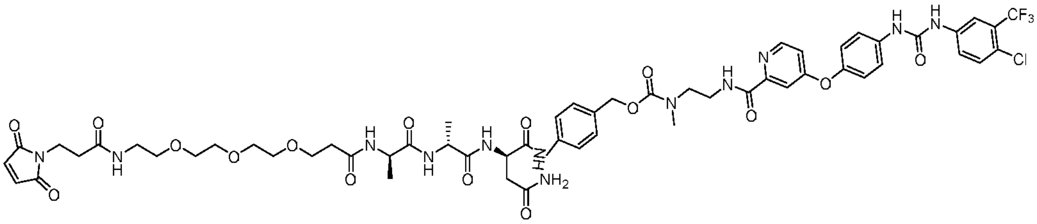

;

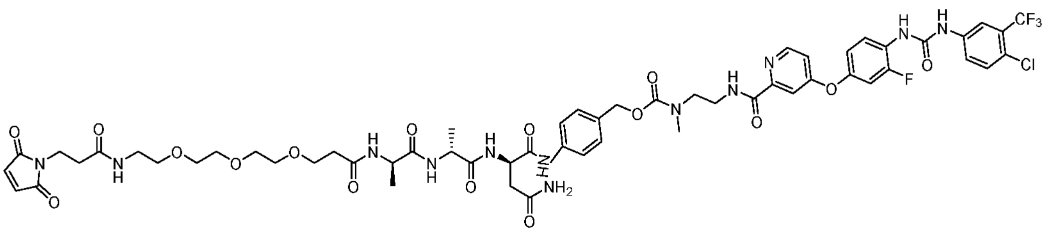

;

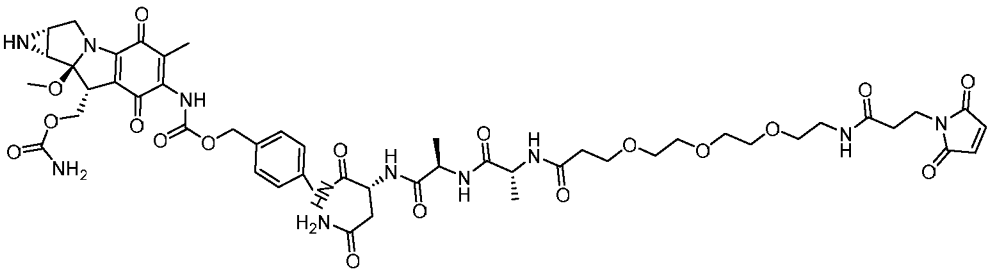

;

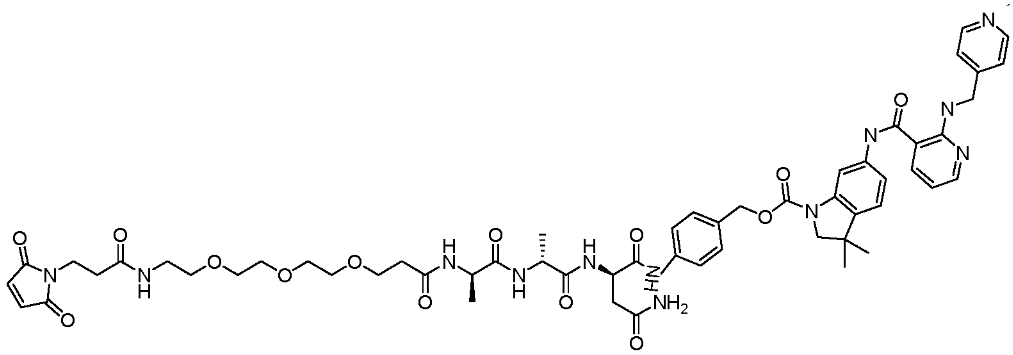

;

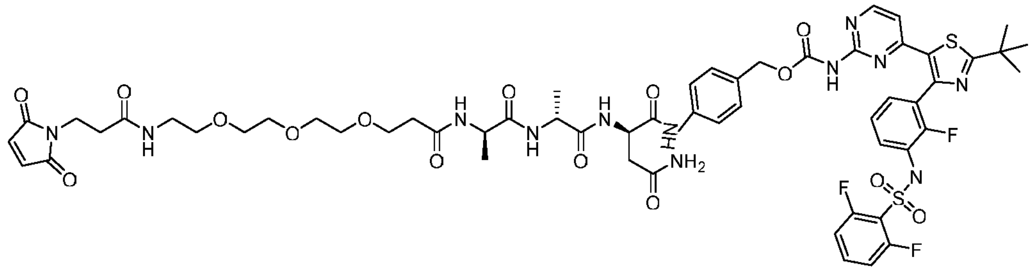

;

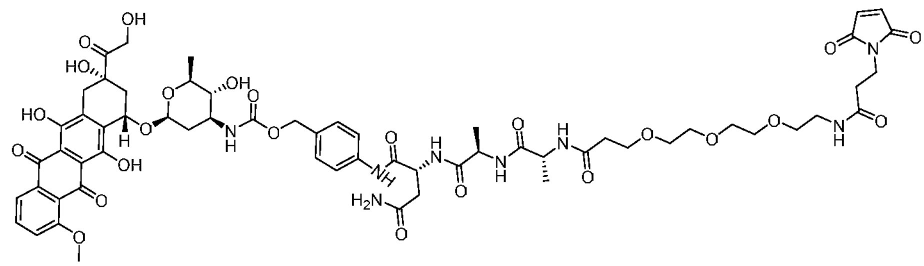

;

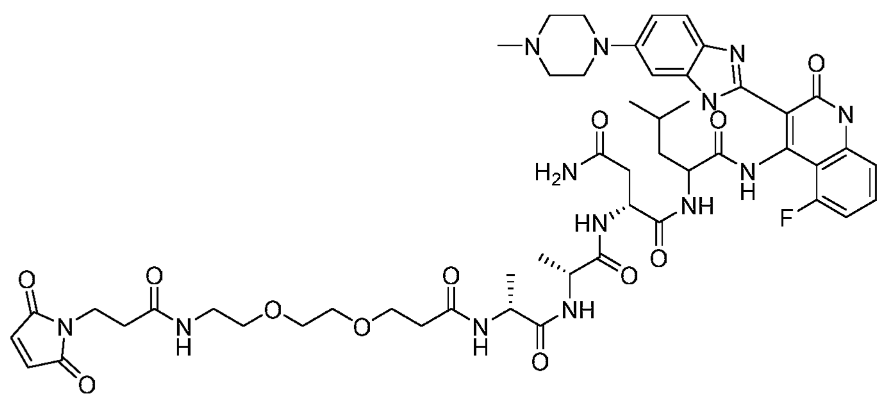

;

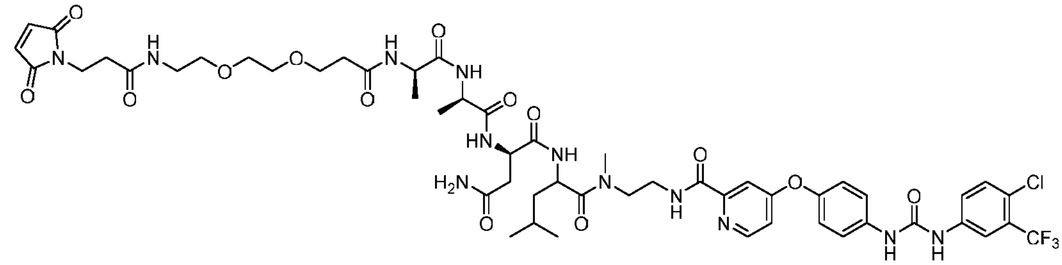

;

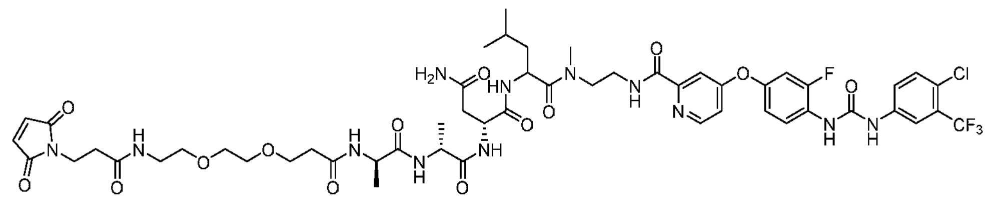

;

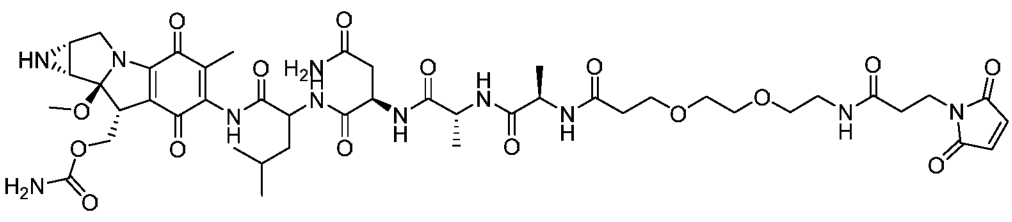

;

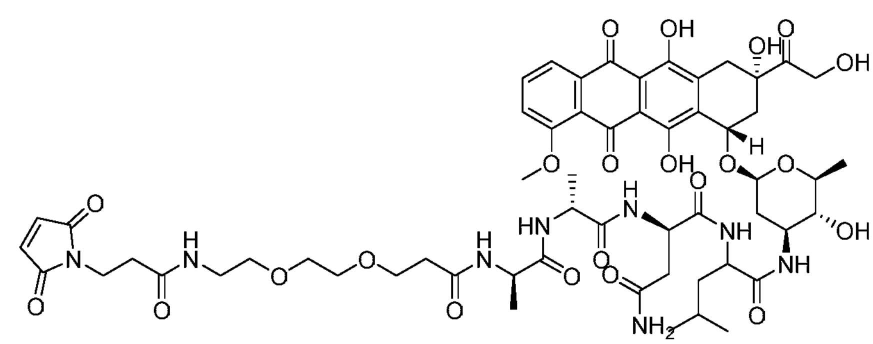

;

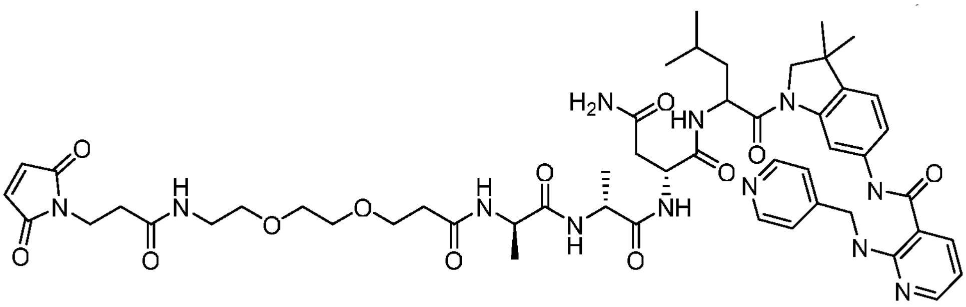

;

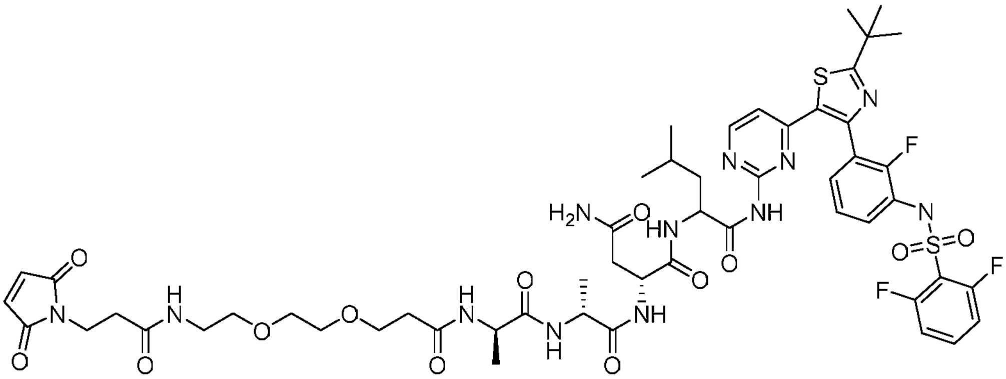

;

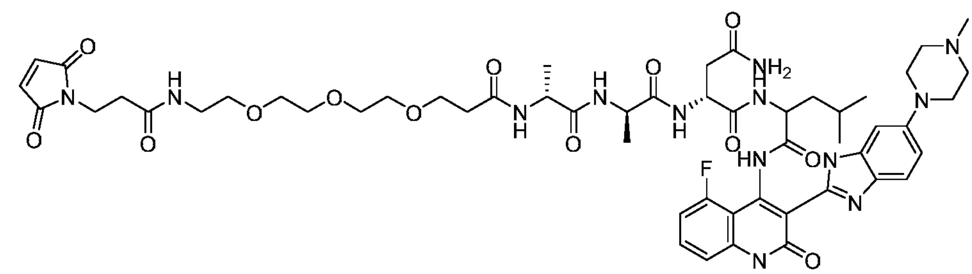

;

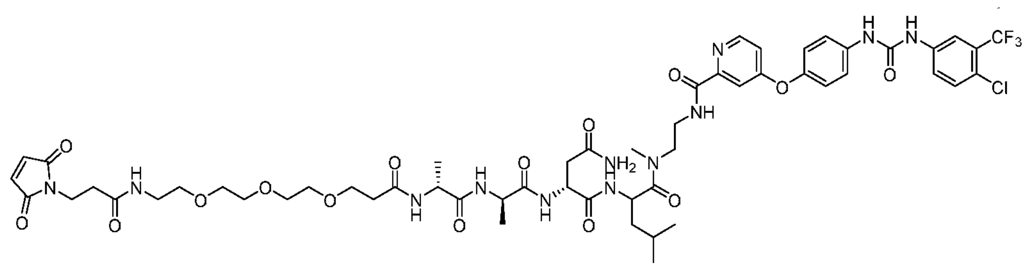

;

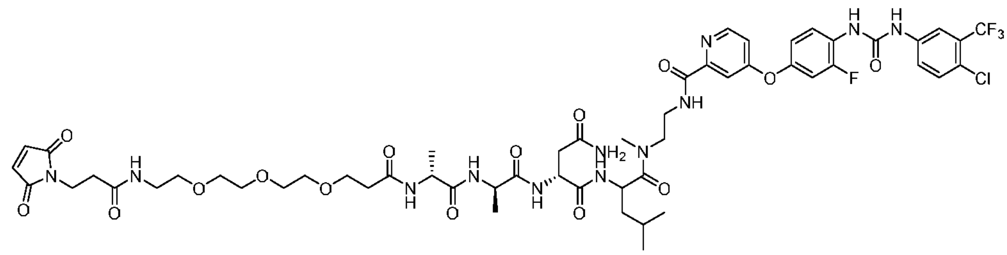

;

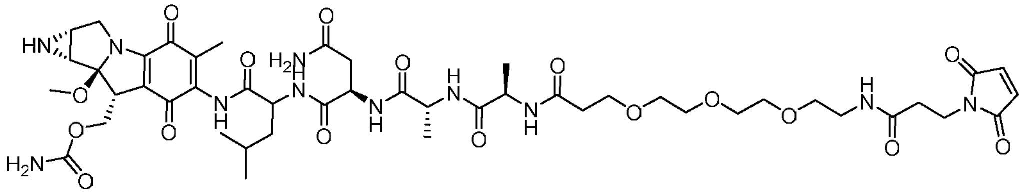

;

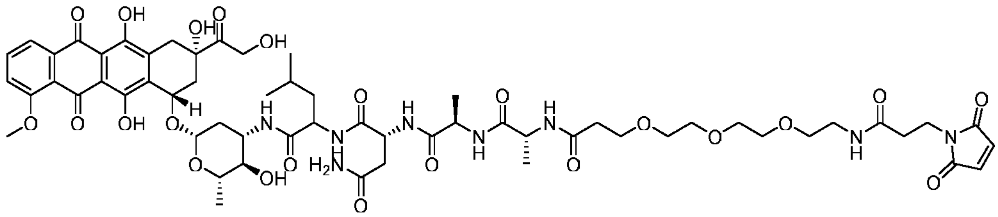

,

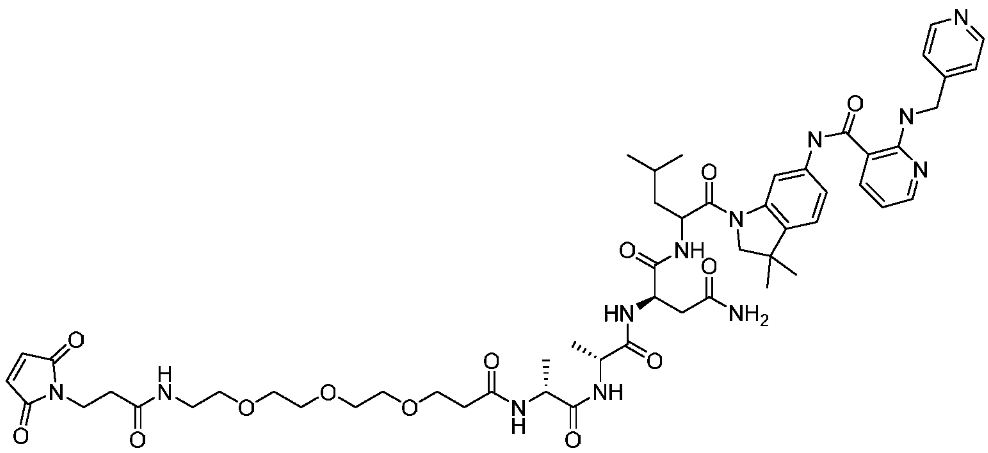

;

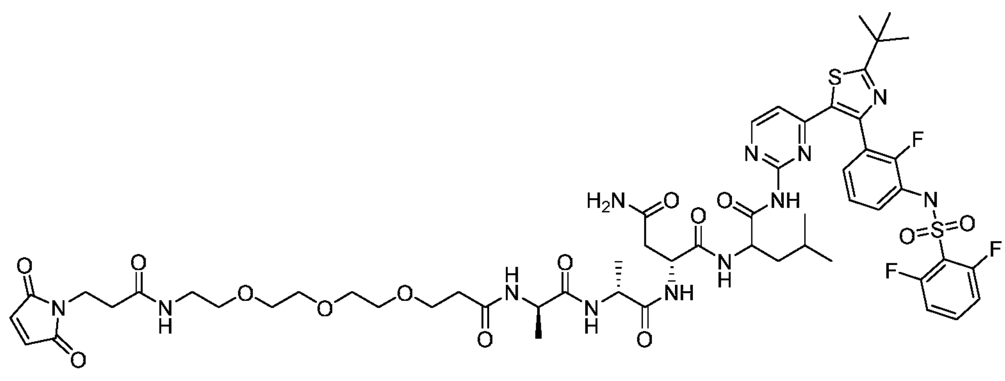

.

**13.** A platinum derivative, prodrug or pharmaceutically acceptable salt thereof having the following structure:

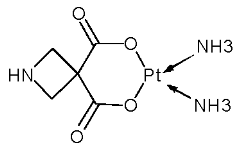

14. The conjugate of claim 11 or 12 formed by covalent coupling with albumin or a pharmaceutically acceptable salt thereof.

15. A pharmaceutical composition comprising the conjugate of claim 11 or 12 or a pharmaceutically acceptable salt thereof, the platinum derivative of claim 13 or a pharmaceutically acceptable salt thereof, or the conjugate of claim 14 or a pharmaceutically acceptable salt thereof, and a pharmaceutically acceptable carrier.

16. Use of the conjugate of claim 11 or 12 or a pharmaceutically acceptable salt thereof, the platinum derivative of claim 13 or a pharmaceutically acceptable salt thereof, or the conjugate of claim 14 or a pharmaceutically acceptable salt thereof in the preparation of a medicament for treating or preventing cancer, fatty liver (including alcoholic and non-alcoholic fatty liver), steatohepatitis, fatty liver disease, liver fibrosis, liver inflammation, and steatosis of liver cell injury; Preferably, the cancer is a solid cancer or a hematological tumor, more preferably a cancer of the bladder, brain, breast/mammary gland, cervix, colon, rectum, esophagus, kidney, liver, lung, nasopharynx, pancreas, prostate, skin, stomach, uterus, ovary, testis and hematological sites.

17. The application of the compound according to any one of claims 1 to 10 in enhancing the water solubility of the compound drug, reducing the drug toxicity, improving the drug efficacy, and/or improving the selectivity of the drug to immune cells, or in preparing a drug with improved water solubility, reduced drug toxicity, improved drug efficacy, and/or improved selectivity of the drug to immune cells, or in preparing a drug molecule for delivering the drug to the liver.

18. An EMC-AANL-DOX compound having a structure represented by the following formula, or a medicament thereof coupled with albumin, for the preparation of a medicament for treating liver cancer, or for the preparation of a medicament for the combined treatment of tumors with an anti-PD-1 antibody:

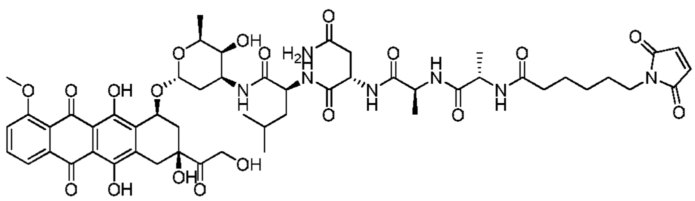

19. Application of the conjugate or the pharmaceutically acceptable salt thereof according to claim 11 or 12, the platinum derivative or the pharmaceutically acceptable salt thereof according to claim 13, or the conjugate or the pharmaceutically acceptable salt thereof according to claim 14 in the preparation of a medicament for inhibiting immunosuppressive cells, inhibiting tumor-associated macrophages, inhibiting MDSC cells, inhibiting angiogenesis, promoting antitumor immunity and/or promoting T lymphocyte proliferation.

20. Application of the conjugate of claim 11 or 12 or a pharmaceutically acceptable salt thereof, the platinum derivative of claim 13 or a pharmaceutically acceptable salt thereof, or the conjugate of claim 14 or a pharmaceutically acceptable salt thereof and an anti-PD-1 antibody in the preparation of a medicament for the combined treatment of tumors.

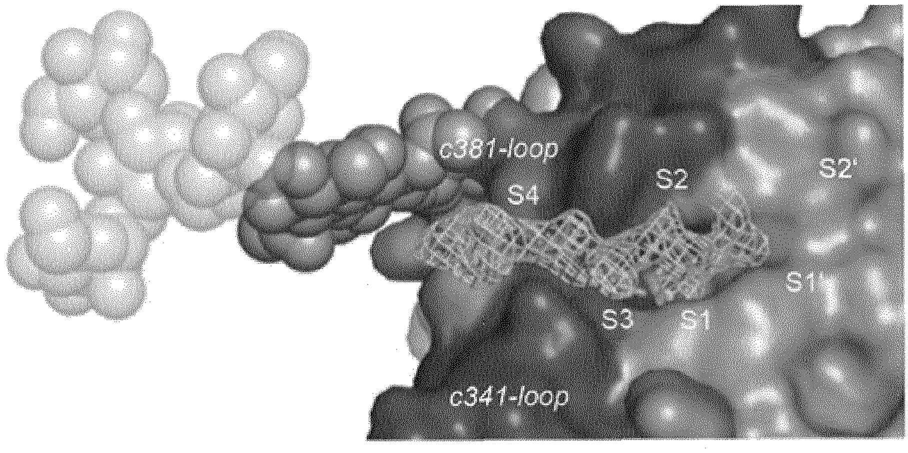


Figure 1

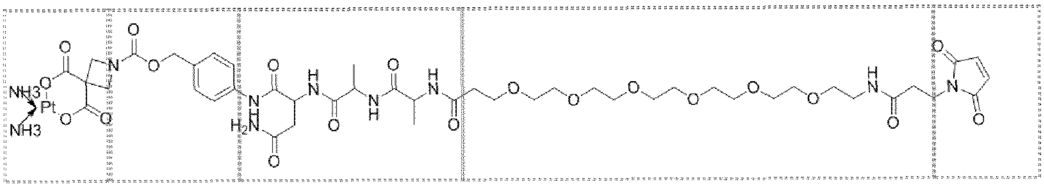

D Drug
A Auxiliary group
C Endoasparaginase
S Selective Group
MI

Figure 2

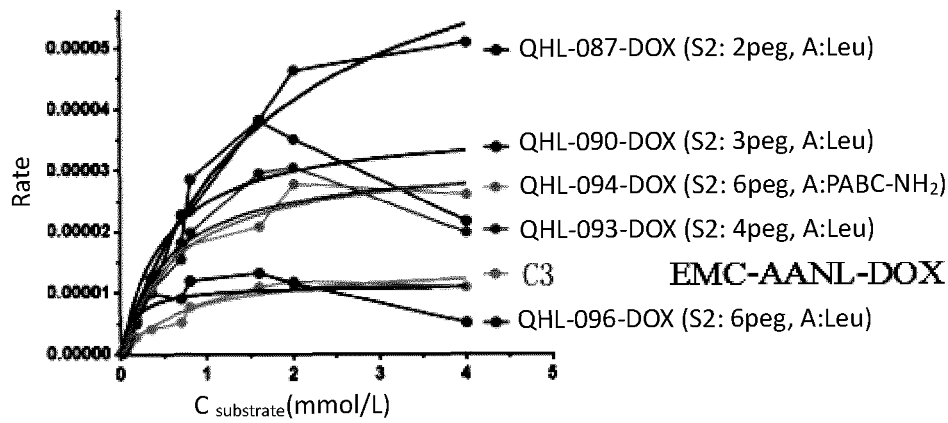


Figure 3

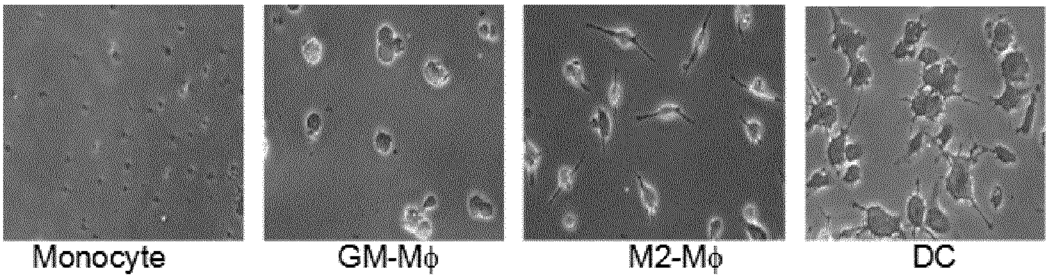


Figure 4

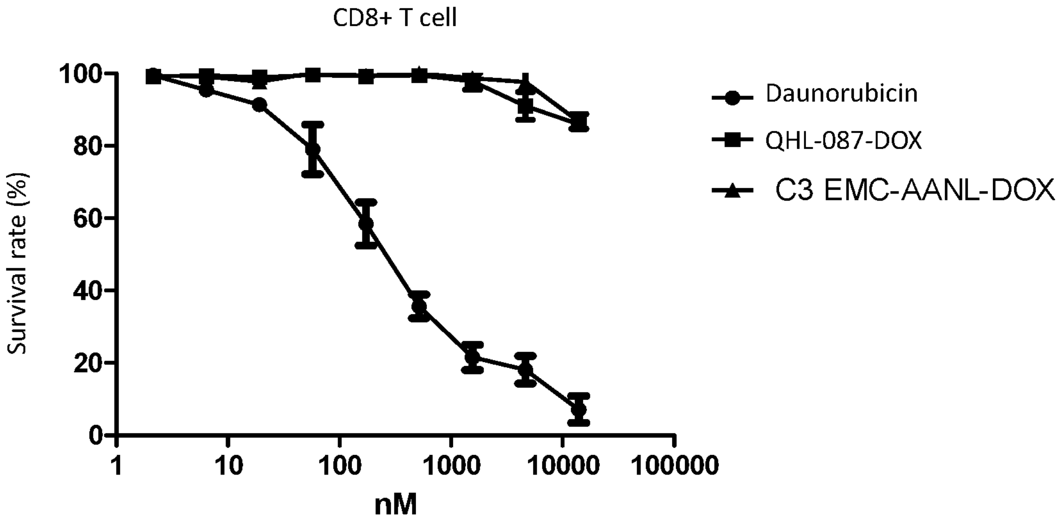


Figure 5

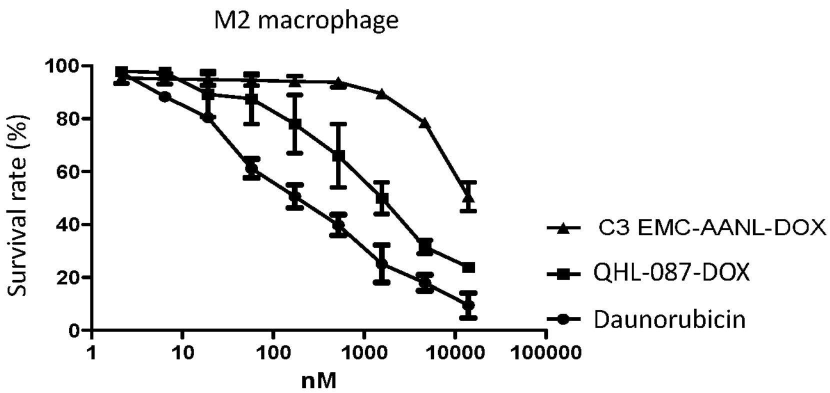


Figure 6

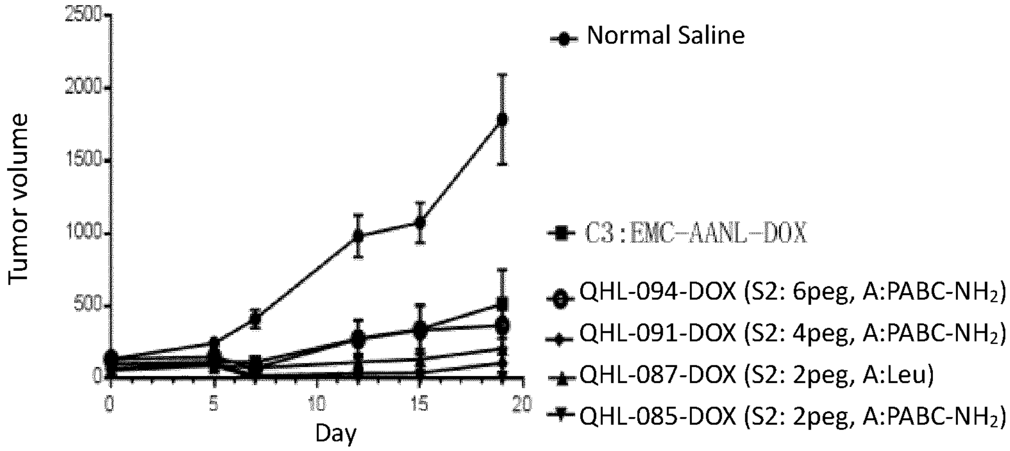


Figure 7

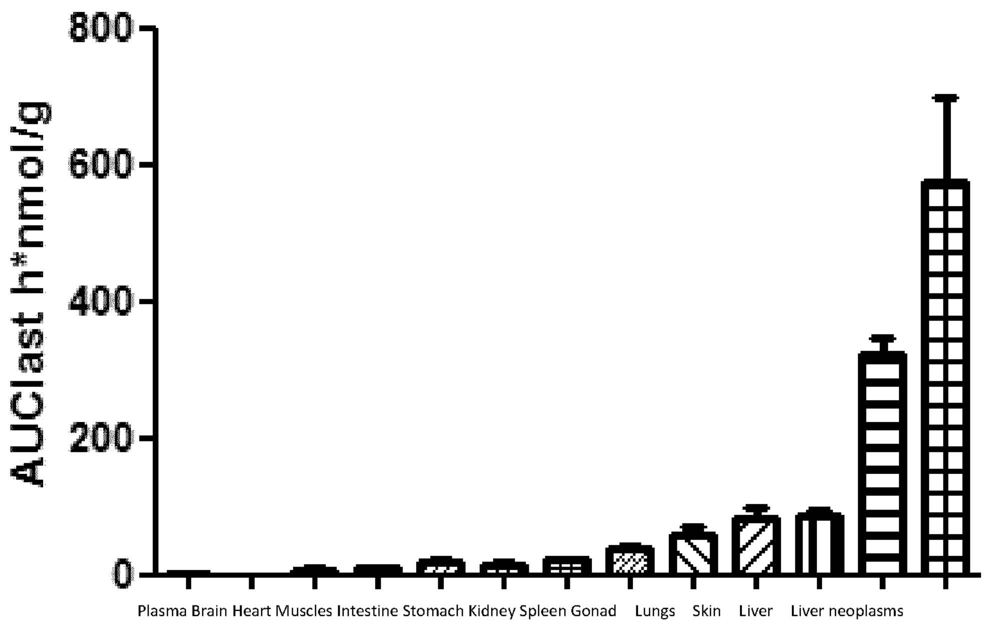


Figure 8

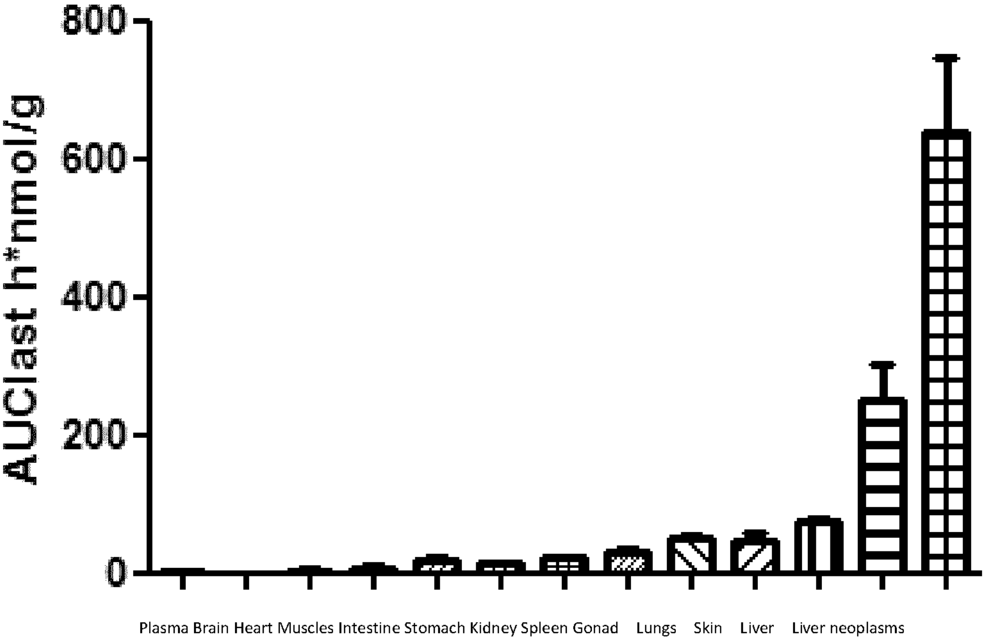
800
600
400
200
0
AUClast h*nmol/g
Plasma Brain Heart Muscles Intestine Stomach Kidney Spleen Gonad Lungs Skin Liver Liver neoplasms

Figure 9

GR1: Normal Saline

GR2: QHL-087-DOX

GR3: Anti-PD-1 antibody

GR4: QHL-087-DOX+
anti-PD-1 antibody

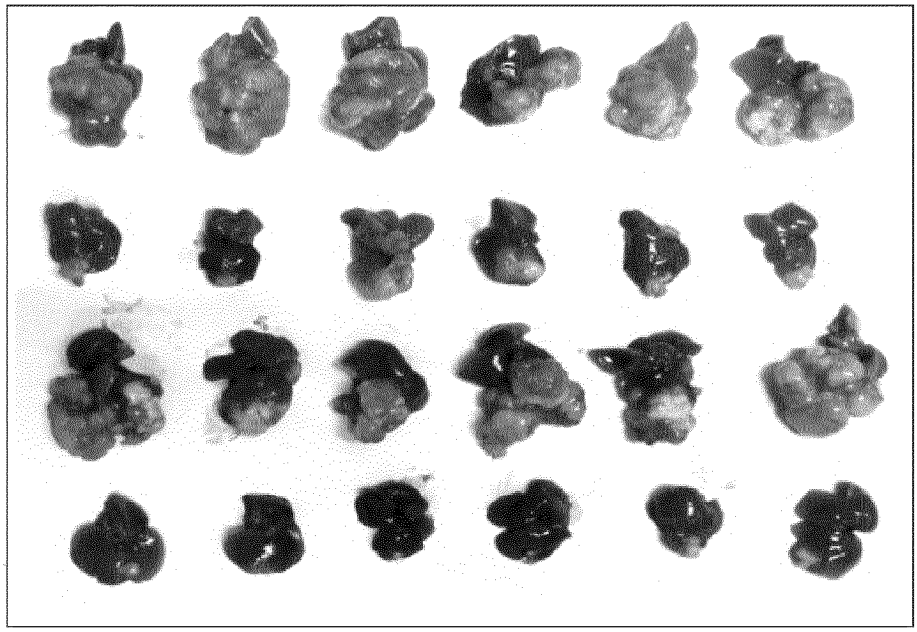

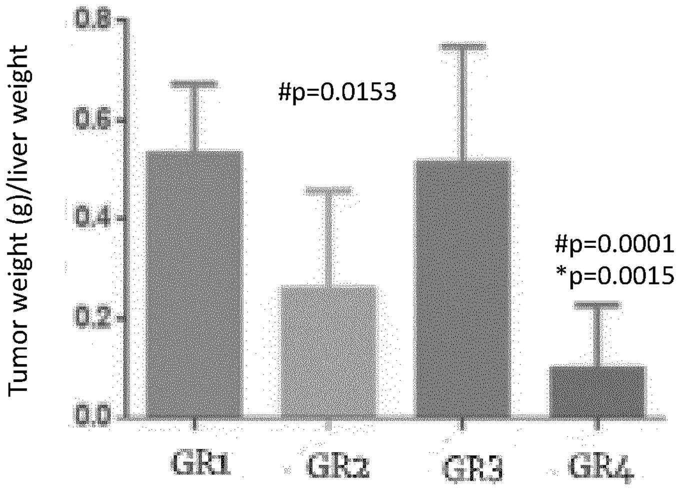


Figure 10

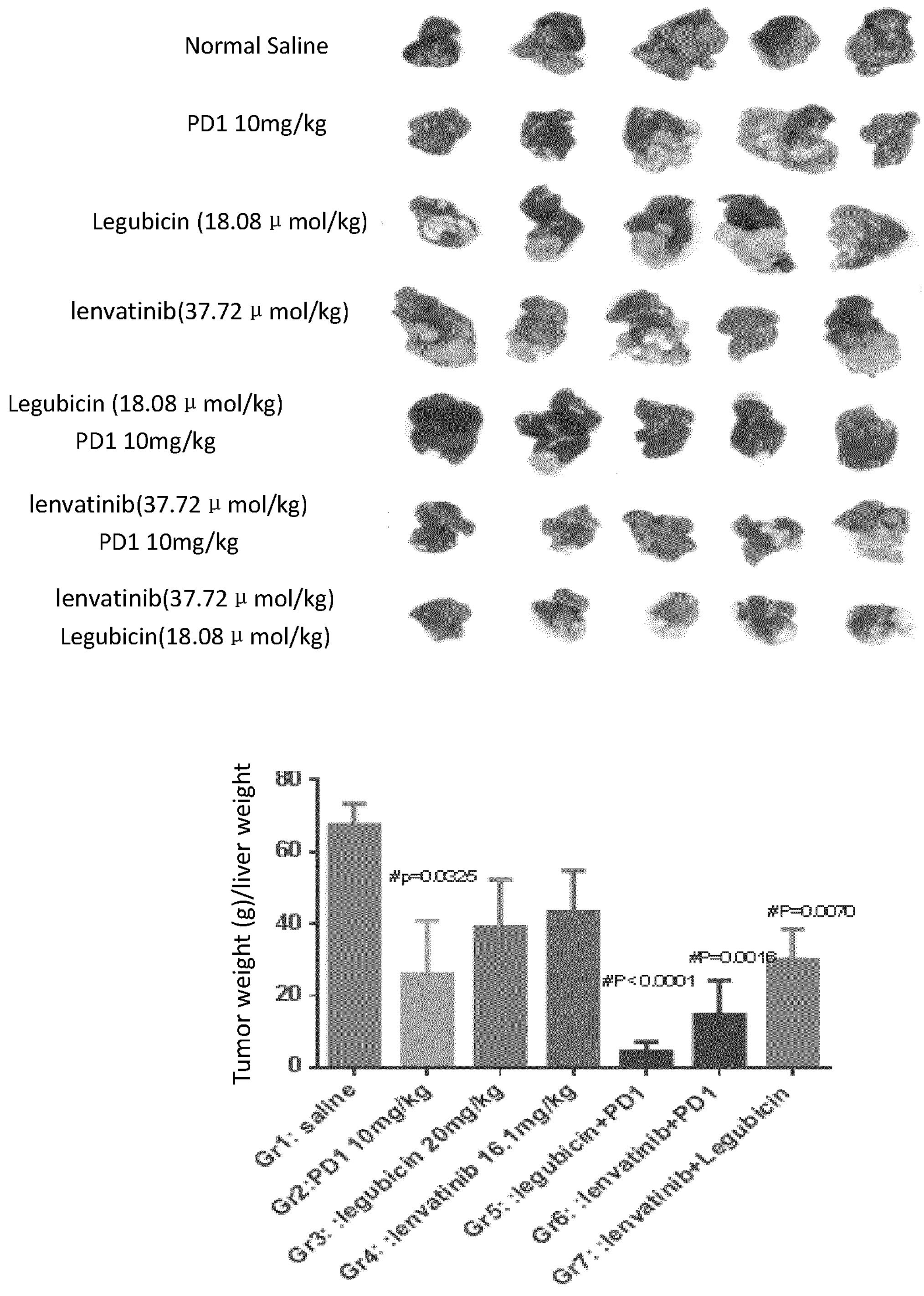

Normal Saline
PD1 10mg/kg
Legubicin (18.08 μ mol/kg)
lenvatinib(37.72 μ mol/kg)
Legubicin (18.08 μ mol/kg)
PD1 10mg/kg
lenvatinib(37.72 μ mol/kg)
PD1 10mg/kg
lenvatinib(37.72 μ mol/kg)
Legubicin(18.08 μ mol/kg)
Tumor weight (g)/liver weight
80
60
40
20
0
#p=0.0325
#P<0.0001
#P=0.0016
#P=0.0070
Gr1: saline
Gr2:PD1 10mg/kg
Gr3: :legubicin 20mg/kg
Gr4: :lenvatinib 16.1mg/kg
Gr5: :legubicin+PD1
Gr6: :lenvatinib+PD1
Gr7: :lenvatinib+Legubicin


Figure 11

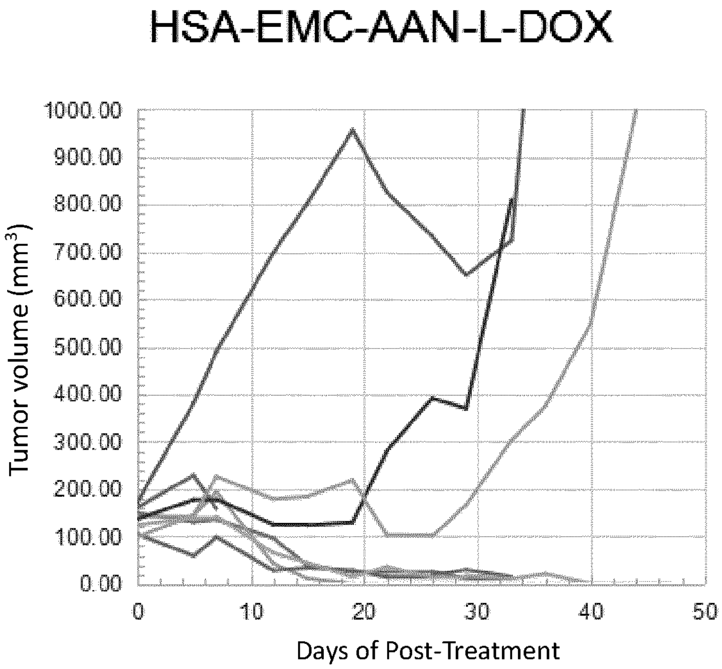
HSA-EMC-AAN-L-DOX
1000.00
900.00
800.00
700.00
600.00
500.00
400.00
300.00
200.00
100.00
0.00
0
10
20
30
40
50
Tumor volume (mm3)
Days of Post-Treatment

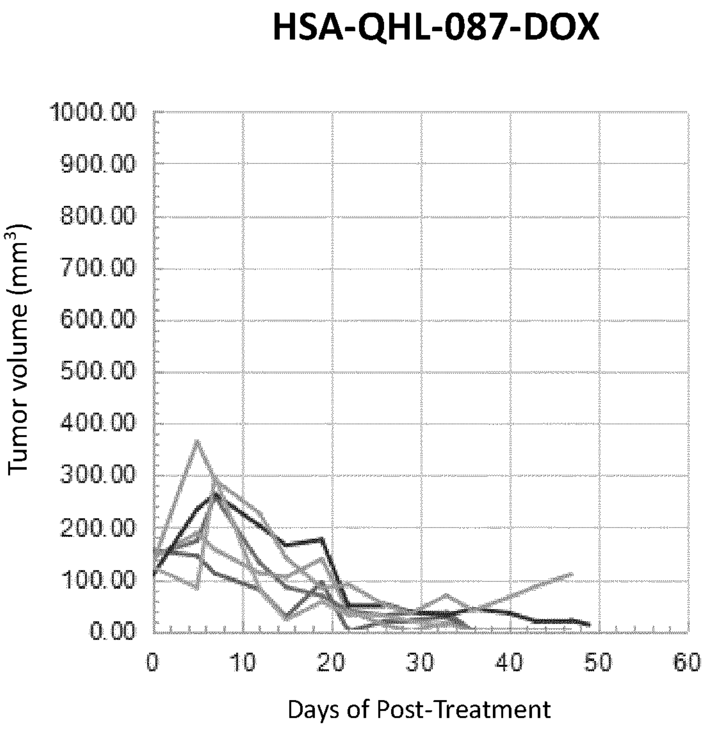
HSA-QHL-087-DOX
1000.00
900.00
800.00
700.00
600.00
500.00
400.00
300.00
200.00
100.00
0.00
0
10
20
30
40
50
60
Tumor volume (mm3)
Days of Post-Treatment

Figure 12

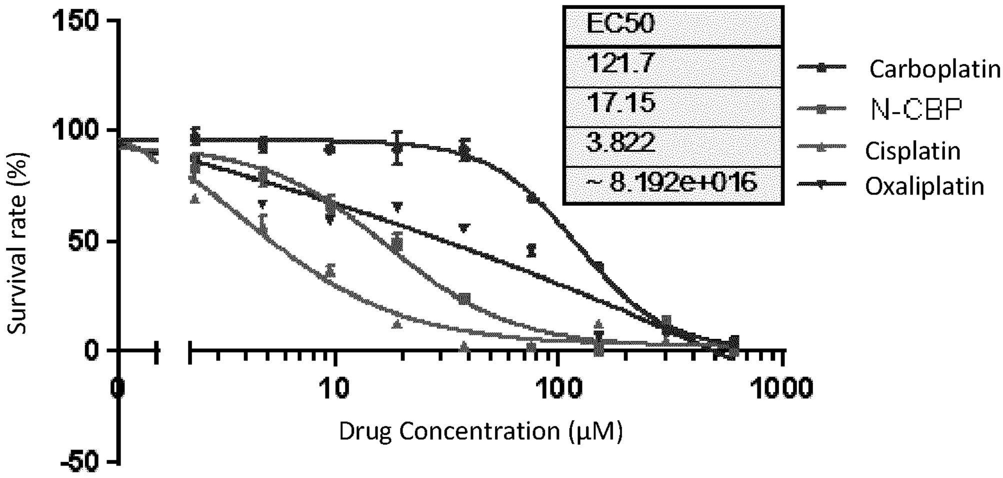


Figure 13

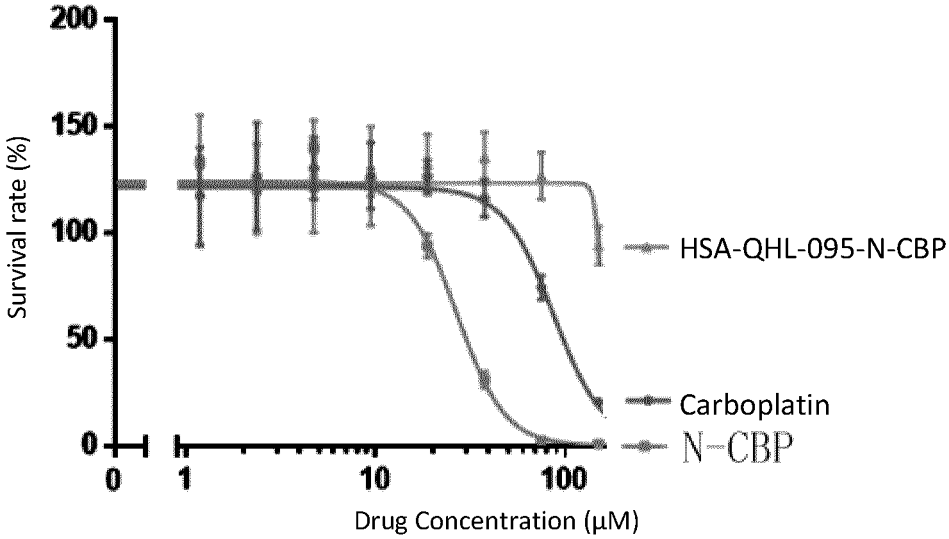


Figure 14

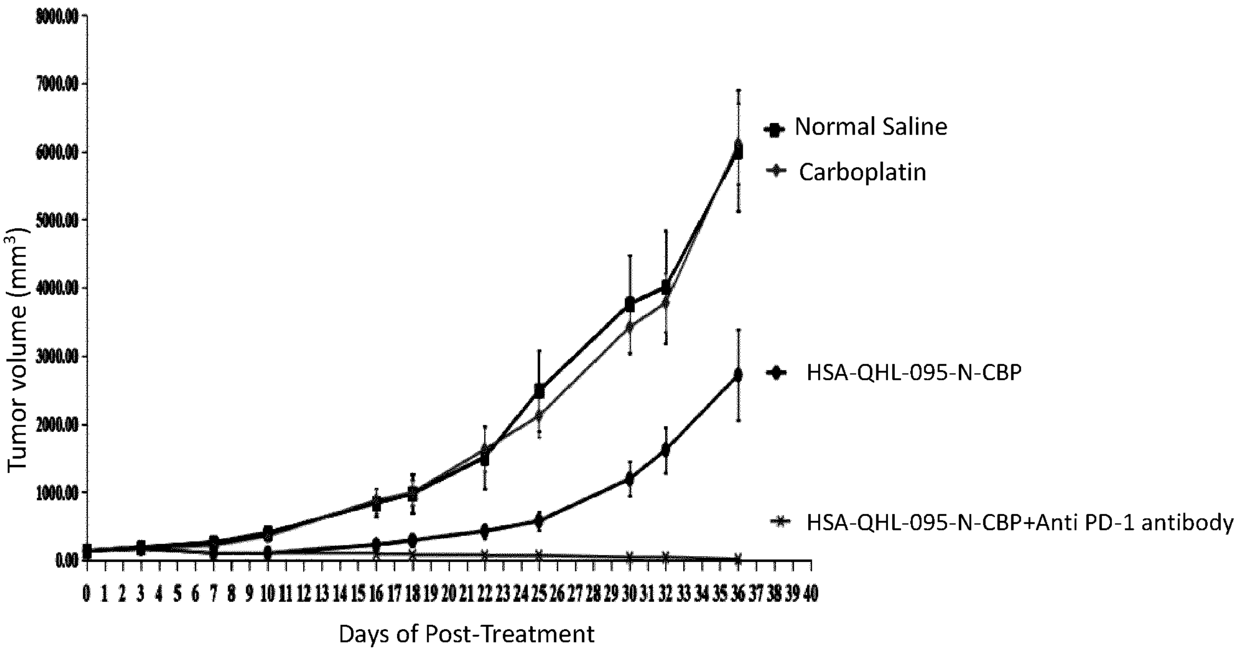
8000.00
7000.00
6000.00
5000.00
4000.00
3000.00
2000.00
1000.00
0.00
Tumor volume (mm3)
0 1 2 3 4 5 6 7 8 9 10 11 12 13 14 15 16 17 18 19 20 21 22 23 24 25 26 27 28 29 30 31 32 33 34 35 36 37 38 39 40
Days of Post-Treatment
Normal Saline
Carboplatin
HSA-QHL-095-N-CBP
HSA-QHL-095-N-CBP+Anti PD-1 antibody

Figure 15

**INTERNATIONAL SEARCH REPORT**

International application No. **PCT/CN2021/077056**

**A. CLASSIFICATION OF SUBJECT MATTER**

C07K 5/083(2006.01)i； C07K 5/093(2006.01)i； C07K 5/103(2006.01)i； A61K 31/704(2006.01)i； A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; STNext: 亚飞生物; 刘辰; 阿霉素; 铂; 天冬酰胺; 聚乙二醇; 马来酰亚胺; 白蛋白; Doxorubicin; Maleimide; Pt; Asparagine; Asn; Legumain; PEG; Maleimide; albumin

**C. DOCUMENTS CONSIDERED TO BE RELEVANT**

| Category* | Citation of document, with indication, where appropriate, of the relevant passages | Relevant to claim No. |
|---|---|---|
| X | CN 103044521 A (YAFEI (SHANGHAI) BIOMEDICAL TECHNOLOGY CO., LTD.) 17 April 2013 (2013-04-17) embodiment 1, description, paragraph [0014], and claim 7 | 1-5, 7-9, 11, 14-20 |
| X | CN 104147612 A (YAFEI (SHANGHAI) BIO-PHARMACEUTICAL CO., LTD.) 19 November 2014 (2014-11-19) claims 1 and 14, and embodiment 6 | 1-5, 7-9, 11, 14-20 |
| Y | CN 104262455 A (YAFEI (SHANGHAI) BIOPHARMACEUTICAL TECHNOLOGY CO., LTD.) 07 January 2015 (2015-01-07) claims 1-5 | 6, 10, 12 |
| X | WO 2014203691 A1 (TAKEUCHI, Y. et al.) 24 December 2014 (2014-12-24) claim 1 | 13, 15 |
| X | CN 1781932 A (NANSHAN PHARMACEUTICAL CO., LTD., CHENGDU) 07 June 2006 (2006-06-07) claims 1-7 | 1-5, 7-9, 11, 14-20 |
| Y | CN 103044521 A (YAFEI (SHANGHAI) BIOMEDICAL TECHNOLOGY CO., LTD.) 17 April 2013 (2013-04-17) embodiment 1, description, paragraph [0014], and claim 7 | 6, 10, 12 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 May 2021** | **20 May 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/077056**

**C. DOCUMENTS CONSIDERED TO BE RELEVANT**

| Category* | Citation of document, with indication, where appropriate, of the relevant passages | Relevant to claim No. |
|---|---|---|
| Y | CN 104147612 A (YAFEI (SHANGHAI) BIO-PHARMACEUTICAL CO., LTD.) 19 November 2014 (2014-11-19) claim 1 and 14, and embodiment 6 | 6, 10, 12 |

**INTERNATIONAL SEARCH REPORT**

International application No.
**PCT/CN2021/077056**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:

[1] Claim 17 relates to the application of a compound in the preparation of a drug with improved aqueous solubility, reduced drug toxicity, improved drug efficacy, and/or reference to drug selectivity for immune cells, which does not comply with PCT Rule 39.1(iv), and a search is made on the basis of the subject name of the compound as described in any one of claims 1-10, in the preparation of a drug with improved aqueous solubility, reduced drug toxicity, improved drug efficacy, and/or improved drug selectivity for immune cells, or in the preparation of drug molecules for delivery of drugs to the liver.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/077056**

| Patent document cited in search report | | | Publication date (day/month/year) | Patent family member(s) | | | Publication date (day/month/year) |
|---|---|---|---|---|---|---|---|
| CN | 103044521 | A | 17 April 2013 | AU | 2015275248 | B2 | 22 February 2018 |
| | | | | AU | 2018203194 | A1 | 24 May 2018 |
| | | | | CN | 103044521 | B | 05 November 2014 |
| | | | | AU | 2018203194 | B2 | 18 July 2019 |
| | | | | AU | 2015275248 | A1 | 25 February 2016 |
| CN | 104147612 | A | 19 November 2014 | CN | 104147612 | B | 24 May 2017 |
| CN | 104262455 | A | 07 January 2015 | CN | 104262455 | B | 03 May 2017 |
| WO | 2014203691 | A1 | 24 December 2014 | JP | WO2014203691 | A1 | 23 February 2017 |
| CN | 1781932 | A | 07 June 2006 | CN | 100381459 | C | 16 April 2008 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201210573744